# EUROPEAN PATENT APPLICATION

(11) **EP 0 845 459 A1**
(43) Date of publication of application: **03.06.1998**
(21) Application number: 98103209.7
(22) Date of filing: 01.06.1992
(51) Int. Cl.: C07D 205/08, C07F 7/18, C07D 477/04

(54) **Azetidinone derivatives useful in the preparation of carbapenem antibiotics**

(30) Priority: 31.05.1991 JP 129576/91; 12.07.1991 JP 172220/91
(62) Divisional of application: 92304997.7
(71) Applicant: Sankyo Company Limited, Tokyo (JP)
(72) Inventor: Hirai, Koichi, Shinagawa-ku, Tokyo 140 (JP); Iwano, Yuji, Shinagawa-ku, Tokyo 140 (JP); Koyama, Hiroo, Westfield, NJ 07090 (US); Nishi, Takahide, Shinagawa-ku, Tokyo 140 (JP); Yoshida, Akira, Shinagawa-ku, Tokyo 140 (JP); Oda, Kozo, Shinagawa-ku, Tokyo 140 (JP)
(74) Representative: Gibson, Christian John Robert

(57) **Abstract**

Compounds of formula (Ia): 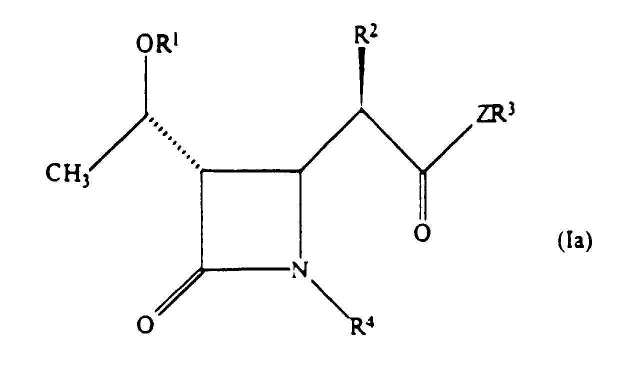 in which: R¹ represents a hydrogen atom or a hydroxy-protecting group; R² represents a methyl group; R³ represents a phenyl group which has a 2-substituent of formula -CYNR⁵R⁶ and no further substituent or has one alkyl substituent, wherein Y represents an oxygen or sulphur atom, and R⁵ and R⁶ are the same or different and each represents an alkyl group, an aryl group, or R⁵ and R⁶ together with the nitrogen atom to which they are attached form a heterocyclic group; R⁴ represents a hydrogen atom or an amino-protecting group; and Z represents a sulphur atom or an oxygen atom,
are valuable intermediates in the preparation of carbapenem compounds and retain a desirable configuration during conversion to such carbapenem compounds.

## Description

The present invention relates to a series of new azetidinone derivatives which may be used as intermediates in the preparation of certain carbapenem antibiotics.

The carbapenem compounds have in common a basic structure which may be represented by the formula (A):

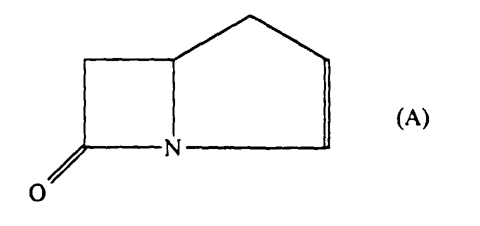

Those carbapenem antibiotics having no substituent at the 1-position are potentially a very useful series of compounds which have extraordinarily potent antibacterial activity. Unfortunately, however, they are chemically unstable and, moreover, are sensitive to dehydropeptidase I in vivo. Dehydropeptidase I is an enzyme which hydrolyses the β-lactam ring in carbapenem antibiotics and which exists in mammalian tissue, for example in the renal cortex. It is responsible for the extensive metabolisation of many otherwise valuable β-lactam antibiotics in animals, including humans, thus greatly reducing their value. Despite these disadvantages, these carbapenem antibiotics are finding increasing use in the treatment of bacterial infections. On the other hand, those carbapenem antibiotics having a 1β-substituent are chemically stable and are resistant to the dehydropeptidase I enzyme. However, none of this series of compounds has been found in nature, and the compounds must, accordingly, be prepared by chemical synthesis. As with many biologically active compounds, the steric configuration of some of the atoms in the molecules of these compounds is of importance and the most interesting compounds have a multi-ring structure whose skeleton may be represented by the formula (B): 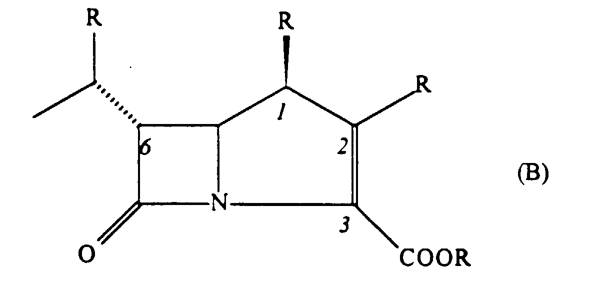 in which each of the symbols R represents any of a variety of substituent groups, some of which may be bite complex, and the different groups represented by R in this formula may be the same or different, although they are normally different from each other. The numbering system shown on this formula is that commonly used in the art for the nomenclature of such compounds and is as used herein.

In the preparation of these compounds, it is necessary to synthesise an azetidinone ring system with various substituent groups preferably in the desired final configuration. This, in general, has proven difficult, although many attempts have been made. For example, U.S. Patent Specifications No. 4,895,939 and 4,772,683 describe the preparation of a compound of formula (C): 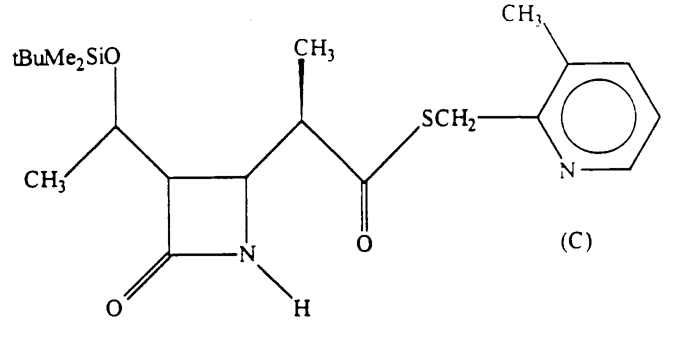 (in which tBu represents a t-butyl group and Me represents a methyl group) by reacting a compound of formula (D): 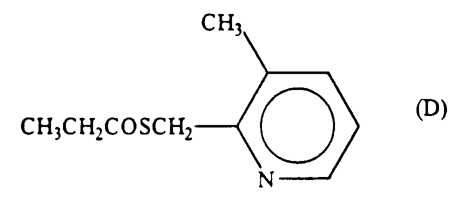 with t-butyldimethylsilyl trifluoroacetate, which has the formula CF₃COOSi(CH₃)₂tBu, in the presence of a base to give a 75 : 25 mixture of compounds of formula (E) and (F): 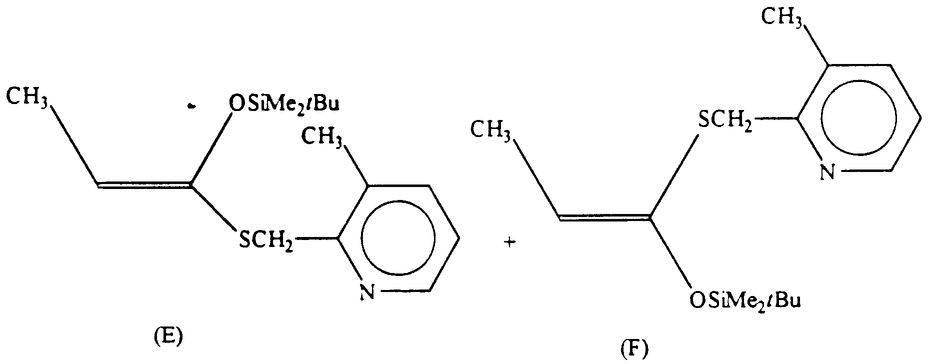 This mixture is then reacted with a compound of formula (G): 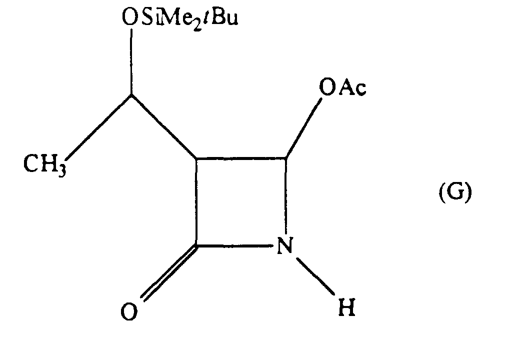 in the presence of a Lewis acid, to give the desired compound of formula (C).

It has been reported that a 2R-isomer of a compound of formula (H): 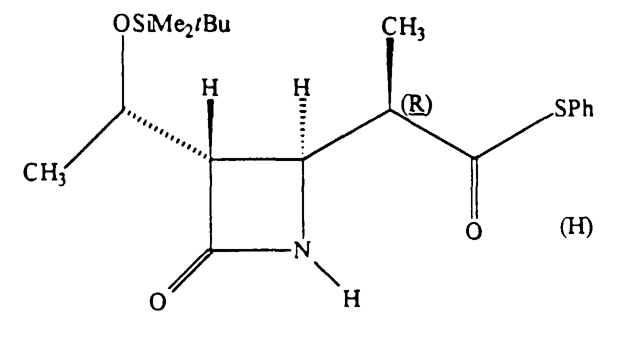 which is a key intermediate in the synthesis of 1β-methylcarbapenem antibiotics, can be synthesised by reacting a silyl enol ether prepared from S-phenyl thiopropionate with (3R,4R)-3-[(1R)-1-t-butyldimethylsilyloxy)ethyl]-4-acetoxy-2-azetidinone or with its 1-trimethylsilyl derivative [T. Shibata et al., Tetrahedron Letters, 26, 4793 (1985); C. U. Kim et al., Tetrahedron Letters, 28, 507 (1987); A. Martel et al., Can. J. Chem., 66, 1537 (1988)].

However, in the syntheses described in these reports, the 2R- and 2S- isomers of the thiopropionic acid derivative of formula (H) are produced in the ratio of 1.6 : 1, 1 : 19 and 1 : 9, respectively. Thus the desired 2R-isomer is prepared in relatively minor amounts and, in most cases, is produced in admixture with a much larger quantity of its less useful 2S-isomer, or, at least, with a substantial quantity of the 2S-isomer, from which its separation is difficult, expensive and inefficient.

There is, therefore, a need for a method of preparing the desired carbapenem antibiotic precursors which allows the required compounds to be obtained in better yields and with the desired isomer as the major product and not in admixture with substantial amounts of an unwanted isomer.

Thus, the compounds of the present invention are those compounds of formula (I): 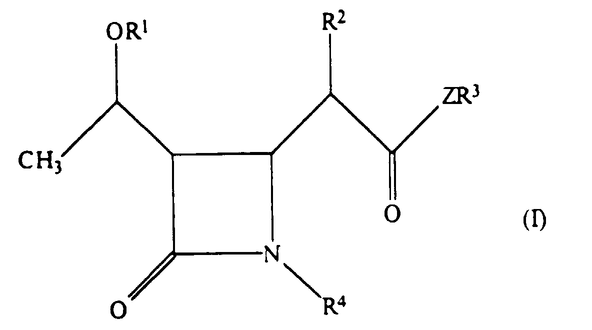 wherein:
R¹ represents a hydrogen atom or a hydroxy-protecting group;
R² represents an alkyl group having from 1 to 6 carbon atoms, an alkoxy group having from 1 to 6 carbon atoms, a halogen atom, an unsubstituted phenyl group, an unsubstituted phenoxy group, or a substituted phenyl or phenoxy group having at least one substituent selected from substituents (a), defined below;
R³ represents:
   a pyridyl group which is unsubstituted or is substituted by at least one substituent selected from substituents (a), defined below;
   a quinolyl group which is unsubstituted or is substituted by at least one substituent selected from substituents (a), defined below; or
   a phenyl group which has a substituent of formula -CYNR⁵R⁶ and no further substituent or has at least one substituent selected from substituents (a), defined below, where
      Y represents an oxygen or sulphur atom; and
      R⁵ and R⁶ are independently selected from alkyl groups having from 1 to 6 carbon atoms, aryl groups as defined below, and aralkyl groups in which the alkyl part has from 1 to 6 carbon atoms and the aryl part is as defined below, or R⁵ and R⁶ together form a group of formula -(CH₂)ₘ-(X)_{P}-(CH₂)ₙ-, wherein
         m and n are independently selected from the cipher 0 and integers from 1 to 5, provided that (m + n) is greater than 1, ℓ is 0 or 1, and X represents an oxygen or sulphur atom or a group of formula =NR⁷, where R⁷ represents an alkyl group having from 1 to 6 carbon atoms, an aliphatic carboxylic acyl group having from 1 to 6 carbon atoms or an aromatic carboxylic acyl group in which the aryl part is as defined below;
R⁴ represents a hydrogen atom or an amino-protecting group; and
Z represents a sulphur atom or an oxygen atom;
said aryl groups and the aryl parts of said aralkyl groups and said aromatic carboxylic acyl groups are carbocyclic aryl groups which have from 6 to 10 carbon atoms in at least one aromatic ring and which are unsubstituted or are substituted by at least one substituent selected from substituents (a), defined below;
said substituents (a) are selected from alkyl groups having from 1 to 6 carbon atoms, alkoxy groups having from 1 to 6 carbon atoms, halogen atoms, cyano groups, nitro groups, hydroxy groups, amino groups, alkylamino groups in which the alkyl part has from 1 to 4 carbon atoms, dialkylamino groups in which each alkyl part has from 1 to 4 carbon atoms and alkylenedioxy groups having from 1 to 3 carbon atoms.

The present invention also provides processes for preparing these compounds and for using them to prepare carbapenem derivatives, which are described in greater detail hereafter.

It is, therefore, an advantage of the present invention that there is provided a series of novel azetidinone derivatives which can be used as intermediates in the preparation of a variety of carbapenem derivatives, including some useful antibiotics.

Other advantages will become apparent as the description proceeds.

The steric configuration of the compounds of the present invention is important and we therefore generally prefer those compounds of formula (I) whose configuration is as shown in formula (Ia):

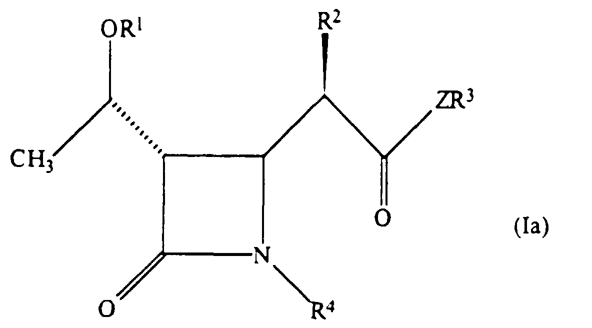

Since the compounds of the present invention are useful as intermediates in the preparation of other compounds and are not themselves used as drugs, the nature of certain groups in the compound whose only function is to protect a group or a part of the molecule from attack during the preparation of those other compounds is not critical. These are the hydroxy-protecting groups which may be represented by R¹ and the amino-protecting groups which may be represented by R⁴.

Where R¹ represents a hydroxy-protecting group, the nature of the group is not critical to the present invention, and it may be selected from a wide range of known groups having regard to criteria usually employed in the art and well known to those skilled in the art, without any particular restriction. Examples of such groups include:
aliphatic acyl groups, preferably: alkanoyl groups having from 1 to 25 carbon atoms, more preferably from 1 to 20 carbon atoms, still more preferably from 1 to 6 carbon atoms, and most preferably from 1 to 4 carbon atoms (such as the formyl, acetyl, propionyl, butyryl, isobutyryl, pivaloyl, valeryl, isovaleryl, hexanoyl, heptanoyl, octanoyl, lauroyl, myristoyl, tridecanoyl, palmitoyl and stearoyl groups, of which the acetyl group is most preferred); halogenated alkanoyl groups preferably having from 2 to 6 carbon atoms, especially halogenated acetyl groups (such as the chloroacetyl, dichloroacetyl, trichloroacetyl and trifluoroacetyl groups); lower alkoxyalkanoyl groups in which the alkoxy part has from 1 to 5, preferably from 1 to 3, carbon atoms and the alkanoyl part has from 2 to 6 carbon atoms and is preferably an acetyl group (such as the methoxyacetyl group); and unsaturated analogues of such groups, especially alkenoyl or alkynoyl groups having from 3 to 6 carbon atoms [such as the acryloyl, methacryloyl, propioloyl, crotonoyl, isocrotonoyl and (E)-2-methyl-2-butenoyl groups];
aromatic acyl groups, preferably arylcarbonyl groups, in which the aryl part has from 6 to 14, more preferably from 6 to 10, still more preferably 6 or 10, and most preferably 6, ring carbon atoms and is a carbocyclic aromatic group, which is unsubstituted or has from 1 to 5, preferably from 1 to 3 substituents, selected from substituents (b), defined and exemplified below, preferably: unsubstituted groups (such as the benzoyl, α-naphthoyl and β-naphthoyl groups); halogenated arylcarbonyl groups (such as the 2-bromobenzoyl and 4-chlorobenzoyl groups); lower alkyl-substituted arylcarbonyl groups, in which the or each alkyl substituent has from 1 to 5, preferably from 1 to 4, carbon atoms (such as the 2,4,6-trimethylbenzoyl and 4-toluoyl groups); lower alkoxy-substituted arylcarbonyl groups, in which the or each alkoxy substituent preferably has from 1 to 5, preferably from 1 to 4, carbon atoms (such as the 4-anisoyl group); nitro-substituted arylcarbonyl groups (such as the 4-nitrobenzoyl and 2-nitrobenzoyl groups); lower alkoxycarbonyl-substituted arylcarbonyl groups, in which the or each alkoxycarbonyl substituent preferably has from 2 to 6 carbon atoms [such as the 2-(methoxycarbonyl)benzoyl group]; and aryl-substituted arylcarbonyl groups, in which the aryl substituent is as defined above, except that, if it is substituted by a further aryl group, that aryl group is not itself substituted by an aryl group (such as the 4-phenylbenzoyl group);
heterocyclic groups having 5 or 6 ring atoms, of which 1 or 2 are hetero-atoms selected from oxygen, sulphur and nitrogen atoms, preferably oxygen or sulphur atoms, which groups may be unsubstituted or may have at least one substituent selected from substituents (b), defined and exemplified below, and oxygen atoms; examples include: the tetrahydropyranyl groups, which may be substituted or unsubstituted, such as the tetrahydropyran-2-yl, 3-bromotetrahydropyran-2-yl and 4-methoxytetrahydropyran-4-yl groups; the tetrahydrothiopyranyl groups, which may be substituted or unsubstituted, such as the tetrahydrothiopyran-2-yl and 4-methoxytetrahydrothiopyran-4-yl groups; the tetrahydrofuranyl groups, which may be substituted or unsubstituted, such as the tetrahydrofuran-2-yl group; and the tetrahydrothienyl groups, which may be substituted or unsubstituted, such as the tetrahydrothien-2-yl group;
tri-substituted silyl groups, in which all three or two or one of the substituents are alkyl groups having from 1 to 5, preferably from 1 to 4, carbon atoms, and, correspondingly, none, one or two of the substituents are aryl groups, as defined above, but preferably phenyl or substituted phenyl groups, preferably: tri(lower alkyl)silyl groups (such as the trimethylsilyl, triethylsilyl, isopropyldimethylsilyl, t-butyldimethylsilyl, methyldiisopropylsilyl, methyldi-t-butylsilyl and triisopropylsilyl groups); and tri(lower alkyl)silyl groups in which one or two of the alkyl groups have been replaced by aryl groups (such as the diphenylmethylsilyl, diphenylbutylsilyl, diphenyl-t-butylsilyl, diphenylisopropylsilyl and phenyldiisopropylsilyl groups);
alkoxyalkyl groups, in which the alkoxy and alkyl parts each have from 1 to 5, preferably from 1 to 4, carbon atoms, especially the alkoxymethyl and alkoxyethyl groups, more especially the alkoxymethyl groups, and such groups which have at least one, preferably from 1 to 5, more preferably from 1 to 3, and most preferably 1, substituents, preferably: lower alkoxymethyl groups and other alkoxyalkyl groups (such as the methoxymethyl, 1,1-dimethyl-1-methoxymethyl, ethoxymethyl, propoxymethyl, isopropoxymethyl, butoxymethyl and t-butoxymethyl groups); lower alkoxy-substituted lower alkoxymethyl groups (such as the 2-methoxyethoxymethyl group); halogenated lower alkoxymethyl groups [such as the 2,2,2-trichloroethoxymethyl and bis(2-chloroethoxy)methyl groups] and lower alkoxy-substituted ethyl groups (such as the 1-ethoxyethyl, 1-methyl-1-methoxyethyl and 1-isopropoxyethyl groups);
other substituted ethyl groups, preferably: halogenated ethyl groups (such as the 2,2,2-trichloroethyl group); and arylselenyl-substituted ethyl groups, in which the aryl part is as defined above [such as the 2-(phenylselenyl)ethyl group];
aralkyl groups, preferably alkyl groups having from 1 to 4, more preferably from 1 to 3 and most preferably 1 or 2, carbon atoms which are substituted with from 1 to 3 aryl groups (in which the aryl group has from 6 to 14 ring carbon atoms, but is otherwise as defined above), which may be unsubstituted (such as the benzyl, phenethyl, 1-phenylethyl, 3-phenylpropyl, α-naphthylmethyl, β-naphthylmethyl, diphenylmethyl, triphenylmethyl, α-naphthyldiphenylmethyl and 9-anthrylmethyl groups) or may be substituted on the aryl part with a lower alkyl group ("lower" meaning "having from 1 to 6 carbon atoms"), a lower alkoxy group, a nitro group, a halogen atom, a cyano group, or an alkylenedioxy group having from 1 to 3 carbon atoms, preferably a methylenedioxy group [such as the 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzoyl, 4-bromobenzyl, 4-cyanobenzyl, 4-cyanobenzyldiphenylmethyl, bis(2-nitrophenyl)methyl and piperonyl groups];
alkoxycarbonyl groups, especially such groups having from 2 to 7, more preferably 2 to 5, carbon atoms and which may be unsubstituted (such as the methoxycarbonyl, ethoxycarbonyl, t-butoxycarbonyl and isobutoxycarbonyl groups) or may be substituted with a halogen atom or a tri-substituted silyl group (as defined above), e.g. a tri(lower alkylsilyl) group (such as the 2,2,2-trichloroethoxycarbonyl and 2-trimethylsilylethoxycarbonyl groups);
alkenyloxycarbonyl groups in which the alkenyl part has from 2 to 6, preferably from 2 to 4, carbon atoms (such as the vinyloxycarbonyl and allyloxycarbonyl groups);
sulpho groups; and
aralkyloxycarbonyl groups, in which the aralkyl part is as defined and exemplified above, but is preferably a benzyl or phenethyl group, more preferably a phenethyl group, and in which the aryl ring, if substituted, preferably has one or two lower alkoxy or nitro substituents (such as the benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl groups).

Of the protecting groups listed above, we prefer: the tri-substituted silyl groups, such as the t-butyldimethylsilyl, trimethylsilyl and triethylsilyl groups; the optionally substituted benzyloxycarbonyl groups, such as the benzyloxycarbonyl and 4-nitrobenzyloxycarbonyl group; and the aliphatic acyl groups, such as the acetyl, chloroacetyl and methoxyacetyl groups. Still more preferred are the tri-substituted silyl groups, especially the t-butyldimethylsilyl and trimethylsilyl groups, more especially the t-butyldimethylsilyl group.

However, the corresponding carbapenem compounds prepared from the compounds of the present invention normally have a hydroxy group in the position corresponding to the group of formula -OR¹, i.e. R¹ is preferably hydrogen, and so the protecting group will be removed before the resulting compound is used in therapy. Its function is, therefore, simply to protect the hydroxy group during preparation of the compounds of the present invention and during conversion of those compounds to the desired carbapenem compounds.

Where R² represents an alkyl group having from 1 to 6 carbon atoms, this may be a straight or branched chain group having from 1 to 6, preferably from 1 to 5, carbon atoms, and examples include the methyl, ethyl, propyl, isopropyl, butyl, isobutyl, sec-butyl, t-butyl, pentyl, isopentyl, neopentyl, 2-methylbutyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups. Of these, we prefer those alkyl groups having from 1 to 4 carbon atoms, preferably the methyl, ethyl, propyl, isopropyl, butyl and isobutyl groups, more preferably a methyl or ethyl group and most preferably the methyl group.

Where R² represents an alkoxy group having from 1 to 6 carbon atoms, this may be a straight or branched chain group having from 1 to 6, preferably from 1 to 5, carbon atoms, and examples include the methoxy, ethoxy, propoxy, isopropoxy, butoxy, isobutoxy, sec-butoxy, t-butoxy, pentyloxy, isopentyloxy, neopentyloxy, 2-methylbutoxy, 1-ethylpropoxy, 4-methylpentyloxy, 3-methylpentyloxy, 2-methylpentyloxy, 1-methylpentyloxy, 3,3-dimethylbutoxy, 2,2-dimethylbutoxy, 1,1-dimethylbutoxy, 1,2-dimethylbutoxy, 1,3-dimethylbutoxy, 2,3-dimethylbutoxy, 2-ethylbutoxy, hexyloxy and isohexyloxy groups. Of these, we prefer those alkoxy groups having from 1 to 4 carbon atoms, preferably the methoxy, ethoxy, propoxy, isopropoxy, butoxy, sec-butoxy and t-butoxy groups, and most preferably the methoxy group.

Where R² represents a halogen atom, this may be a fluorine, chlorine, bromine or iodine atom, preferably a fluorine or chlorine atom, and most preferably a chlorine atom.

Where R² represents a phenyl or phenoxy group, this may be a substituted or unsubstituted group. If substituted, the substituents are preferably selected from substituents (a) defined above and exemplified below. Examples of such substituted groups include the 2-chlorophenyl, 3-chlorophenyl, 4-chlorophenyl, 4-cyanophenyl, 4-nitrophenyl, 2,4-dichlorophenyl, 2,5-dichlorophenyl, 2,4,5-trichlorophenyl, 2-methylphenyl, 3-methylphenyl, 4-methylphenyl, 2,4-dichloro-3-methylphenyl, 4-aminophenyl, 4-methylaminophenyl, 4-dimethylaminophenyl, 4-hydroxyphenyl, 4-methoxyphenyl, 3-methoxyphenyl, 2-methoxyphenyl, 3-chloro-4-methoxyphenyl, 2-chlorophenoxy, 3-chlorophenoxy, 4-chlorophenoxy, 4-cyanophenoxy, 4-nitrophenoxy, 2,4-dichlorophenoxy, 2,5-dichlorophenoxy, 2,4,5-trichlorophenoxy, 2-methylphenoxy, 3-methylphenoxy, 4-methylphenoxy, 2,4-dichloro-3-methylphenoxy, 4-aminophenoxy, 4-methylaminophenoxy, 4-dimethylaminophenoxy, 4-hydroxyphenoxy, 4-methoxyphenoxy, 3-methoxyphenoxy, 2-methoxyphenoxy, and piperonyl (3,4-methylenedioxyphenyl) groups. However, the unsubstituted phenyl and phenoxy groups are preferred.

Of the groups and atoms listed above, R² preferably represents a methyl group, an ethyl group, a methoxy group or a chlorine atom, most preferably a methyl group.

Where R³ represents a pyridyl group, this may be unsubstituted or it may be substituted by at least one substituent selected from substituents (a), defined above and exemplified below. The pyridyl group itself may be a 2-, 3- or 4- pyridyl group and, if it is substituted, the number of substituents is limited only by the number of substitutable positions (four) and possibly by steric constraints. In general the number of substituents is preferably 1 or 2, and more preferably 1. The pyridyl group, whether unsubstituted or substituted, is preferably the 2-pyridyl group. Although the group may be substituted by any one or more of substitutents (a), exemplified below, preferred examples of substituted groups include the 3-methyl-2-pyridyl, 4-methyl-2-pyridyl, 5-methyl-2-pyridyl, 2-methyl-3-pyridyl, 4-methyl-3-pyridyl, 5-methyl-3-pyridyl, 2-methyl-4-pyridyl, 3-methyl-4-pyridyl, 5-methyl-4-pyridyl, 3-ethyl-2-pyridyl, 4-ethyl-2-pyridyl, 5-ethyl-2-pyridyl, 2-ethyl-3-pyridyl, 4-ethyl-3-pyridyl, 5-ethyl-3-pyridyl, 2-ethyl-4-pyridyl, 3-ethyl-4-pyridyl, 5-ethyl-4-pyridyl, 3-chloro-2-pyridyl, 4-chloro-2-pyridyl, 5-chloro-2-pyridyl, 2-chloro-3-pyridyl, 4-chloro-3-pyridyl, 5-chloro-3-pyridyl, 2-chloro-4-pyridyl, 3-chloro-4-pyridyl, 5-chloro-4-pyridyl, 3-methoxy-2-pyridyl, 4-methoxy-2-pyridyl, 5-methoxy-2-pyridyl, 2-methoxy-3-pyridyl, 4-methoxy-3-pyridyl, 5-methoxy-3-pyridyl, 2-methoxy-4-pyridyl, 3-methoxy-4-pyridyl, 5-methoxy-4-pyridyl, 3-nitro-2-pyridyl, 4-nitro-2-pyridyl, 5-nitro-2-pyridyl, 2-nitro-3-pyridyl, 4-nitro-3-pyridyl, 5-nitro-3-pyridyl, 2-nitro-4-pyridyl, 3-nitro-4-pyridyl and 5-nitro-4-pyridyl groups, of which the 3-methyl-2-pyridyl, 4-methyl-2-pyridyl and 5-methyl-2-pyridyl groups are more preferred.

Where R³ represents a quinolyl group, this may be unsubstituted or it may be substituted by at least one substituent selected from substituents (a), defined above and exemplified below. The quinolyl group itself may be a 2-, 3- or 4- quinolyl group and, if it is substituted, the number of substituents is limited only by the number of substitutable positions (four) and possibly by steric constraints. In general the number of substituents is preferably 1 or 2, and more preferably 1. The quinolyl group, whether unsubstituted or substituted, is preferably the 2-quinolyl group. Although the group may be substituted by any one or more of substitutents (a), exemplified below, preferred examples of substituted groups include the 3-methyl-2-quinolyl, 4-methyl-2-quinolyl, 5-methyl-2-quinolyl, 2-methyl-3-quinolyl, 4-methyl-3-quinolyl, 5-methyl-3-quinolyl, 2-methyl-4-quinolyl, 3-methyl-4-quinolyl, 5-methyl-4-quinolyl, 3-ethyl-2-quinolyl, 4-ethyl-2-quinolyl, 5-ethyl-2-quinolyl, 2-ethyl-3-quinolyl, 4-ethyl-3-quinolyl, 5-ethyl-3-quinolyl, 2-ethyl-4-quinolyl, 3-ethyl-4-quinolyl, 5-ethyl-4-quinolyl, 3-chloro-2-quinolyl, 4-chloro-2-quinolyl, 5-chloro-2-quinolyl, 2-chloro-3-quinolyl, 4-chloro-3-quinolyl, 5-chloro-3-quinolyl, 2-chloro-4-quinolyl, 3-chloro-4-quinolyl, 5-chloro-4-quinolyl, 3-methoxy-2-quinolyl, 4-methoxy-2-quinolyl, 5-methoxy-2-quinolyl, 2-methoxy-3-quinolyl, 4-methoxy-3-quinolyl, 5-methoxy-3-quinolyl, 2-methoxy-4-quinolyl, 3-methoxy-4-quinolyl, 5-methoxy-4-quinolyl, 3-nitro-2-quinolyl, 4-nitro-2-quinolyl, 5-nitro-2-quinolyl, 2-nitro-3-quinolyl, 4-nitro-3-quinolyl, 5-nitro-3-quinolyl, 2-nitro-4-quinolyl, 3-nitro-4-quinolyl and 5-nitro-4-quinolyl groups, of which the 3-methyl-2-quinolyl, 4-methyl-2-quinolyl and 5-methyl-2-quinolyl groups are more preferred.

However, the unsubstituted pyridyl and quinolyl groups are preferred, and the pyridyl group is most preferred.

Where R³ represents a phenyl group, this is substituted by a carbamoyl or heterocyclic-carbonyl group of formula -CONR⁵R⁶ or a (thiocarbamoyl) or heterocyclic-(thiocarbonyl) group of formula -CSNR⁵R⁶, in which R⁵ and R⁶ are as defined above. In addition, the phenyl group may optionally be substituted by one or more further substitutents selected from substituents (a), defined above and exemplified below.

Where R⁵ or R⁶ represents an alkyl group, this has from 1 to 6 carbon atoms and may be a straight or branched chain group. Examples of such groups are as given above in relation to the groups which may be represented by R². Of these, the methyl, ethyl, propyl, isopropyl, butyl, isobutyl and pentyl groups are preferred, and the methyl, ethyl and propyl groups are more preferred, the ethyl group being most preferred.

Where R⁵ or R⁶ represents an aryl group, this has from 6 to 10, preferably 6 or 10, carbon atoms, and may be, for example, a phenyl or naphthyl (1- or 2-naphthyl) group. The group may be unsubstituted or it may be substituted by one or more substituents selected from substituents (a), defined above and exemplified below. Examples of such substituted aryl groups include the 2-chlorophenyl, 3-chlorophenyl, 4-chlorophenyl, 2-chloro-1-naphthyl, 1-chloro-2-naphthyl, 1-methyl-2-naphthyl, 4-cyanophenyl, 4-nitrophenyl, 2,4-dichlorophenyl, 2,5-dichlorophenyl, 2,4,5-trichlorophenyl, 2-methylphenyl, 3-methylphenyl, 4-methylphenyl, 2,4-dichloro-3-methylphenyl, 4-aminophenyl, 4-methylaminophenyl, 4-dimethylaminophenyl, 4-hydroxyphenyl, 4-methoxyphenyl, 3-methoxyphenyl, 2-methoxyphenyl, 3-chloro-4-methoxyphenyl and piperonyl groups, of which the 2-methylphenyl, 4-methoxyphenyl, 3-chlorophenyl, 4-fluorophenyl, 4-methylphenyl and 4-chlorophenyl groups are preferred. However, the unsubstituted phenyl and naphthyl groups are more preferred and the phenyl group is most preferred.

Where R⁵ or R⁶ represents an aralkyl group, the aryl group may be any of those exemplified above in relation to the aryl groups which may be represented by R⁵ and R⁶, and the alkyl part may be any of those alkyl groups exemplified above in relation to the alkyl groups which may be represented by R². Preferred examples of such groups include the benzyl, phenethyl (i.e. 2-phenylethyl), 1-phenylethyl, 3-phenylpropyl, 4-phenylbutyl, 5-phenylpentyl, 6-phenylhexyl, 2-phenylpropyl, 1-phenylpropyl, 1-naphthylmethyl, 2-naphthylmethyl, 2-(2-naphthyl)ethyl, benzhydryl (i.e. diphenylmethyl) and trityl (i.e. triphenylmethyl) groups and analogues of such groups in which the phenyl and naphthyl groups are replaced by the substituted groups exemplified above in relation to R⁵ and R⁶. Of these, the benzyl and phenethyl groups are preferred and the benzyl group is most preferred.

Where R⁵ and R⁶ represent separate groups, these groups may be the same or different, although it is generally most convenient if they are the same.

Alternatively, R⁵ and R⁶ may together represent a group of formula -(CH₂)ₘ-(X)_{ℓ}-(CH₂)ₙ-, wherein m and n are independently selected from the cipher 0 and integers from 1 to 5, provided that (m + n) is greater than 1, ℓ is 0 or 1, and X represents an oxygen or sulphur atom or a group of formula =NR⁷, where R⁷ represents an alkyl group having from 1 to 6 carbon atoms, an aliphatic carboxylic acyl group having from 1 to 6 carbon atoms or an aromatic carboxylic acyl group in which the aryl part is as defined above. In this group, (m + n) must be greater than 1, and, when ℓ is 0, preferably total 4, 5, 6 or 7, to form, with the nitrogen atom to which they are attached, a pyrrolidinyl, piperidyl, azepinyl or azocinyl group, respectively. When ℓ is 1, (m + n) preferably totals 3, 4, 5 or 6, and (m - n) is preferably -1, 0 or +1; more preferably m and n are each 2. Where X represents a group of formula =NR⁷, and R⁷ represents an alkyl group having from 1 to 6 carbon atoms, these may be any of the alkyl groups exemplified above in relation to the alkyl groups which may be represented by R², but the methyl and ethyl groups are preferred. Examples of aliphatic acyl groups and aromatic acyl groups which may be represented by R⁷ include the corresponding groups examplified above in relation to the hydroxy-protecting groups which may be represented by R¹. Of these, the alkanoyl groups having from 1 to 4 carbon atoms (particularly the formyl, acetyl, propionyl and butyryl groups) and the benzoyl and methyl-substituted benzoyl groups (particularly the p-toluoyl group) are preferred.

Particularly preferred groups of formula -(CH₂)ₘ-(X)_{P}-(CH₂)ₙ- include those groups of formula:
-(CH₂)₂-,
-(CH₂)₃-,
-(CH₂)₄-,
-(CH₂)₅-,
-(CH₂)₆-,
-(CH₂)₇-,
-(CH₂)₈-,
-(CH₂)₂O(CH₂)₂-,
-(CH₂)₂O(CH₂)₃-,
-(CH₂)₃O(CH₂)₃-,
-(CH₂)₄O(CH₂)₄-,
-(CH₂)₂S(CH₂)₂-,
-(CH₂)₃S(CH₂)₃-,
-(CH₂)₂S(CH₂)₃-,
-(CH₂)₂NMe(CH₂)₂-,
-(CH₂)₂NBoz(CH₂)₂-,
-(CH₂)₂NAc(CH₂)₂-,
-(CH₂)₂NAc(CH₂)₃-, and
-(CH₂)₂NEt(CH₂)₃-,
where Ac represents an acetyl group, Boz represents a benzoyl group, Et represents an ethyl group and Me represents a methyl group. Of these, the more preferred groups are those of formula:
-(CH₂)₄-,
-(CH₂)₅-,
-(CH₂)₆-, and
-(CH₂)₂O(CH₂)₂-,

Where R⁴ represents a amino-protecting group, the nature of the group is not critical to the present invention, and it may be selected having regard to criteria usually employed in the art and well known to those skilled in the art, without any particular restriction. Examples of such groups include:
tri-substituted silyl groups, in which all three or two or one of the substituents are alkyl groups having from 1 to 5, preferably from 1 to 4, carbon atoms, and none, one or two of the substituents are aryl groups, as defined above, but preferably phenyl or substituted phenyl groups, preferably: tri(lower alkyl)silyl groups (such as the trimethylsilyl, triethylsilyl, isopropyldimethylsilyl, t-butyldimethylsilyl, methyldiisopropylsilyl, methyldi-t-butylsilyl and triisopropylsilyl groups); and tri(lower alkyl)silyl groups in which one or two of the alkyl groups have been replaced by aryl groups (such as the diphenylmethylsilyl, diphenylbutylsilyl, diphenyl-t-butylsilyl, diphenylisopropylsilyl and phenyldiisopropylsilyl groups);
aliphatic acyl groups, preferably: alkanoyl groups having from 1 to 25 carbon atoms, more preferably from 1 to 20 carbon atoms, still more preferably from 1 to 6 carbon atoms, and most preferably from 1 to 4 carbon atoms (such as the formyl, acetyl, propionyl, butyryl, isobutyryl, pivaloyl, valeryl, isovaleryl, hexanoyl, heptanoyl, octanoyl, lauroyl, myristoyl, tridecanoyl, palmitoyl and stearoyl groups, of which the acetyl group is most preferred); halogenated alkanoyl groups having from 2 to 6 carbon atoms, especially halogenated acetyl groups (such as the chloroacetyl, dichloroacetyl, trichloroacetyl and trifluoroacetyl groups); lower alkoxyalkanoyl groups in which the alkoxy part has from 1 to 5, preferably from 1 to 3, carbon atoms and the alkanoyl part has from 2 to 6 carbon atoms and is preferably an acetyl group (such as the methoxyacetyl group); and unsaturated analogues of such groups, especially alkenoyl or alkynoyl groups having from 3 to 6 carbon atoms [such as the acryloyl, methacryloyl, propioloyl, crotonoyl, isocrotonoyl and (E)-2-methyl-2-butenoyl groups]; and
aromatic acyl groups, preferably arylcarbonyl groups, in which the aryl part has from 6 to 14, more preferably from 6 to 10, still more preferably 6 or 10, and most preferably 6, ring carbon atoms and is a carbocyclic group, which is unsubstituted or has from 1 to 5, preferably from 1 to 3 substituents, selected from substituents (b), defined and exemplified below, preferably: unsubstituted groups (such as the benzoyl, α-naphthoyl and β-naphthoyl groups); halogenated arylcarbonyl groups (such as the 2-bromobenzoyl and 4-chlorobenzoyl groups); lower alkyl-substituted arylcarbonyl groups, in which the or each alkyl substituent has from 1 to 5, preferably from 1 to 4, carbon atoms (such as the 2,4,6-trimethylbenzoyl and 4-toluoyl groups); lower alkoxy-substituted arylcarbonyl groups, in which the or each alkoxy substituent preferably has from 1 to 5, preferably from 1 to 4, carbon atoms (such as the 4-anisoyl group); nitro-substituted arylcarbonyl groups (such as the 4-nitrobenzoyl and 2-nitrobenzoyl groups);
lower alkoxycarbonyl-substituted arylcarbonyl groups, in which the or each alkoxycarbonyl substituent preferably has from 2 to 6 carbon atoms [such as the 2-(methoxycarbonyl)benzoyl group]; and aryl-substituted arylcarbonyl groups, in which the aryl substituent is as defined above, except that, if it is substituted by a further aryl group, that aryl group is not itself substituted by an aryl group (such as the 4-phenylbenzoyl group).

Of these, the tri-substituted silyl groups are preferred and the trimethylsilyl and t-butyldimethylsilyl groups are most preferred.

Examples of the groups and atoms which may be included in substituents (a) are:
alkyl groups having from 1 to 6 carbon atoms, such as those exemplified above in relation to R²;
alkoxy groups having from 1 to 6 carbon atoms, such as those exemplified above in relation to R²;
halogen atoms, such as those exemplified above in relation to R²;
cyano groups, nitro groups, hydroxy groups and amino groups;
alkylamino groups in which the alkyl part has from 1 to 4 carbon atoms, such as the methylamino, ethylamino, propylamino, isopropylamino, butylamino, isobutylamino, sec-butylamino and t-butylamino groups;
dialkylamino groups in which each alkyl part has from 1 to 4 carbon atoms, such as the dimethylamino, diethylamino, dipropylamino, diisopropylamino, dibutylamino, diisobutylamino, methylethylamino and methylbutylamino groups; and
alkylenedioxy groups having from 1 to 3 carbon atoms, such as the methylenedioxy, ethylenedioxy, trimethylenedioxy and propylenedioxy groups, of which the methylenedioxy group is most preferred.

Examples of the groups and atoms which may be included in substituents (b) are those groups and atoms exemplified above in relation to substitutents (a), and:
alkoxycarbonyl groups, especially such groups having from 2 to 7, more preferably 2 to 5, carbon atoms and which may be unsubstituted (such as the methoxycarbonyl, ethoxycarbonyl, t-butoxycarbonyl and isobutoxycarbonyl groups) or substituted with a halogen atom or a tri-substituted silyl group, e.g. a tri(lower alkylsilyl) group (such as the 2,2,2-trichloroethoxycarbonyl and 2-trimethylsilylethoxycarbonyl groups); and
aryl groups, such as those exemplified above in relation to R⁵ and R⁶.

Of the compounds of the present invention, one class of preferred compounds are those compounds of formulae (I) and (Ia), in which:
R¹ represents a hydrogen atom or a hydroxy-protecting group;
R² represents an alkyl group having from 1 to 6 carbon atoms, an alkoxy group having from 1 to 6 carbon atoms, a halogen atom, an unsubstituted phenyl group or an unsubstituted phenoxy group;
R³ represents:
   a pyridyl group which is unsubstituted or is substituted by at least one substituent selected from substituents (a'), defined below; or
   a quinolyl group which is unsubstituted or is substituted by at least one substituent selected from substituents (a'), defined below;
R⁴ represents a hydrogen atom or an amino-protecting group; and
Z represents a sulphur atom;
said substituents (a') are selected from alkyl groups having from 1 to 6 carbon atoms, alkoxy groups having from 1 to 6 carbon atoms and halogen atoms.

Of these, a more preferred class of compounds of the present invention are those compounds of formulae (I) and (Ia), in which:
R¹ represents:
   a hydrogen atom;
   a tri-substituted silyl group, in which all three or two or one of the substituents are alkyl groups having from 1 to 5 carbon atoms, and none, one or two of the substituents are aryl groups, as defined above;
   an aliphatic acyl group having from 1 to 6 carbon atoms;
   a halogenated alkanoyl group having from 2 to 6 carbon atoms;
   an alkoxyalkanoyl group in which the alkoxy part has from 1 to 5 carbon atoms and the alkanoyl part has from 2 to 6 carbon atoms; or
   an aralkyloxycarbonyl group, in which the aryl part is as defined above, and the alkyl part has from 1 to 4 carbon atoms;
R² represents a methyl, ethyl, methoxy or ethoxy group;
R³ represents:
   a pyridyl group which is unsubstituted or is substituted by at least one substituent selected from substituents (a''), defined below; or
   a quinolyl group which is unsubstituted or is substituted by at least one substituent selected from substituents (a''), defined below;
R⁴ represents a hydrogen atom or a tri-substituted silyl group, in which all three or two or one of the substituents are alkyl groups having from 1 to 5 carbon atoms, and none, one or two of the substituents are aryl groups, as defined above; and
Z represents a sulphur atom;
said substituents (a'') are selected from methyl, ethyl, methoxy and ethoxy groups.

Of these, the most preferred class of compounds of the present invention are those compounds of formulae (I) and (Ia), in which:
R¹ represents:
   a hydrogen atom;
   a tri-substituted silyl group, in which all three or two or one of the substituents are alkyl groups having from 1 to 5 carbon atoms, and none, one or two of the substituents are aryl groups, as defined above;
   an aliphatic acyl group having from 1 to 6 carbon atoms;
   a halogenated alkanoyl group having from 2 to 6 carbon atoms;
   an alkoxyalkanoyl group in which the alkoxy part has from 1 to 5 carbon atoms and the alkanoyl part has from 2 to 6 carbon atoms; or
   an aralkyloxycarbonyl group, in which the aryl part is as defined above, and the alkyl part has from 1 to 4 carbon atoms;
R² represents a methyl or ethyl group;
R³ represents:
   a pyridyl group which is unsubstituted or is substituted by at least one methyl group; or
   a quinolyl group which is unsubstituted or is substituted by at least one methyl group;
R⁴ represents a hydrogen atom or a tri-substituted silyl group, in which all three or two or one of the substituents are alkyl groups having from 1 to 5 carbon atoms, and none, one or two of the substituents are aryl groups, as defined above; and
Z represents a sulphur atom.

An alternative preferred class of compounds of the present invention are those compounds of formulae (I) and (Ia), in which:
R¹ represents a hydrogen atom or a hydroxy-protecting group;
R² represents a methyl group;
R³ represents a phenyl group which has a substituent of formula -CYNR⁵R⁶ and no further substituent or has at least one substituent selected from substituents (a'), defined below, where
   Y represents an oxygen or sulphur atom; and
   R⁵ and R⁶ are independently selected from alkyl groups having from 1 to 6 carbon atoms, aryl groups as defined above, and aralkyl groups in which the alkyl part has from 1 to 6 carbon atoms and the aryl part is as defined above, or
   R⁵ and R⁶ together form a group of formula -(CH₂)ₘ-(X)_{P}-(CH₂)ₙ-, wherein
      m and n are independently selected from the cipher 0 and integers from 1 to 5, provided that (m + n) is greater than 1, ℓ is 0 or 1, and X represents an oxygen or sulphur atom or a group of formula =NR⁷, where R⁷ represents an alkyl group having from 1 to 6 carbon atoms, an aliphatic carboxylic acyl group having from 1 to 6 carbon atoms or an aromatic carboxylic acyl group in which the aryl part is as defined above;
R⁴ represents a hydrogen atom or an amino-protecting group; and
Z represents a sulphur atom;
said substituents (a') are selected from alkyl groups having from 1 to 6 carbon atoms, alkoxy groups having from 1 to 6 carbon atoms and halogen atoms.

An alternative more preferred class of compounds of the present invention are those compounds of formulae (I) and (Ia), in which:
R¹ represents:
   a hydrogen atom; a tri-substituted silyl group, in which all three or
   two or one of the substituents are alkyl groups having from 1 to 5 carbon atoms, and none, one or two of the substituents are aryl groups, as defined above;
   an aliphatic acyl group having from 1 to 6 carbon atoms;
   a halogenated alkanoyl group having from 2 to 6 carbon atoms;
   an alkoxyalkanoyl group in which the alkoxy part has from 1 to 5 carbon atoms and the alkanoyl part has from 2 to 6 carbon atoms; or
   an aralkyloxycarbonyl group, in which the aryl part is as defined above, and the alkyl part has from 1 to 4 carbon atoms;
R² represents a methyl group;
R³ represents a phenyl group which has a substituent of formula -CONR⁵R⁶ and no further substituent or has at least one substituent selected from substituents (a''), defined below, where
   R⁵ and R⁶ are independently selected from alkyl groups having from 1 to 3 carbon atoms, phenyl groups which are unsubstituted or are substituted by at least one substituent selected from substituents (a''), defined below, and aralkyl groups in which the alkyl part has 1 or 2 carbon atoms and the aryl part is a phenyl group which is unsubstituted or is substituted by at least one substituent selected from substituents (a''), defined below, or
   R⁵ and R⁶ together form a group of formula -(CH₂)ₘ-(X)_{P}-(CH₂)ₙ-, wherein
      m and n are independently selected from the cipher 0 and integers from 1 to 5, provided that (m + n) is greater than 1, ℓ is 0 or 1, and X represents an oxygen or sulphur atom or a group of formula =NR⁷, where R⁷ represents a methyl group, an ethyl group, an aliphatic carboxylic acyl group having from 2 to 4 carbon atoms or a benzoyl group which is unsubstituted or is substituted by at least one substituent selected from substituents (a''), defined below;
R⁴ represents a hydrogen atom or a tri-substituted silyl group, in which all three or two or one of the substituents are alkyl groups having from 1 to 5 carbon atoms, and none, one or two of the substituents are aryl groups, as defined above; and
Z represents a sulphur atom;
said substituents (a'') are selected from methyl, ethyl, methoxy and ethoxy groups.

An alternative most preferred class of compounds of the present invention are those compounds of formulae (I) and (Ia), in which:
R¹ represents:
   a hydrogen atom;
   a tri-substituted silyl group, in which all three or two or one of the substituents are alkyl groups having from 1 to 5 carbon atoms, and none, one or two of the substituents are aryl groups, as defined above;
   an aliphatic acyl group having from 1 to 6 carbon atoms;
   a halogenated alkanoyl group having from 2 to 6 carbon atoms;
   an alkoxyalkanoyl group in which the alkoxy part has from 1 to 5 carbon atoms and the alkanoyl part has from 2 to 6 carbon atoms; or
   an aralkyloxycarbonyl group, in which the aryl part is as defined above, and the alkyl part has from 1 to 4 carbon atoms;
R² represents a methyl group;
R³ represents a phenyl group which has a substituent of formula -CONR⁵R⁶ and no further substituent or has at least one substituent selected from methyl and methoxy groups, where
   R⁵ and R⁶ are independently selected from alkyl groups having from 1 to 3 carbon atoms, or
   R⁵ and R⁶ together form a group of formula
      -(CH₂)₄-
      -(CH₂)₅-
      -(CH₂)₆- or
      -(CH₂)₂-O-(CH₂)₂-;
R⁴ represents a hydrogen atom; and
Z represents a sulphur atom.

The compounds of the present invention necessarily contain several assymetric carbon atoms and can, therefore, form optical isomers, including, for example, that shown above as formula (Ia). In addition, depending upon the nature of the various substituent groups, other optical and geometric isomers may be possible. Although all such isomers are shown herein by a single formula, the present invention embraces the use of all such isomers as well as mixtures thereof. Where a mixture of the compounds of the present invention is obtained, these may be separated by conventional resolution techniques. Alternatively, in appropriate cases, a mixture of isomers may be used. However, it should be remembered that it is an advantage of the present invention that the desired 1β-isomer can be obtained readily and in a good yield.

Specific examples of compounds of the present invention are shown in the following formulae (I-1) to (I-3), in which the various substituent groups are as defined in the corresponding one of Tables 1 to 3, i.e Table 1 relates to formula (I-1), Table 2 relates to formula (I-2) and Table 3 relates to formula (I-3). In the Tables, the following abbreviations are used:
- Ac: acetyl
- Boz: benzoyl
- Bu: butyl
- iBu: isobutyl
- tBu: t-butyl
- Bz: benzyl
- Et: ethyl
- Me: methyl
- Ph: phenyl
- Pn: pentyl
- Pr: propyl
- iPr: isopropyl
- Prn: propionyl
- Pyr: pyridyl
- Quin: quinolyl

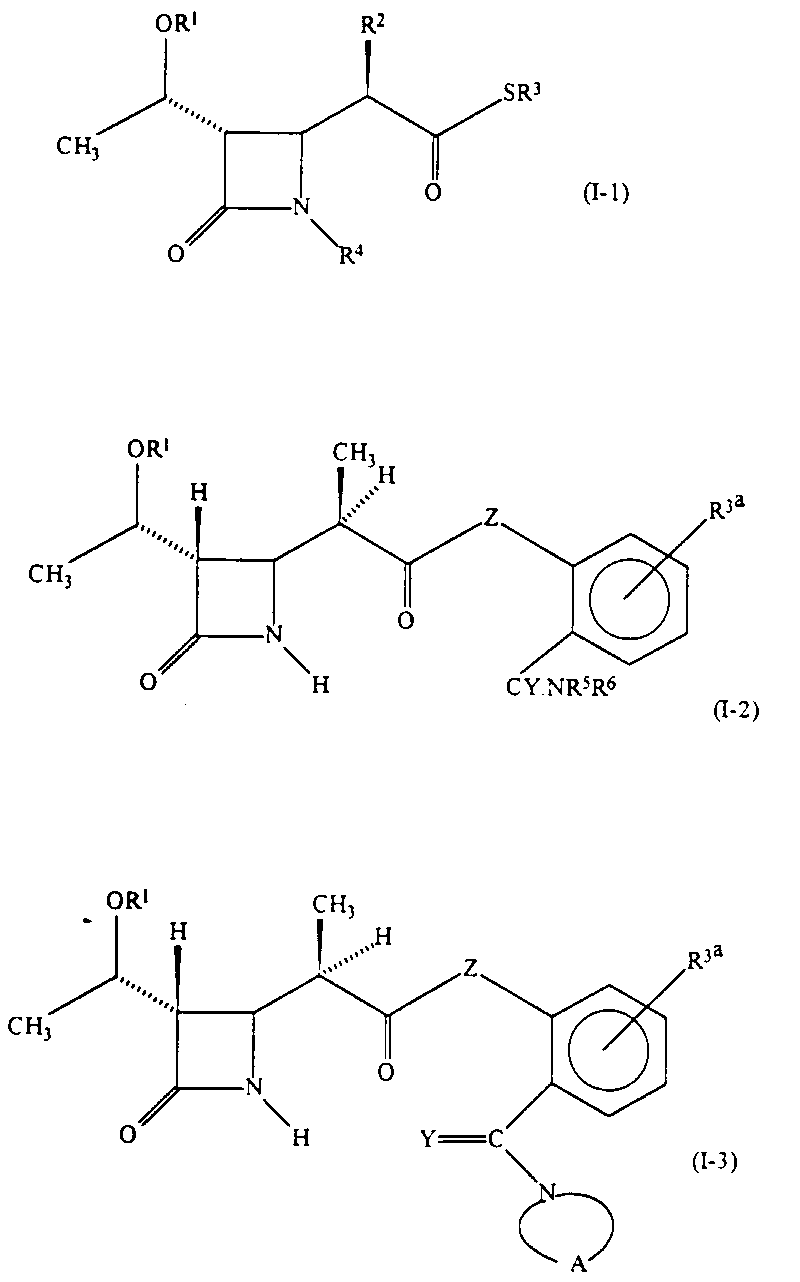

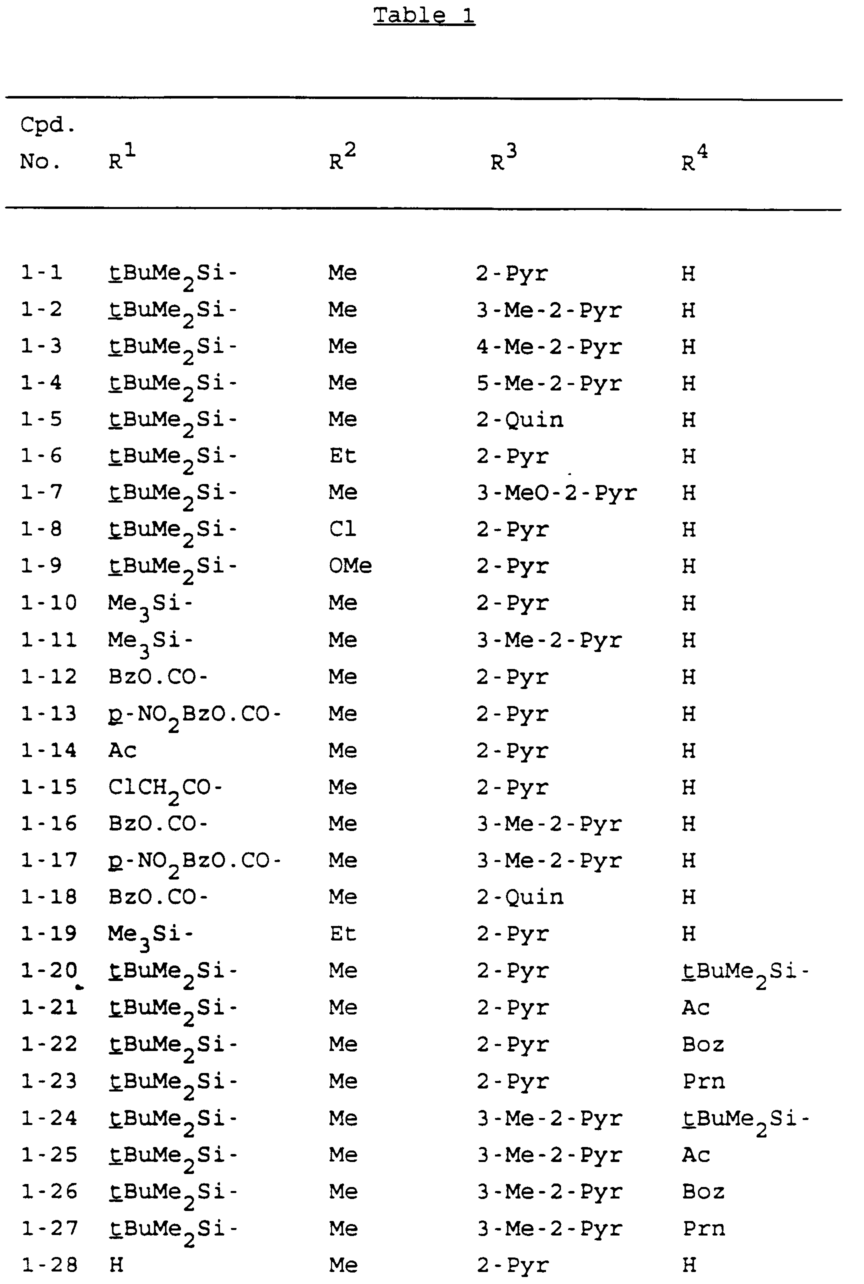

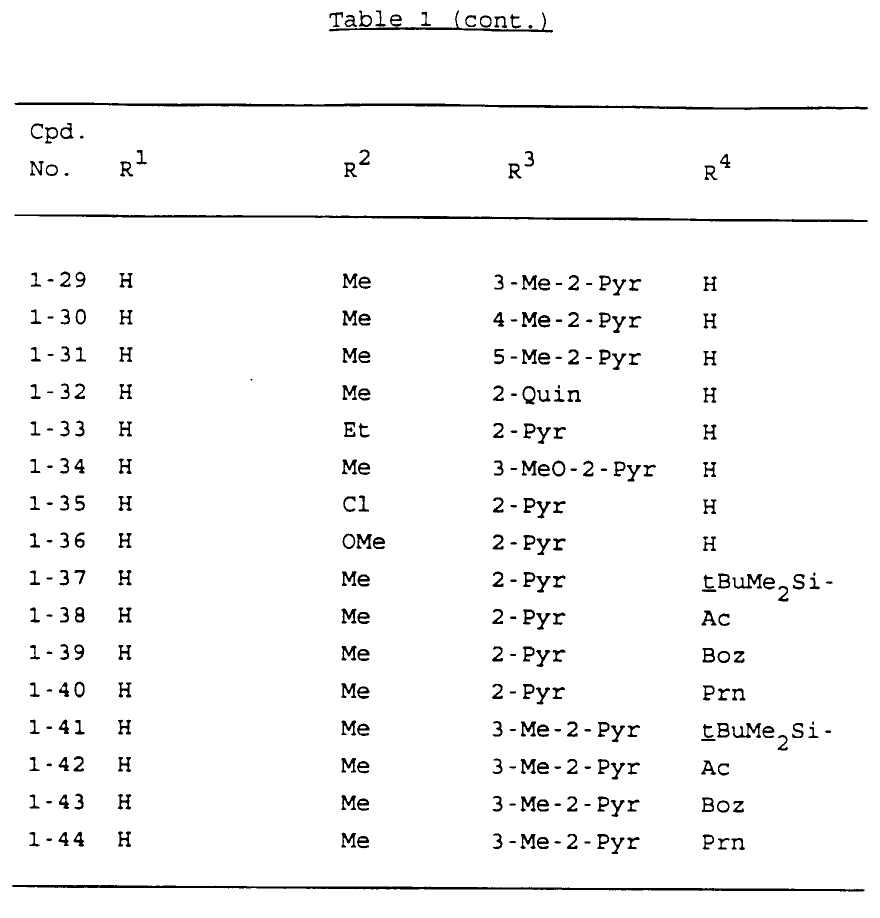

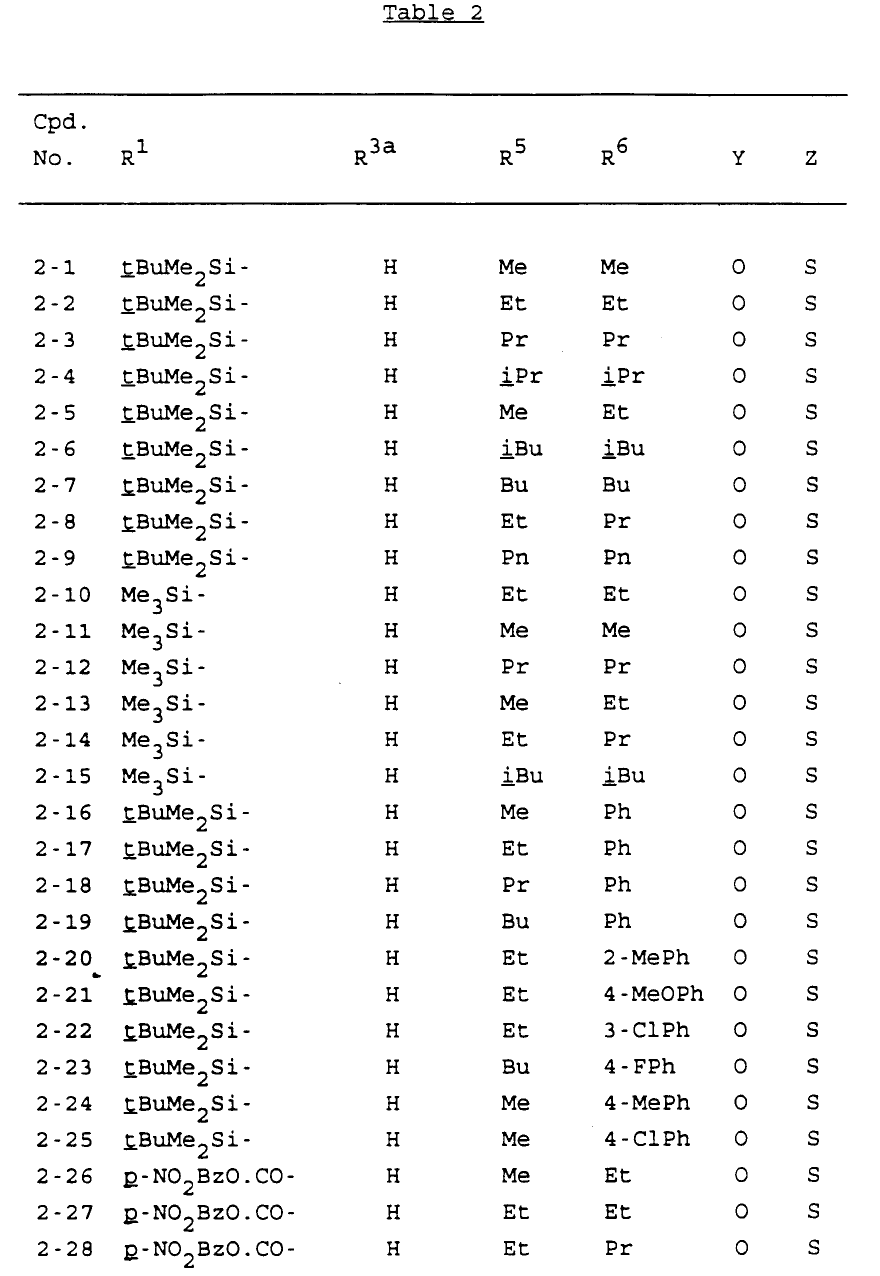

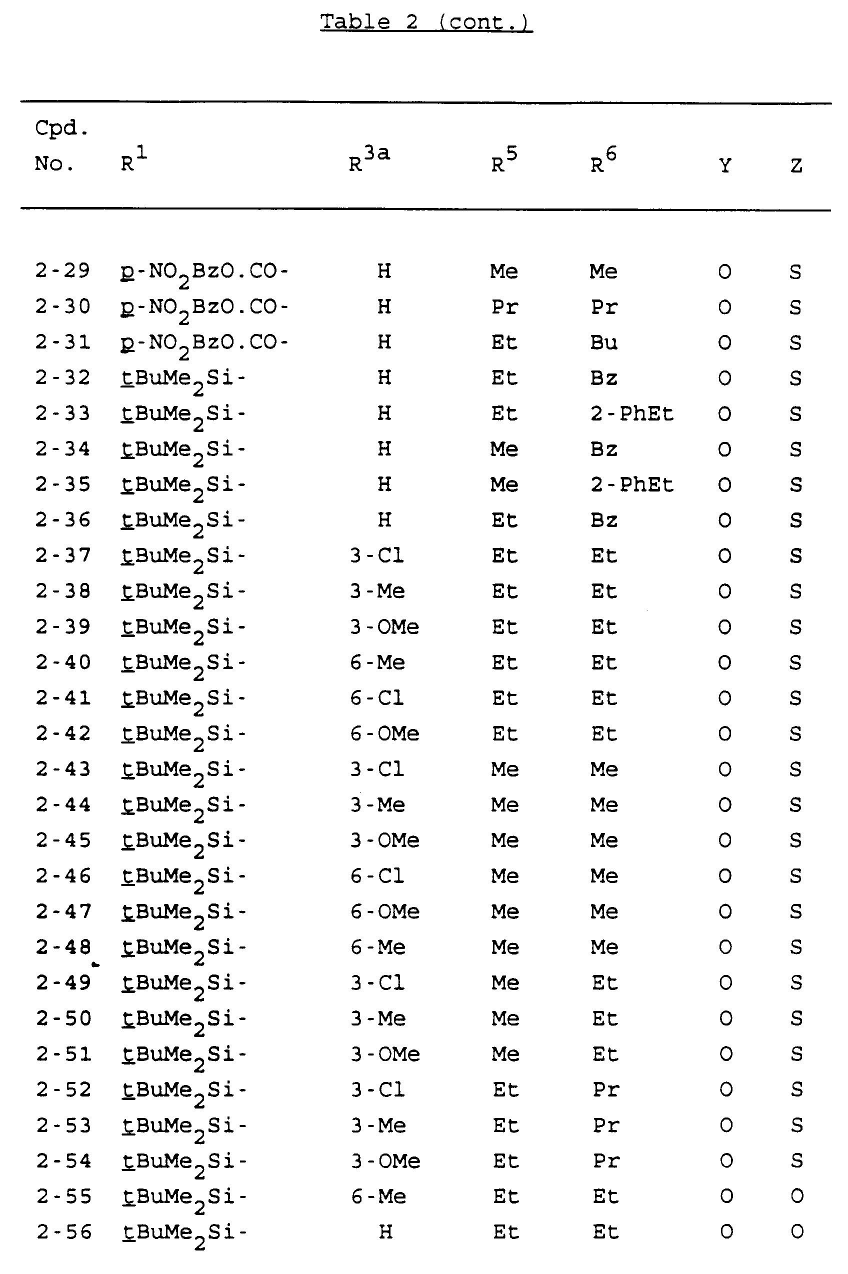

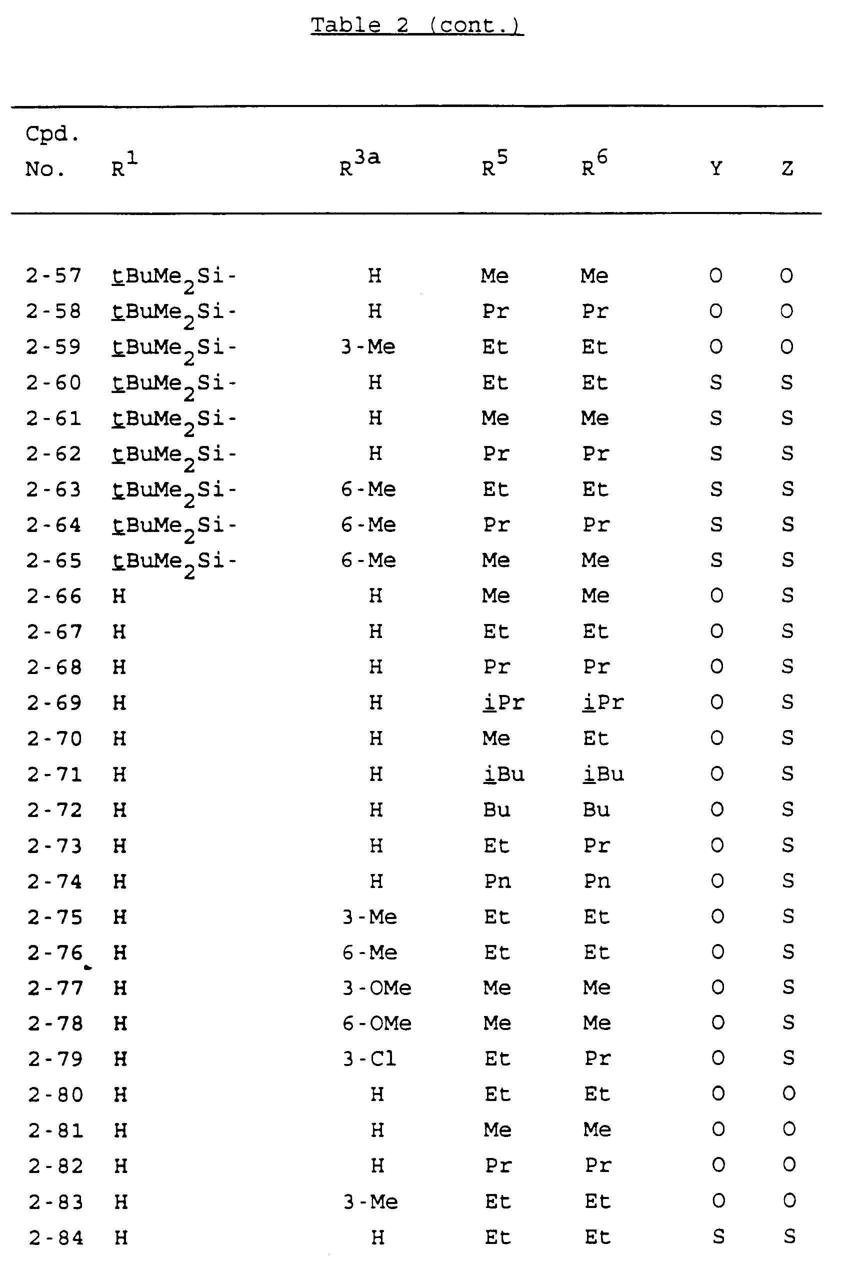

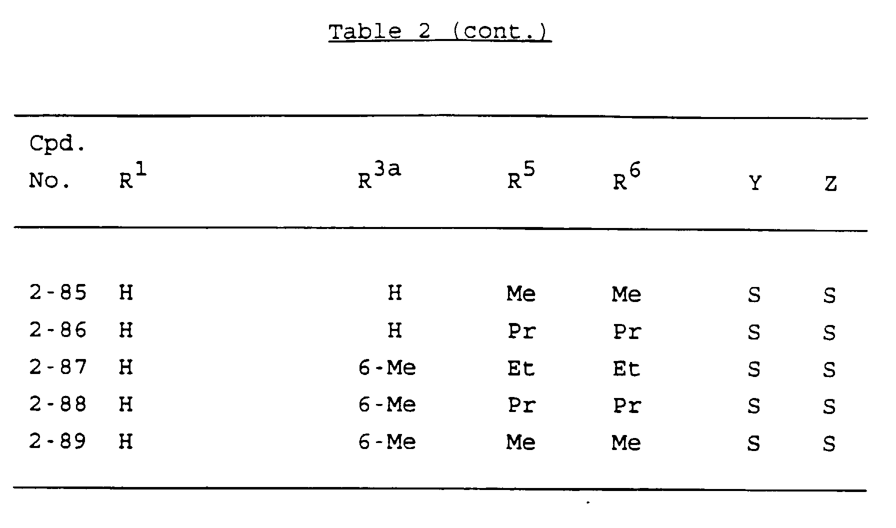

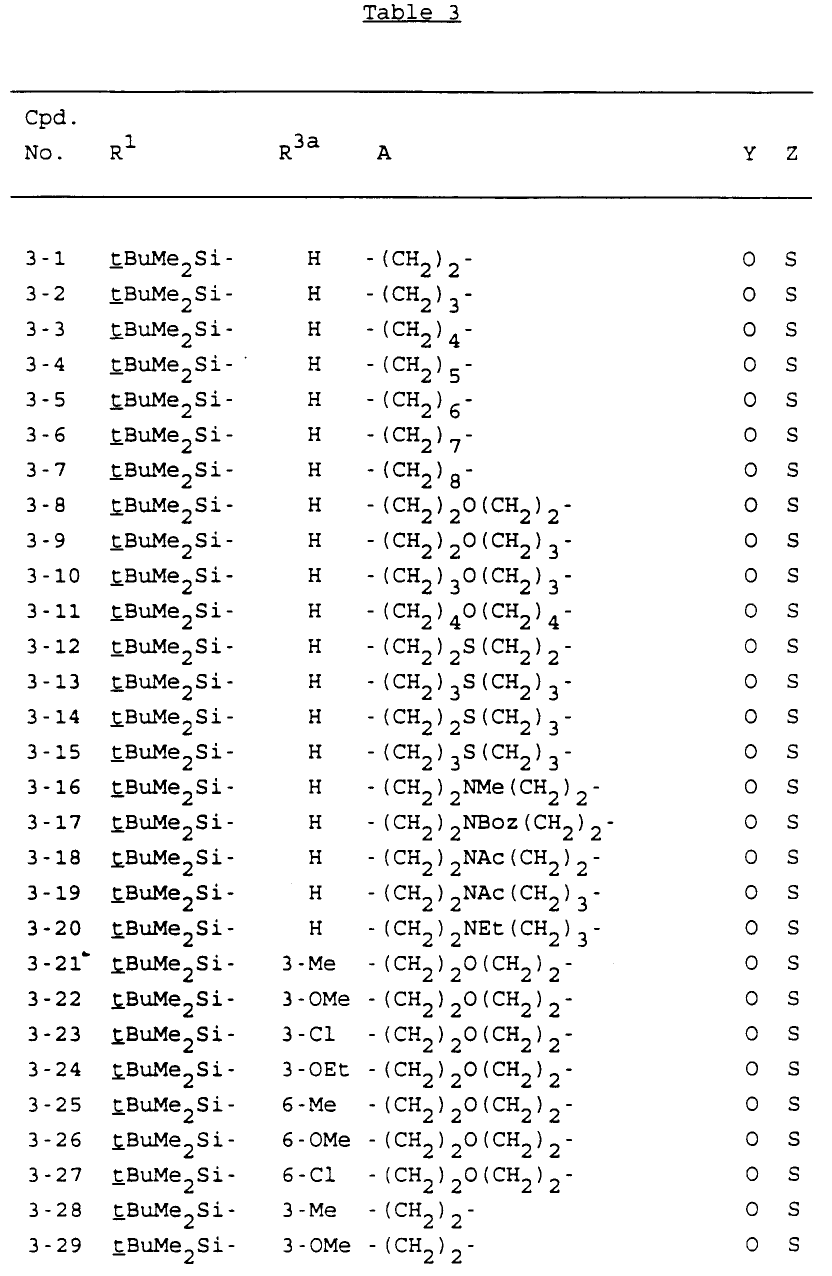

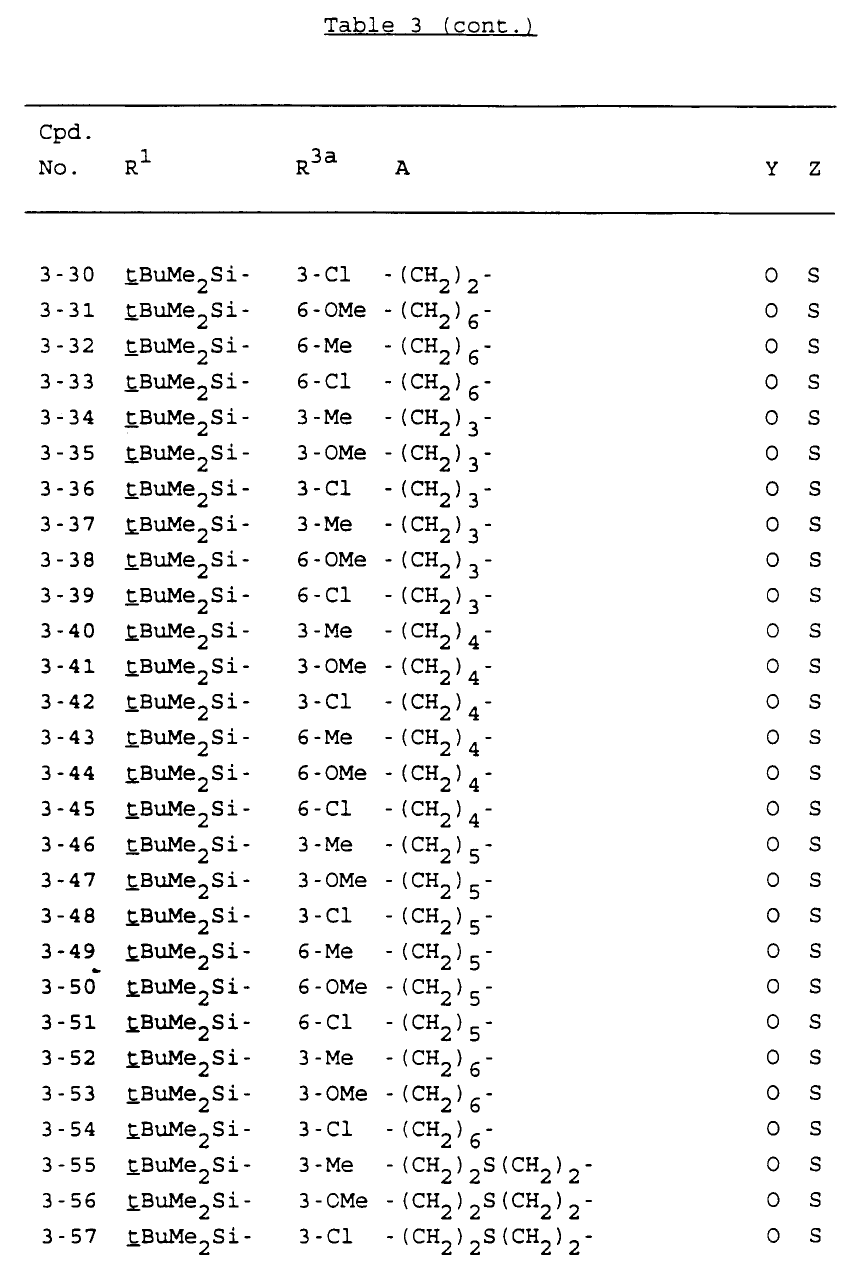

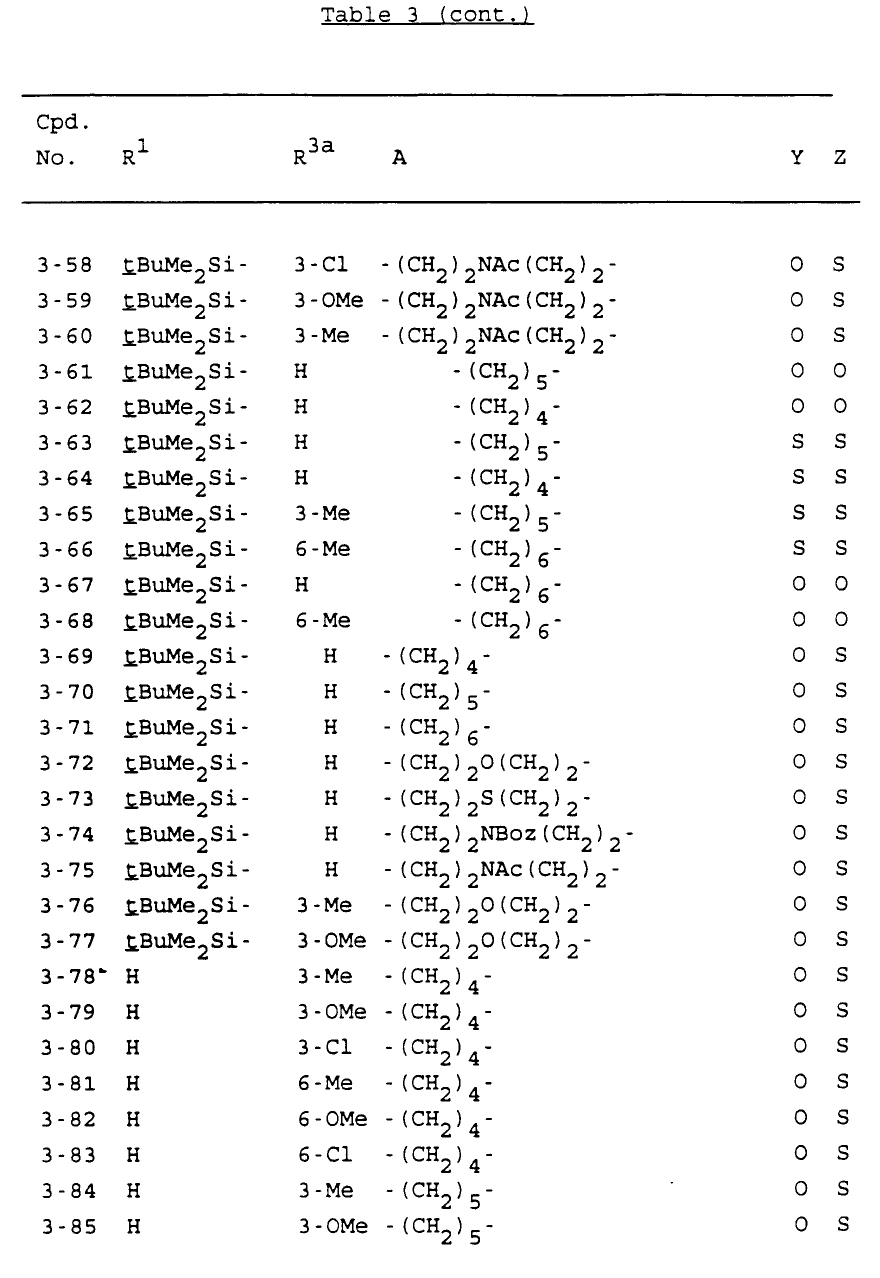

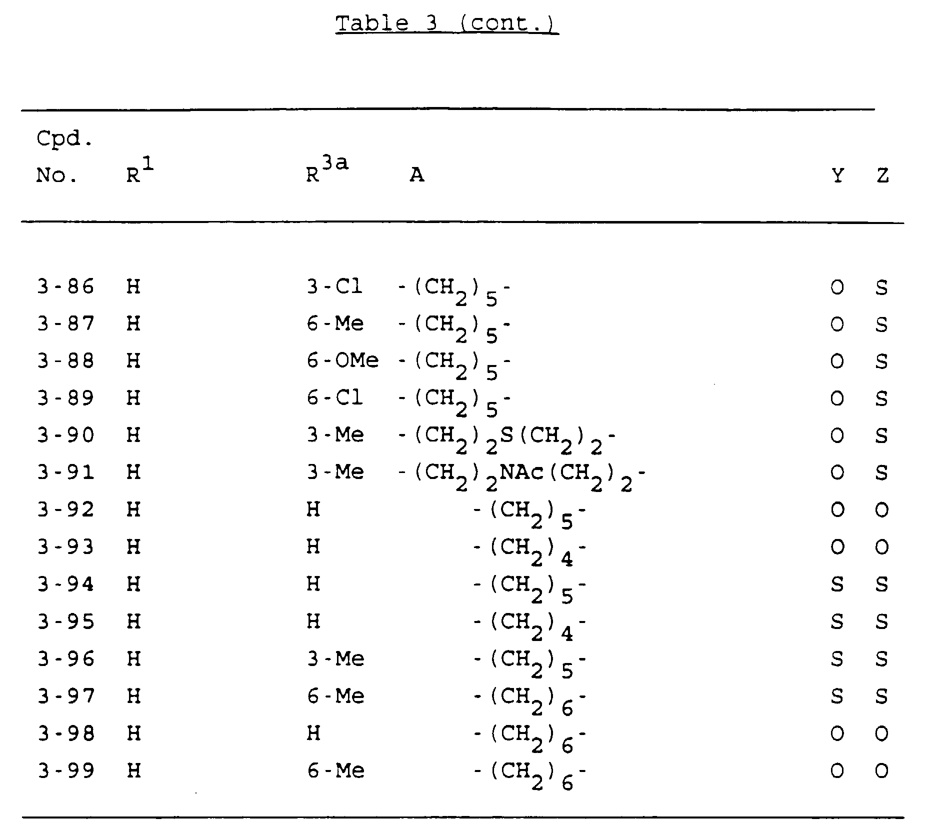

Of the compounds listed above, the following are preferred, that is to say Compounds No. 1-1, 1-5, 1-10, 1-13, 1-14, 1-16, 1-20, 1-22, 1-24, 1-26, 1-28, 1-32, 1-37, 1-39, 1-41, 1-43, 2-1, 2-2, 2-3, 2-6, 2-16, 2-26, 2-27, 2-28, 2-29, 2-30, 2-31, 2-37, 2-38, 2-39, 2-40, 2-41, 2-42, 2-55, 2-56, 2-59, 2-60, 2-63, 2-66, 2-68, 2-69, 2-70, 2-71, 2-75, 3-3, 3-4, 3-5, 3-8, 3-21, 3-25, 3-46, 3-47, 3-48, 3-49, 3-50, 3-51, 3-61, 3-63, 3-65, 3-69, 3-70, 3-71, 3-72, 3-76, 3-84, 3-85, 3-86, 3-87, 3-88, 3-89, 3-92, 3-94 and 3-96, and the following are more preferred, that is to say Compounds No. 1-1, 1-5, 1-10, 1-13, 1-20, 1-22, 1-24, 1-26, 1-28, 1-32, 1-37, 1-39, 1-41, 1-43, 2-1, 2-2, 2-3, 2-6, 2-16, 2-26, 2-27, 2-28, 2-29, 2-30, 2-31, 2-40, 2-56, 2-60, 2-63, 2-66, 2-68, 2-69, 2-70, 2-71, 3-3, 3-4, 3-5, 3-8, 3-21, 3-25, 3-61, 3-63, 3-65, 3-69, 3-70, 3-71 and 3-72. The most preferred specific compounds of the present invention are Compounds No.:
1-1. (3S,4S)-3[(1R)-1-t-Butyldimethylsilyloxyethyl]-4-[(1R)-(2-pyridylthiocarbonyl)ethyl]azetidin-2-one;
1-5. (3S,4S)-3-[(1R)-1-t-Butyldimethylsilyloxyethyl]-4-[(1R)-(2-quinolinethiocarbonyl)ethyl]azetidin-2-one;
1-10. (3S,4S)-3[(1R)-1-Trimethylsilyloxyethyl]-4-[(1R)-(2-pyridylthiocarbonyl)ethyl]azetidin-2-one;
1-13. (3S,4S)-3[(1R)-1-p-Nitrobenzyloxycarbonyloxyethyl]-4-[(1R)-(2-pyridylthiocarbonyl)ethyl]azetidin-2-one;
1-28. (3S,4S)-3[(1R)-1-Hydroxyethyl]-4-[(1R)-(2-pyridylthiocarbonyl)ethyl]azetidin-2-one;
1-32. (3S,4S)-3-[(1R)-1-Hydroxyethyl]-4-[(1R)-(2-quinolinethiocarbonyl)ethyl]azetidin-2-one;
2-2. S-2-Diethylcarbamoylphenyl 2(R)-{(3S,4S)-3-[1(R)-(t-butyldimethylsilyloxy)ethyl]-2-oxo-4-azetidinyl}thiopropionate;
2-3. S-2-Dipropylcarbamoylphenyl 2(R)-{(3S,4S)-3-[1(R)-(t-butyldimethylsilyloxy)ethyl]-2-oxo-4-azetidinyl}thiopropionate;
2-56. 2-Diethylcarbamoylphenyl 2(R)-{(3S,4S)-3-[1(R)-(t-butyldimethylsilyloxy)ethyl]-2-oxo-4-azetidinyl}propionate;
2-60. S-2-[Diethyl(thiocarbamoyl)]phenyl 2(R)-{(3S,4S)-3-[1(R)-(t-butyldimethylsilyloxy)ethyl]-2-oxo-4-azetidinyl}thiopropionate;
2-67. S-2-Diethylcarbamoylphenyl 2(R)-{(3S,4S)-3-[1(R)-hydroxyethyl]-2-oxo-4-azetidinyl}thiopropionate;
3-3. S-2-(1-Pyrrolidinylcarbonyl)phenyl 2(R)-{(3S,4S)-3-[1(R)-(t-butyldimethylsilyloxy)ethyl]-2-oxo-4-azetidinyl}thiopropionate;
3-4. S-2-(1-Piperidylcarbonyl)phenyl 2(R)-{(3S,4S)-3-[1(R)-(t-butyldimethylsilyloxy)ethyl]-2-oxo-4-azetidinyl}thiopropionate;
3-5. S-2-(1-Azepinylcarbonyl)phenyl 2(R)-{(3S,4S)-3-[1(R)-(t-butyldimethylsilyloxy)ethyl]-2-oxo-4-azetidinyl}thiopropionate;
3-8. S-2-Morpholinocarbonylphenyl 2(R)-{(3S,4S)-3-[1(R)-(t-butyldimethylsilyloxy)ethyl]-2-oxo-4-azetidinyl}thiopropionate.

The compounds of the present invention can be prepared by a variety of methods, whose general techniques are known in the art for the preparation of compounds of this type. For example, they may be prepared by reacting a compound of formula (II): 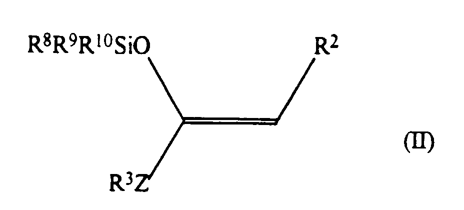 (in which R², R³ and Z are as defined above, and R⁸, R⁹ and R¹⁰ are the same or different and each represents an alkyl group having from 1 to 4 carbon atoms or a phenyl group) with a compound of formula (III): 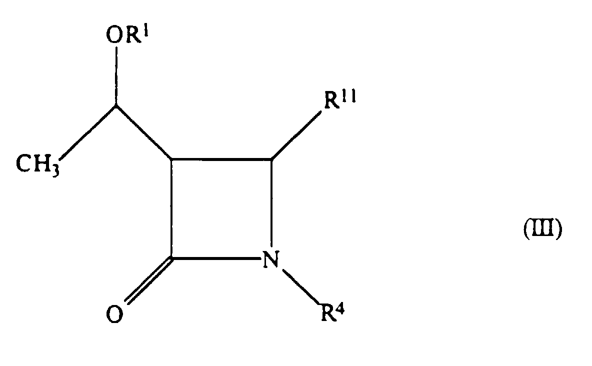 (in which R¹ and R⁴ are as defined above, and R¹¹ represents an acyloxy, alkylsulphonyl, arylsulphonyl, alkylsufinyl or arylsulphinyl group, all of which are as more precisely defined and exemplified below).

More preferably, the compound of formula (III) has the configuration shown in formula (IIIa): 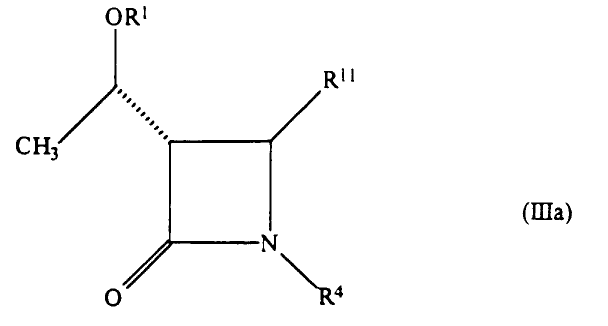 to produce a compound of formula (Ia):

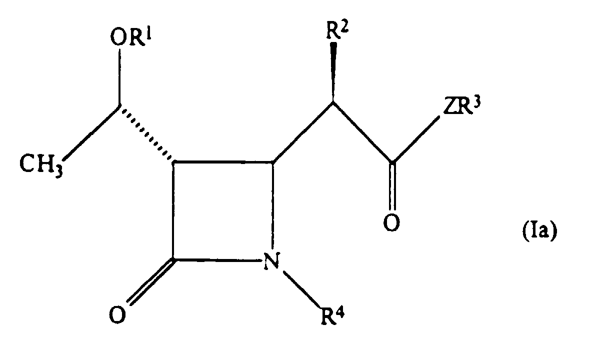

Examples of the alkyl groups which may be represented by R⁸, R⁹ and R¹⁰ include the methyl, ethyl, propyl, isopropyl, butyl, sec-butyl and t-butyl groups. Examples of preferred groups of formula SiR⁸R⁹R¹⁰ include the t-butyldimethylsilyl, trimethylsilyl, triethylsilyl, triisopropylsilyl and t-butyldiphenylsilyl groups.

The acyloxy groups which may be represented by R¹¹ are carboxylic acyloxy groups which may be aliphatic or aromatic. In the case of the aliphatic acyloxy groups, these preferably have from 1 to 6, more preferably from 2 to 4, carbon atoms and may be alkanoyloxy, haloalkanoyloxy or alkenoyloxy groups, the alkanoyloxy groups being preferred. Examples of such groups include the acetoxy, propionyloxy and butyryloxy groups. In the case of the aromatic acyloxy groups, these are arylcarbonyloxy groups and the aryl part may be as defined and exemplified above. A preferred such group is the benzoyloxy group. In the case of the alkylsulphonyl and alkylsulphinyl groups, the alkyl parts have from 1 to 6, preferably from 1 to 4, carbon atoms and examples of the alkyl moieties of such groups include the methyl, ethyl, propyl and isopropyl groups. In the case of the arylsulphonyl and arylsulphinyl groups, the aryl part may be as defined and exemplified above, and examples of the aryl moieties of such groups include the phenyl and p-tolyl groups. Preferably R¹¹ represents an acetoxy, benzoyloxy, phenylsulphonyl, phenylsulphinyl, tolylsulphinyl or methylsulphinyl group.

In this reaction, a silyl enol ether of formula (II) is reacted with an azetidinone derivative of formula (III). This reaction normally and preferably takes place in the presence of a solvent and in the presence of a Lewis acid.

Examples of Lewis acids which may be used in the reaction include: zinc chloride, zinc bromide, zinc iodide and boron trifluoride etherate. Of these, we prefer to use zinc chloride or zinc iodide.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, especially halogenated aliphatic hydrocarbons, such as methylene chloride, chloroform and 1,2-dichloroethane; ethers such as tetrahydrofuran and 1,2-dimethoxyethane; and nitriles, such as acetonitrile. Of these, we prefer to use methylene chloride, chloroform or 1,2-dichloroethane.

The reaction can take place over a wide range of temperatures, and the precise reaction temperature is not critical to the present invention. In general, we find it convenient to carry out the reaction at a temperature of from -10°C to 70°C and more preferably from 10°C to 50°C. The time required for the reaction may also vary widely, depending on many factors, notably the reaction temperature and the nature of the reagents and solvent employed. However, provided that the reaction is effected under the preferred conditions outlined above, a period of from 10 minutes to 24 hours will usually suffice.

The compound of formula (I) thus obtained can be recovered from the reaction mixture by any conventional method. For example, one suitable recovery procedure comprises adding a solvent, such as methylene chloride or ethyl acetate, to the reaction mixture, separating and washing the organic layer with water and finally separating the desired compound by suitable means, such as thin layer chromatography or flash chromatography through silica gel, or purifying it by means of crystallisation or recrystallisation.

The silyl enol ether of formula (II), used as a starting material in this reaction, can be prepared by reacting a compound of formula (IV):

R²CH₂.CO.ZR³ (IV)

(in which R², R³ and Z are as defined above) with an active silyl compound of formula (V):

R⁸R⁹R¹⁰SiW (V)

(in which R⁸, R⁹ and R¹⁰ are as defined above, and W represents a leaving group, for example a halogen atom, such as a chlorine atom, or a sulphonyloxy group, such as a trifluoromethanesulphonyloxy group) in the presence of a base.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: ethers such as diethyl ether, 1,2-dimethoxyethane and tetrahydrofuran; and hydrocarbons, especially aliphatic hydrocarbons, such as hexane or cyclohexane; mixtures of of any two or more of these solvents may also be employed.

The yield of the reaction can be improved by adding one or more of the following solvents to one or more of the solvents listed above. Examples of such additional solvents include: hexamethylphosphoric triamide (HMPA), N,N-dimethylformamide, N,N-dimethylacetamide, N,N'-dimethylpropyleneurea, N-methylpyrrolidone and N-methylpiperidone.

Examples of bases which may be used in the reaction include lithium, sodium or potassium salts of diisopropylamine, hexamethyldisilazane, dicyclohexylamine or 2,2,6,6-tetramethylpiperidine.

In some cases, the addition of one or more additional bases as well as the bases listed above will improve the yield of the compound of formula (II). Examples of such bases include tertiary amines, such as triethylamine.

The reaction can take place over a wide range of temperatures, and the precise reaction temperature is not critical to the present invention. In general, we find it convenient to carry out the reaction at a temperature of from -90°C to 20°C and more preferably from -78°C to -20°C. The time required for the reaction may also vary widely, depending on many factors, notably the reaction temperature and the nature of the reagents and solvent employed. However, provided that the reaction is effected under the preferred conditions outlined above, a period of from 5 minutes to 4 hours and more preferably from 10 minutes to 30 minutes will usually suffice.

The resulting compound of formula (II) can then be recovered from the reaction mixture by conventional means. For example, it can be recovered in a good yield by adding water, a saturated aqueous solution of sodium hydrogencarbonate or triethylamine to the reaction mixture, extracting the mixture with an organic solvent and then purifying it by means of column chromatography or distillation.

Where R³ represents a phenyl group, R² represents a methyl group and Z represents a sulphur atom, the resulting compound of formula (IV) is a substituted S-phenyl thiopropionate, which can be used as a starting compound in the above reaction. This may be prepared as shown in the following Reaction Schemes A and B:

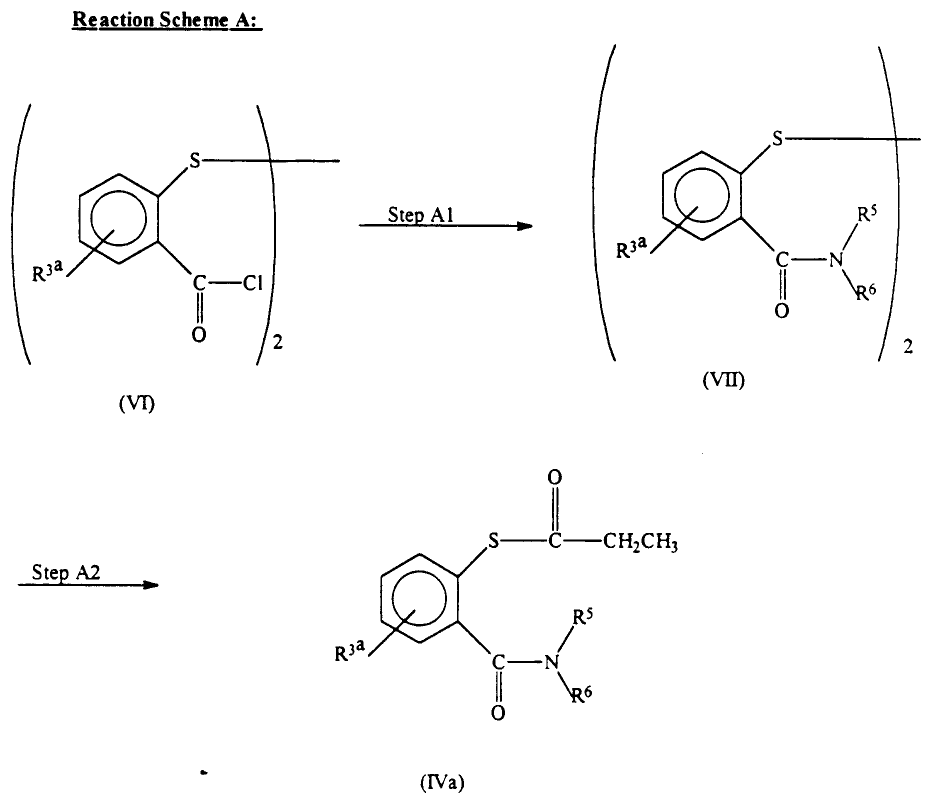

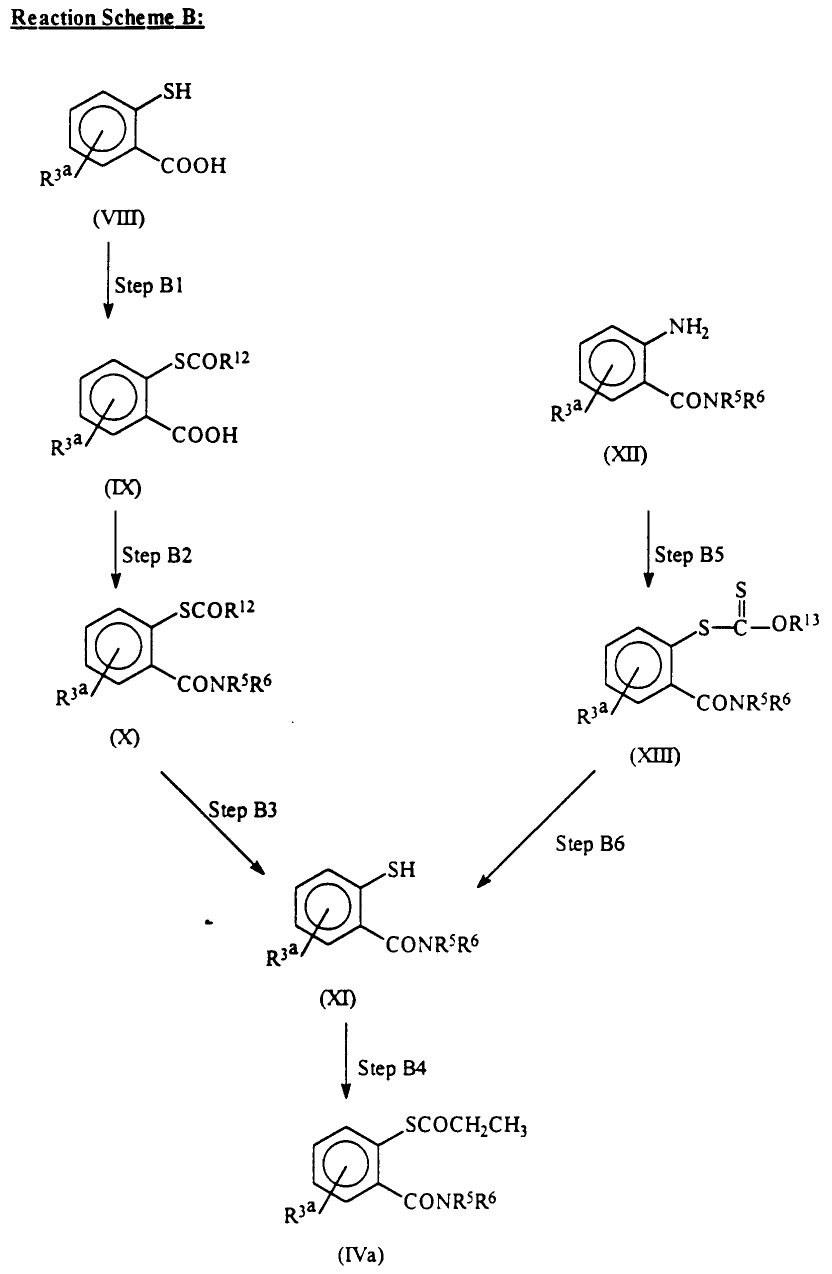

In the above formulae, R^{3a}, R⁵ and R⁶ are as defined above. R¹² represents an aryl group (as defined above), such as a phenyl or tolyl group, and R¹³ represents a lower alkyl group having from 1 to 6 carbon atoms, preferably from 1 to 4 carbon atoms, such as a methyl or ethyl group.

In Reaction Scheme A, a substituted S-phenyl thiopropionate, that is to say a compound of formula (IVa), can be prepared from a compound of formula (VI), which, in turn, can readily be obtained by reacting a 2,2'-dithiobenzoic acid derivative with a halogenating agent, such as thionyl chloride.

In Step A1, a secondary amine, R⁵R⁶NH, is reacted with the compound of formula (VI) in the presence of an organic amine, such as triethylamine, or an inorganic base such, as sodium carbonate, to give the compound of formula (VII). In Step A2, this compound of formula (VII) is reacted with propionic anhydride in the presence of a metal having reducing activity, such as zinc.

Alternatively, in Reaction Scheme B, a compound of formula (IVa) is prepared using a thiosalicylic acid derivative of formula (VIII) as the starting material.

In Step B1 of this reaction scheme, the thiol group of a compound of formula (VIII) is protected by reacting the compound of formula (VIII) with an aromatic acyl halide, such as benzoyl chloride, to give the protected compound of formula (IX).

In Step B2, the compound of formula (IX) is reacted with a secondary amine, R⁵R⁶NH, in the presence of a dehydrating condensing agent, such as 2-chloro-1-methylpyridinium iodide, to produce an amide compound of formula (X).

In Step B3, the aromatic acyl protecting group, R¹²CO, is removed by treating the compound of formula (X) with a base, such as sodium methoxide, to give a compound of formula (XI).

In Step B4, the thiol group of the compound of formula (XI) is propionylated by reacting it with an active derivative of propionic acid, such as propionyl chloride or propionic anhydride, in the presence of a base, to give the desired substituted S-phenyl thiopropionate of formula (IVa).

Alternatively, the compound of formula (XI) can also be prepared from an anthranilic acid derivative of formula (XII) by conventional means (e.g. as disclosed in Organic Syntheses Coll. Vol. III, page 809, the disclosure of which is incorporated herein by reference) via Steps B5 and B6. In Step B5, a compound of formula (XII) is diazotised and then reacted with potassium S-ethyl dithiocarbonate, to give a compound of formula (XIII). In Step B6, this compound of formula (XIII) is hydrolysed with a base, such as potassium hydroxide, to give the compound of formula (XI).

Reaction Scheme B illustrates the preparation of compounds of formula (IV) in which R³ represents a substituted phenyl group, Y represents an oxygen atom and Z represents a sulphur atom, that is a compound of formula (IVa). Corresponding compounds in which Y represents a sulphur atom or Z represents an oxygen atom can be prepared as illustrated in the following Reaction Schemes C and D:

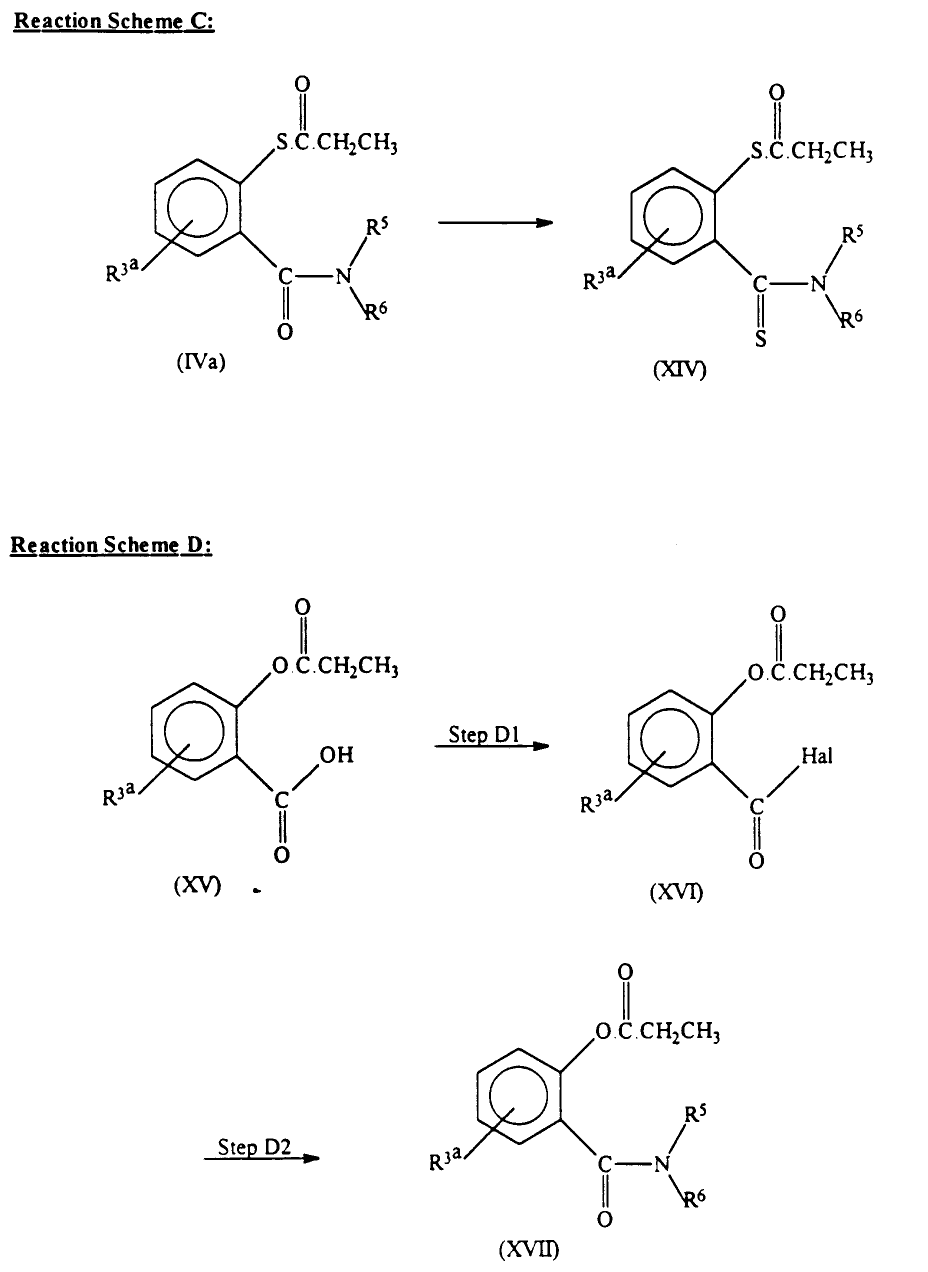

In the above formulae, R^{3a}, R⁵ and R⁶ are as defined above and Hal represents a halogen atom.

In Reaction Scheme C, the carbonyl group forming part of the amido group at the 2-position of the compound of formula (IVa) is converted to a thiocarbonyl group by reaction with a thiolating agent, such as Lawesson's reagent or phosphorus pentasulphide. This reaction is well known in the art and may be carried out using solvents, reaction temperatures and reaction times as are conventional and well known.

In Step D1 of Reaction Scheme D, the carboxy group at the 2-position of the compound of formula (IVa) is halogenated, preferably chlorinated, using a conventional halogenating agent, such as oxalyl chloride, oxalyl bromide, thionyl chloride or thionyl bromide, and using conventional halogenating conditions, to give the compound of formula (XVI). This compound of formula (XVI) is then reacted with a secondary amine of formula R⁵R⁶NH in the presence of an organic tertiary amine, such as triethylamine, or of an inorganic base, such as sodium carbonate, to give the desired compound of formula (XVII).

The azetidinone derivatives of formula (I) in accordance with the present invention can readily be converted to the corresponding carbapenem compounds by reaction with an appropriate mercaptan derivative of formula R¹⁴SH, which may be effected by conventional means (e.g. as described in Japanese Patent Application Kokai No. Sho 60-19764) to produce a compound of formula (XVIII), which can then be cyclised by conventional means (e.g. as described in Japanese Patent Publication No. Sho 62-54427), to give a carbapenem derivative of formula (XIX), as shown in the following Reaction Schemes E and E':

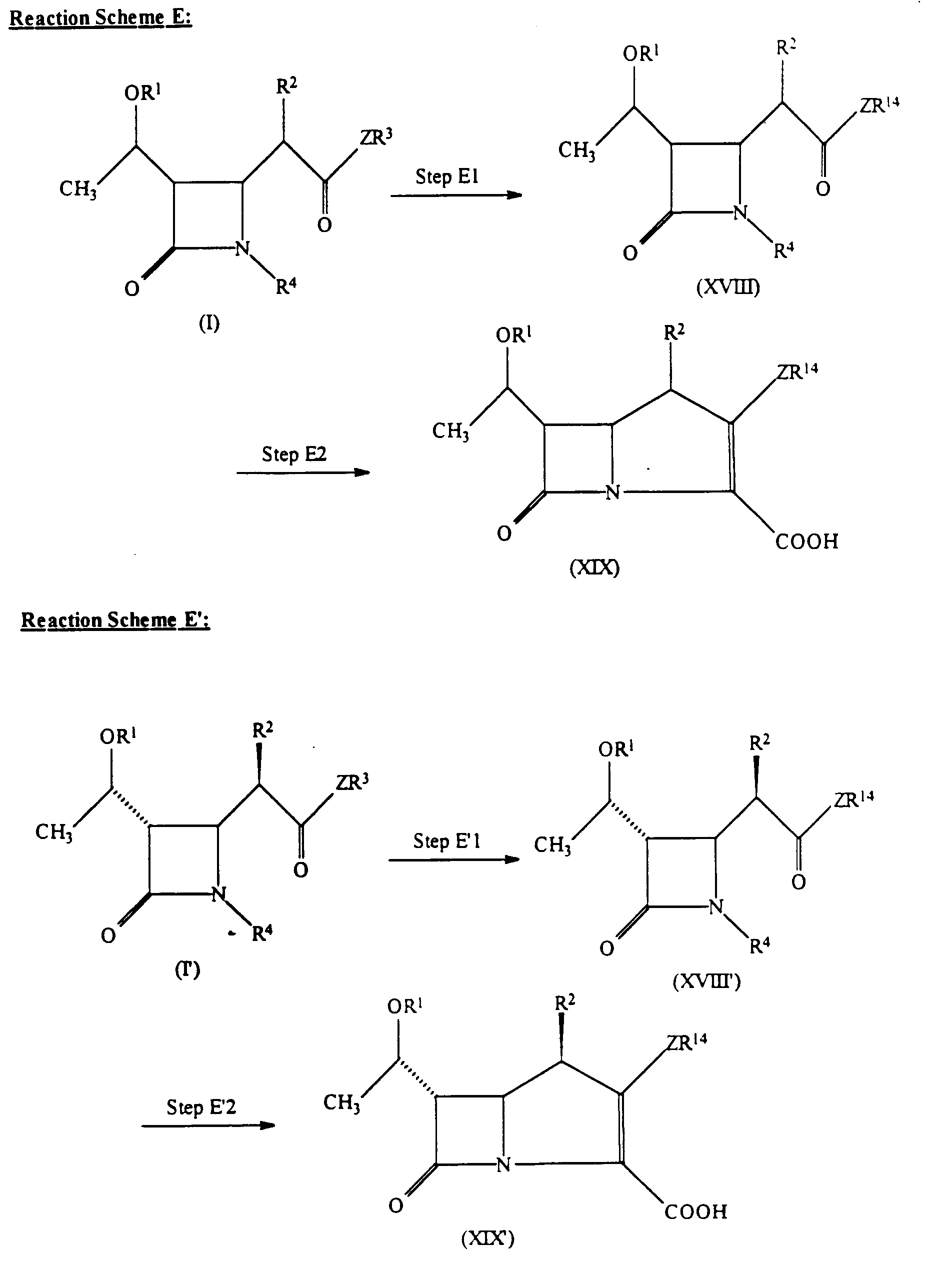

In these formulae, Z, R¹, R², R³ and R⁴ are as defined above and R¹⁴ represents a variety of organic groups of a type commonly employed, at the indicated position, in carbapenem derivatives. As can be seen from Reaction Scheme E', the configuration at the azetidine 3-position and at the carbon atom to which the group represented by R² is attached are conserved, and so this provides a convenient and efficient way of producing these valuable compounds.

In contrast, the compound of formula (C), described in U.S. Patent Specifications No. 4,895,939 and 4,772,683, referred to above, does not appear suitable for this reaction because it does not easily react with a mercaptan of formula R¹⁴SH in the first step of Reaction Scheme E, and cannot, therefore, readily form the compound of formula (XVII). On the other hand, in the reactions described by T. Shibata et al. [Tetrahedron Letters, 26, 4793 (1985)], C. U. Kim et al. [Tetrahedron Letters, 28, 507 (1987)] and A. Martel et al. [Can. J. Chem., 66, 1537 (1988)] the desired 2R-isomer is produced in a relatively low yield in admixture with a large quantity of the unwanted 2S-isomer, and so is difficult and expensive to isolate.

The present invention is further illustrated by the following non-limiting Examples, which illustrate the preparation of the compounds of the present invention. The subsequent Preparations 1 to 12, 19 to 38 and 41 to 43 illustrate the preparation of starting materials for use in these Examples, and Preparations 13 to 18, 39 and 40 illustrate the use of the compounds of the present invention to prepare other compounds, leading ultimately to the desired carbapenem compounds.

### EXAMPLE 1

### (3S,4S)-3[(1R)-1-t-Butyldimethylsilyloxyethyl]-4-[(1R)-(2-pyridylthiocarbonyl)ethyl]azetidin-2-one (Compound No. 1-1)

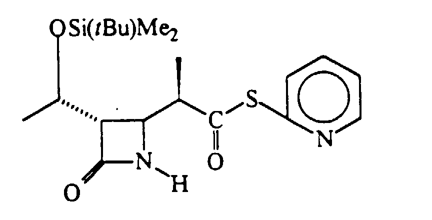

### 1(a) Z(O)-2-(1-t-Butyldimethylsilyloxy-1-propenylthio)pyridine

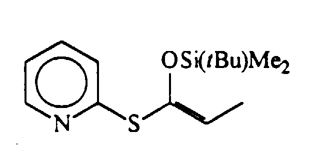

22.5 ml of a solution containing 1.2 equivalents of butyllithium in hexane were added at room temperature to a solution of 7.5 ml of hexamethyldisilazane in 50 ml of tetrahydrofuran, and the resulting mixture was stirred for 30 minutes, to prepare 1.2 equivalents of lithium hexamethyldisilazane. The reaction mixture was then cooled to -78°C, and 6.25 ml (1.2 equivalents) of hexamethylphosphoric triamide, 8.37 ml (2 equivalents) of triethylamine and 9.60 g (2 equivalents) of t-butyldimethylsilyl chloride were added, in that order, followed by a solution of 5 g of 2-propionylthiopyridine (prepared as described in Preparation 1) in 10 ml of tetrahydrofuran. The reaction mixture was then stirred for 10 minutes, after which it was diluted with ethyl acetate. The organic layer was separated, washed twice with water and then dried over anhydrous magnesium sulphate. The solvent was then removed by distillation under reduced pressure, and the residue was subjected to fractional distillation, to give 8.4 g (yield 88%) of the title compound, boiling at 130°C/0.1 mmHg (13.3 Pa).
¹H Nuclear Magnetic Resonance Spectrum (CDCℓ₃, 270 MHz), δ ppm:
   0.09 (6H, singlet);
   0.88 (9H, singlet);
   1.73 (3H, doublet, J = 6.6 Hz);
   5.45 (1H, quartet, J = 6.6 Hz);
   6.97 - 7.02 (1H, multiplet);
   7.32 (1H, doublet, J = 8.6 Hz);
   7.51 - 7.57 (1H, multiplet);
   8.42 (1H, doublet, J = 4 Hz).

This procedure was repeated, except that the hexamethylphosphoric triamide was replaced by the additives shown below. In all cases, the amount of lithium hexamethyldisilazane was 1.2 equivalents, and 2 equivalents each of triethylamine and t-butyldimethylsilyl chloride were employed. The reaction temperature was -78°C. Using 1,3-dimethyl-3,4,5,6-tetrahydro-2(1H)-pyrimidinone as the additive, and carrying out the reaction for 10 minutes, the title compound was obtained in a yield of 88%. Using N,N-dimethylformamide as the additive, and carrying out the reaction for 10 minutes, the title compound was obtained in a yield of 80%. Using N,N-dimethylacetamide as the additive, and carrying out the reaction for 1 hour, the title compound was obtained in a yield of 46%.

### 1(b) (3S,4S)-3[(1R)-1-t-Butyldimethylsilyloxyethyl]-4-[(1R)-(2-pyridylthiocarbonyl)ethyl]azetidin-2-one

163 mg (2 equivalents) of anhydrous zinc chloride (freshly fused) were added to a solution of 171 mg of Z(O)-(3S,4R)-3-[(1R)-1-t-butyldimethylsilyloxyethyl]-4-acetoxyazetidin-2-one and 337 mg (2 equivalents) of 2-(1-t-butyldimethylsilyloxy-1-propenylthio)pyridine [prepared as described in step (a) above] in 15 ml of methylene chloride, and the mixture was stirred at a bath temperature of 12°C for 15 hours. At the end of this time, the reaction mixture was mixed with methylene chloride, and the organic layer was washed three times with water. It was then dried over anhydrous magnesium sulphate, and the solvent was removed by distillation under reduced pressure. The residue was purified by flash chromatography through silica gel, using a 1 : 1 by volume mixture of cyclohexane and ethyl acetate as the eluent, to give 170 mg (yield 72%) of the title compound having an Rf value of 0.2 and melting at 109°C.
Infrared Absorption Spectrum (KBr), νₘₐₓ cm⁻¹:
   1757, 1718, 1696, 1564, 3181, 3099.
¹H Nuclear Magnetic Resonance Spectrum (CDCℓ₃, 270 MHz), δ ppm:
   0.07 (6H, singlet);
   0.87 (9H, singlet);
   1.19 (3H, doublet, J = 5.9 Hz);
   1.35 (3H, doublet, J = 7.2 Hz);
   3.0 - 3.05 (2H, multiplet);
   3.99 (1H, doublet of doublets, J = 1.98 & 5.28 Hz);
   4.19 - 4.23 (1H, multiplet);
   5.90 (1H, singlet);
   7.3 - 7.32 (1H, multiplet);
   7.60 (1H, doublet, J = 7.9 Hz);
   7.73 - 7.93 (1H, multiplet);
   8.63 (1H, doublet, J = 3.9 Hz).

### 1(c) (3S,4S)-3[(1R)-1-t-Butyldimethylsilyloxyethyl]-4-[(1R)-(2-pyridylthiocarbonyl)ethyl]azetidin-2-one

This provides an alternative method of preparing the same compound as was prepared in step (b) above.

200 mg (5.7 mmole) of Z(O)-(3S,4S)-3-[(1R)-1-t-butyldimethylsilyloxyethyl]-4-phenylsulphinylazetidin-2-one (prepared by a procedure similar to that described in Preparation 3) were added to a solution of 160 mg (5.7 mmole) of 2-(1-t-butyldimethylsilyloxy-1-propenylthio)pyridine [prepared as described in step (a) above] in 18 ml of methylene chloride, and the resulting mixture was stirred at 15°C for 4 hours. At the end of this time, the reaction mixture was mixed with methylene chloride, and the organic layer was separated and then washed with water and with a saturated aqueous solution of sodium chloride, in that order. The solution was then dried over anhydrous magnesium sulphate, and the solvent was removed by distillation under reduced pressure. The resulting residue was purified by column chromatography using a 1 : 1 by volume mixture of cyclohexane and ethyl acetate as the eluent, to give 60 mg (yield 28.7%) of a 18 : 1 mixture of the title compound and an isomer therof, in which the methyl group forming part of the ethyl group at the 4-position of the azetidinone ring is in the α-configuration, instead of the β-configuration.

### EXAMPLE 2

### (3S,4S)-3-[(1R)-1-t-Butyldimethylsilyloxyethyl]-4-[(1R)-(2-quinolinethiocarbonyl)ethyl]azetidin-2-one (Compound No. 1-5)

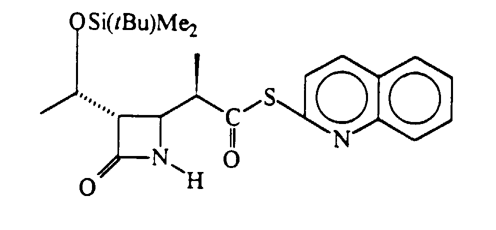

### 2(a) Z(O)-2-(1-t-Butyldimethylsilyloxy-1-propenylthio)quinoline

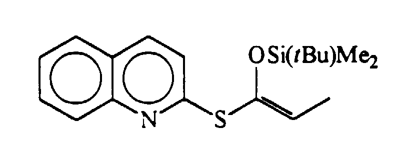

0.9 ml of a solution containing 1.2 equivalents of butyllithium in hexane was added at room temperature to a solution of 0.3 ml of hexamethyldisilazane in 10 ml of anhydrous tetrahydrofuran, and the resulting mixture was stirred for 30 minutes. The reaction mixture was then cooled to -78°C, and 0.25 ml (1.2 equivalents) of hexamethylphosphoric triamide, 0.33 ml (2 equivalents) of triethylamine and 360 mg (2 equivalents) of t-butyldimethylsilyl chloride were added, in that order, to the mixture, followed by 300 mg of 2-propionylthioquinoline (prepared as described in Preparation 2). The reaction mixture was then stirred for 10 minutes, after which it was mixed with ethyl acetate and the organic layer was separated and washed with water. The solvent was removed by distillation under reduced pressure, and the resulting residue was purified by flash column chromatography, using a 10 : 1 by volume mixture of cyclohexane and ethyl acetate as the eluent, to give 320 mg (yield 80%) of the title compound having an Rf value of 0.8.
¹H Nuclear Magnetic Resonance Spectrum (CDCℓ₃, 270 MHz), δ ppm:
   0.10 (6H, singlet);
   0.89 (9H, singlet);
   1.73 (3H, doublet, J = 6.9 Hz);
   5.53 (1H, quartet, J = 6.9 Hz);
   7.43 - 7.50 (2H, multiplet);
   7.66 - 7.73 (1H, multiplet);
   7.74 (1H, doublet, J = 8 Hz);
   7.96 (1H, doublet, J = 8.6 Hz);
   8.00 (1H, doublet, J = 8.6 Hz).

### 2(b) (3S,4S)-3-[(1R)-1-t-Butyldimethylsilyloxyethyl]-4-[(1R)-(2-quinolinethiocarbonyl)ethyl]azetidin-2-one

120 mg (2 equivalents) of anhydrous zinc chloride were added to a solution of 126 mg of Z(O)-(3S,4R)-3-[(1R)-1-t-butyldimethylsilyloxyethyl]-4-acetoxyazetidin-2-one and 320 mg (2 equivalents) of 2-(1-t-butyldimethylsilyloxy-1-propenylthio)quinoline [prepared as described in step (a) above] in 15 ml of anhydrous methylene chloride, and the resulting mixture was stirred at a bath temperature of 28 - 30°C for 3 hours. At the end of this time, the reaction mixture was mixed with methylene chloride. The organic layer was then separated, washed with water and dried over anhydrous magnesium sulphate. The solvent was then removed by distillation under reduced pressure. The resulting residue was purified by flash chromatography using a 1 : 1 by volume mixture of cyclohexane and ethyl acetate as the eluent, to give 62 mg (yield 32%) of the title compound having an Rf value of 0.2.
¹H Nuclear Magnetic Resonance Spectrum (CDCℓ₃, 270 MHz), δ ppm:
   0.01 (6H, singlet);
   0.88 (9H, singlet);
   1.22 (3H, doublet, J = 6 Hz);
   1.40 (3H, doublet, J = 7.2 Hz);
   3.07 - 3.11 (2H, multiplet);
   4.04 (1H, doublet of doublets, J = 2 & 5.28 Hz);
   4.22 - 4.26 (1H, multiplet);
   5.91 (1H, singlet);
   7.63 - 7.68 (2H, multiplet);
   7.7 - 7.8 (1H, multiplet);
   7.87 (1H, doublet, J = 8.5 Hz);
   8.11 (1H, doublet, J = 8.5 Hz);
   8.22 (1H, doublet, J = 8.58 Hz).

### EXAMPLE 3

### (3S,4S)-3[(1R)-1-t-Butyldimethylsilyloxyethyl]-4-{(1R)-1-[3-methyl-2-pyridylthiocarbonyl]ethyl}azetidin-2-one (Compound No. 1-2)

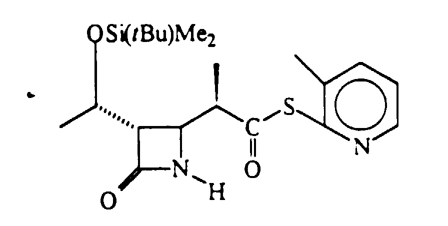

### 3(a) Z(O)-2-(1-t-Butyldimethylsilyloxy-1-propenylthio)-3-methylpyridine

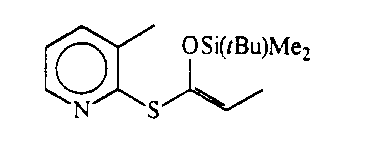

15.17 ml (24.3 mmole) of a 1.6 M solution of butyllithium in hexane were added dropwise at -78°C to a solution of 5.59 ml (26.5 mmole) of hexamethyldisilazane in a mixture of 40 ml of tetrahydrofuran and 4.61 ml (26.5 mmole) of hexamethylphosphoric triamide, and then 6.65 g (44.1 mmole) of t-butyldimethylsilyl chloride and 9.25 ml (66.3 mmole) of triethylamine were added to the resulting mixture. The reaction mixture was then stirred for a further 10 minutes, after which a solution of 4.00 g (22.1 mmole) of 2-propionylthio-3-methylpyridine in 10 ml of anhydrous tetrahydrofuran was added dropwise to the reaction mixture. The mixture was then stirred at -78°C for 10 minutes, and the temperature of the reaction mixture was allowed to rise to room temperature. A saturated aqueous solution of sodium hydrogencarbonate was then mixed with the mixture, and the resulting mixture wag extracted three times, each time with 60 ml of pentane. The extract was dried over anhydrous magnesium sulphate, and then the solvent was removed by distillation under reduced pressure. The resulting residue was purified by column chromatography through alumina, using a 5 : 1 by volume mixture of hexane and ethyl acetate as the eluent, to give 3.38 g (yield 52%) of the title compound as a colourless oil.
¹H Nuclear Magnetic Resonance Spectrum (CDCℓ₃), δ ppm:
   0.04 (6H, singlet);
   0.85 (9H, singlet);
   1.74 (3H, doublet, J = 7 Hz);
   2.25 (3H, singlet);
   5.33 (3H, quartet, J = 7 Hz);
   6.95 (1H, doublet of doublets, J = 7 & 5 Hz);
   7.33 (1H, doublet of doublets, J = 7 & 1 Hz);
   8.37 (1H, doublet of doublets, J = 5 & 1 Hz).

### 3(b) (3S,4S)-3[(1R)-1-t-Butyldimethylsilyloxyethyl]-4-{(1R)-1-[3-methyl-2-pyridylthiocarbonyl]ethyl}azetidin-2-one

190 mg (1.39 mmole) of zinc chloride were added to a solution of 200 mg (0.70 mmole) of Z(O)-(3S,4S)-3-[(1R)-1-t-butyldimethylsilyloxyethyl]-4-acetoxyazetidin-2-one in 5 ml of methylene chloride, and then a solution of 411 mg of 2-(1-t-butyldimethylsilyloxy-1-propenylthio)-3-methylpyridine [prepared as described in step (a) above] in 2 ml of methylene chloride was added thereto, whilst ice-cooling and under an atmosphere of nitrogen. The resulting mixture was then stirred at room temperature for 8 hours, after which 50 ml of methylene chloride were added. The organic layer was washed with ice-water and dried over anhydrous magnesium sulphate, and the solvent was removed by distillation under reduced pressure. The resulting residue was purified by medium pressure column chromatography through silica gel, using a 1 : 2 by volume mixture of hexane and ethyl acetate as the eluent, to give 246 mg (yield 87%) of the title compound as colourless crystals. A specimen for analysis was recrystallised from diisopropyl ether and was found to melt at 120 - 122°C.
¹H Nuclear Magnetic Resonance Spectrum (CDCℓ₃), δ ppm:
   0.07 (6H, singlet);
   0.87 (9H, singlet);
   1.20 (3H, doublet, J = 6 Hz);
   1.35 (3H, doublet, J = 7 Hz);
   2.36 (3H, singlet);
   3.02 - 3.13 (2H, multiplet);
   4.00 (1H, doublet of doublets, J = 4 & 2 Hz);
   4.20 (1H, multiplet);
   5.89 (1H, broad singlet);
   7.28 (1H, doublet of doublets, J = 8 & 5 Hz);
   7.64 (1H, doublet of doublets, J = 8 & 1 Hz);
   8.50 (1H, doublet of doublets, J = 5 & 1 Hz).

### EXAMPLE 4

### (3S,4S)-3[(1R)-1-t-Butyldimethylsilyloxyethyl]-4-{(1R)-1-[4-methyl-2-pyridylthiocarbonyl]ethyl}azetidin-2-one (Compound No. 1-3)

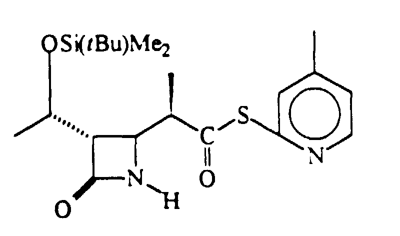

### 4(a) Z(O)-2-(1-t-Butyldimethylsilyloxy-1-propenylthio)-4-methylpyridine

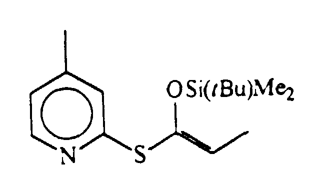

Following a procedure similar to that described in Example 3(a), but using 1.51 g (8.33 mmole) of 2-propionylthio-4-methylpyridine, 1.25 g (yield 51%) of the title compound was obtained as a colourless oil.
¹H Nuclear Magnetic Resonance Spectrum (CDCℓ₃), δ ppm:
   0.09 (6H, singlet);
   0.89 (9H, singlet);
   1.74 (3H, doublet, J = 7 Hz);
   2.30 (3H, singlet);
   5.43 (1H, quartet, J = 7 Hz);
   6.82 (1H, doublet, J = 5 Hz);
   7.14 (1H, singlet);
   8.27 (1H, doublet, J = 5 Hz).

### 4(b) (3S,4S)-3[(1R)-1-t-Butyldimethylsilyloxyethyl]-4-{(1R)-1-[4-methyl-2-pyridylthiocarbonyl]ethyl}azetidin-2-one

Following a procedure similar to that described in Example 3(b), but using 500 mg (1.74 mmole) of Z(O)-(3S,4S)-3-[(1R)-1-t-butyldimethylsilyloxyethyl]-4-acetoxyazetidin-2-one, 1.03 g (3.49 mmole) of 2-(1-t-butyldimethylsilyloxy-1-propenylthio)-4-methylpyridine [prepared as described in step (a) above] and 474 mg (3.48 mmole) of zinc chloride, 502 mg (yield 71%) of the title compound were obtained as colourless crystals. A specimen for analysis was recrystallised from diisopropyl ether and melted at 123 - 125°C.
¹H Nuclear Magnetic Resonance Spectrum (CDCℓ₃), δ ppm:
   0.07 (6H, singlet);
   0.87 (9H, singlet);
   1.19 (3H, doublet, J = 6 Hz);
   1.35 (3H, doublet, J = 7 Hz);
   2.40 (3H, singlet);
   2.95 - 3.10 (2H, multiplet);
   3.98 (1H, doublet of doublets, J = 5 & 2 Hz);
   4.21 (1H, multiplet);
   5.92 (1H, broad);
   7.13 (1H, doublet of doublets, J = 5 & 1 Hz);
   7.42 (1H, doublet, J = 1 Hz);
   8.48 (1H, doublet, J = 5 Hz).

### EXAMPLE 5

### (3S,4S)-3[(1R)-1-t-Butyldimethylsilyloxyethyl]-4-{(1R)-1-[5-methyl-2-pyridylthiocarbonyl]ethyl}azetidin-2-one (Compound No. 1-4)

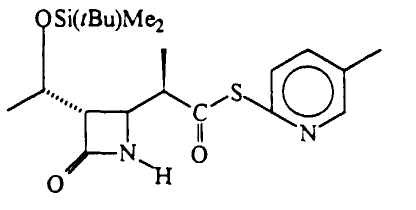

### 5(a) Z(O)-2-(1-t-Butyldimethylsilyloxy-1-propenylthio)-5-methylpyridine

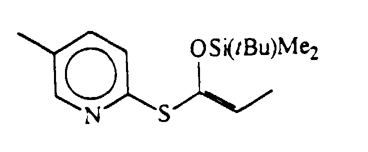

Following a procedure similar to that described in Example 3(a), but using 1.80 g (9.93 mmole) of 2-propionylthio-5-methylpyridine, 1.50 g (yield 51%) of the title compound was obtained as a colourless oil.
¹H Nuclear Magnetic Resonance Spectrum (CDCℓ₃), δ ppm:
   0.09 (6H, singlet);
   0.88 (9H, singlet);
   1.72 (3H, doublet, J = 7 Hz);
   2.27 (3H, singlet);
   5.42 (1H, quartet, J = 7 Hz);
   7.22 (1H, doublet, J = 8 Hz);
   7.36 (1H, doublet of doublets, J = 8 & 2 Hz);
   8.26 (1H, doublet, J = 2 Hz).

### 5(b) (3S,4S)-3[(1R)-1-t-Butyldimethylsilyloxyethyl]-4-{(1R)-1-[5-methyl-2-pyridylthiocarbonyl]ethyl}azetidin-2-one

Following a procedure similar to that described in Example 3(b), but using 500 mg (1.74 mmole) of Z(O)-(3S,4S)-3-[(1R)-1-t-butyldimethylsilyloxyethyl]-4-acetoxyazetidin-2-one, 1.03 g (3.49 mmole) of 2-(1-t-butyldimethylsilyloxy-1-propenylthio)-5-methylpyridine [prepared as described in step (a) above] and 474 mg (3.48 mmole) of zinc chloride, 583 mg (yield 82%) of the title compound were obtained as colourless crystals. A specimen for analysis was recrystallised from diisopropyl ether and melted at 86 - 88°C.
¹H Nuclear Magnetic Resonance Spectrum (CDCℓ₃), δ ppm:
   0.07 (6H, singlet);
   0.87 (9H, singlet);
   1.19 (3H, doublet, J = 6 Hz);
   1.34 (3H, doublet, J = 7 Hz);
   2.37 (3H, singlet);
   2.95 - 3.08 (2H, multiplet);
   3.98 (1H, doublet of doublets, J = 5 & 2 Hz);
   4.21 (1H, multiplet);
   5.90 (1H, broad singlet);
   7.46 (1H, doublet, J = 8 Hz);
   7.56 (1H, doublet of doublets, J = 8 & 2 Hz);
   8.47 (1H, doublet, J = 2 Hz).

### EXAMPLE 6

### S-2-Diethylcarbamoylphenyl 2(R)-{(3S,4S)-3-[1(R)-(t-butyldimethylsilyloxy)ethyl]-2-oxo-4-azetidinyl}thiopropionate and its 2(S)-isomer (Compound No. 2-2)

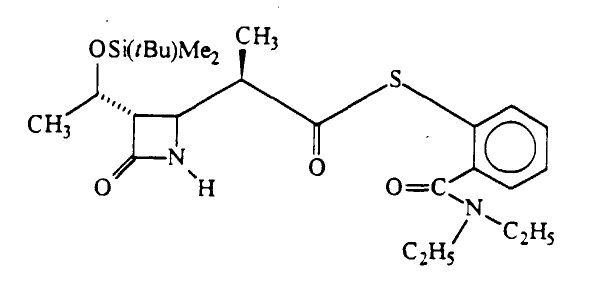

330 mg (2.42 mmole) of anhydrous zinc chloride were added to a solution of 911 mg (2.40 mmole) of 1-t-butyldimethylsilyloxy-1-(2-diethylcarbamoyl)phenylthio-1-propene (prepared as described in Preparation 19) and 347 mg (1.21 mmole) of (3R,4R)-3-[1(R)-(t-butyldimethylsilyloxy)ethyl]-4-acetoxy-2-azetidinone in 12 ml of methylene chloride, and the resulting mixture was stirred at room temperature for 2 hours. At the end of this time, the reaction mixture was diluted with ethyl acetate and the mixture was washed with water and dried over anhydrous magnesium sulphate. The solvent was then removed by distillation under reduced pressure. The resulting residue was purified by chromatography through a Lobar column, using a 1 : 1 by volume mixture of hexane and ethyl acetate as the eluent, to give 61 mg (yield 10%) of the 2S-isomer of the title compound, melting at 125 - 126.5°C (after recrystallisation from a mixture of hexane and ethyl acetate), and 487 mg (yield 82%) of the 2R-isomer of the title compound, melting at 130.5 - 132°C (after recrystallisation from diisopropyl ether).
Infrared Absorption Spectrum (KBr), νₘₐₓ cm⁻¹ (2S-isomer):
3182, 1765, 1711, 1629, 953, 774;
(2R-isomer):
3086, 1762, 1700, 1637, 965, 829.
Nuclear Magnetic Resonance Spectrum (CDCℓ₃, 270 MHz), δ ppm:
(2S-isomer): 0.06 (3H, singlet);
0.07 (3H, singlet);
0.87 (9H, singlet);
1.05 (3H, triplet, J = 7 Hz);
1.26 (3H, doublet, J = 6 Hz);
1.26 (3H, triplet, J = 7 Hz);
1.28 (3H, doublet, J = 7 Hz);
2.71 - 2.83 (2H, multiplet);
3.00 - 3.21 (2H, nonet-like, J = 7 Hz);
3.35 - 3.80 (2H, broad);
3.63 (1H, doublet, J = 9 Hz);
4.14 (1H, quintet, J = 6 Hz);
7.00 - 7.30 (1H, broad singlet);
7.30 - 7.35 (1H, multiplet);
7.42 - 7.53 (3H, multiplet);
(2R-isomer):
0.08 (6H, singlet);
0.87 (9H, singlet);
1.03 (3H, triplet, J = 7 Hz);
1.21 (3H, doublet, J = 6 Hz);
1.25 (3H, triplet, J = 7 Hz);
1.29 (3H, doublet, J = 7 Hz);
2.96 - 3.15 (4H, multiplet);
3.20 - 3.85 (2H, broad);
3.96 (1H, doublet of doublets, J = 2 & 4 Hz);
4.19 (1H, quintet, J = 6 Hz);

5.90 - 6.10 (1H, broad singlet);
7.30 - 7.35 (1H, multiplet);
7.41 - 7.51 (3H, multiplet).
Mass spectrum (m/z): (2R- and 2S-isomers) 492 (M⁺, C₂₅H₄₀N₂O₄SSi).

| Elemental analysis: | | | | |
|---|---|---|---|---|
| Calculated for C₂₅H₄₀N₂O₄SSi: | C, 60.94%; | H, 8.18%; | N, 5.69%; | S, 6.51%. |
| Found, 2S-isomer: | C, 60.72%; | H, 8.01%; | N, 5.70; | S, 6.57%. |
| Found, 2R-isomer: | C, 60.85%; | H, 8.10%; | N, 5.62%; | S, 6.50%. |

### EXAMPLES 7 TO 17

Following a similar procedure to that described in Example 6, the following compounds were also synthesised.

### EXAMPLE 7

### S-2-Dimethylcarbamoylphenyl 2(R)-{(3S,4S)-3-[1(R)-(t-butyldimethylsilyloxy)ethyl]-2-oxo-4-azetidinyl}thiopropionate (Compound No. 2-1)

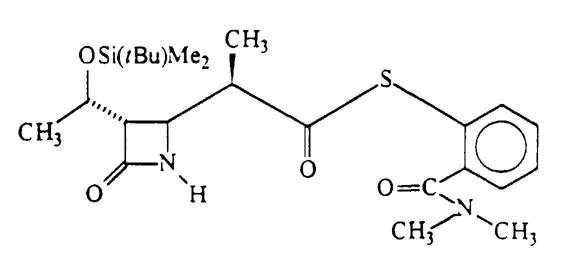

The yield of the 2R-isomer was 79%, and the ratio of the yields of 2R-isomer to 2S-isomer was 4.9 : 1.
Nuclear Magnetic Resonance Spectrum (CDCℓ₃), δ ppm:
   0.08 (6H, singlet);
   0.88 (9H, singlet);
   1.21 (3H, doublet, J = 6 Hz);
   1.29 (3H, doublet, J = 7 Hz);
   2.79 (3H, singlet);
   2.96 - 3.08 (2H, multiplet);
   3.10 (3H, singlet);
   3.94 (1H, doublet of doublets, J = 2 & 5 Hz);
   4.19 (1H, doublet of quartets, J = 5 & 6 Hz);
   6.10 - 6.20 (1H, broad singlet);
   7.31 - 7.36 (1H, multiplet);
   7.40 - 7.70 (3H, multiplet).

The 2R-isomer was in the form of needle-like crystals, melting at 99 - 101°C (after recrystallisation from a mixture of ethyl acetate and hexane).

### EXAMPLE 8

### S-2-Dipropylcarbamoylphenyl 2(R)-{(3S,4S)-3-[1(R)-(t-butyldimethylsilyloxy)ethyl]-2-oxo-4-azetidinyl}thiopropionate (Compound No. 2-3)

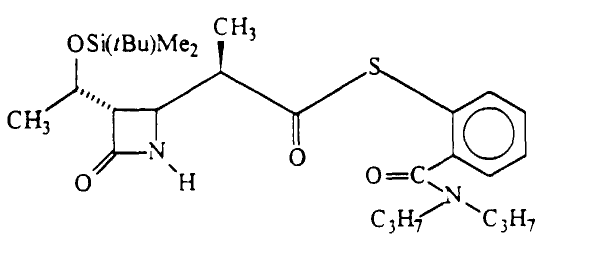

The yield of the 2R-isomer was 74%, and the ratio of the yields of 2R-isomer to 2S-isomer was 3.5 : 1.
Nuclear Magnetic Resonance Spectrum (CDCℓ₃), δ ppm:
   0.08 (6H, singlet);
   0.72 (3H, triplet, J = 7 Hz);
   0.88 (9H, singlet);
   1.00 (3H, triplet, J = 7 Hz);
   1.22 (3H, doublet, J = 6 Hz);
   1.20 - 1.40 (3H, broad);
   1.46 (2H, sextet, J = 7 Hz);
   1.70 (2H, sextet, J = 7 Hz);
   2.91 - 3.06 (2H, multiplet);
   3.10 - 3.80 (2H, broad);
   3.96 (2H, doublet of doublets, J = 2 & 4 Hz);
   4.19 (1H, doublet of quartets, J = 5 & 6 Hz);
   5.90 - 6.20 (1H, broad singlet);
   7.92 - 7.35 (1H, multiplet);
   7.40 - 7.52 (3H, multiplet).

The 2R-isomer was in the form of needle-like crystals, melting at 112 - 113°C (after recrystallisation from a mixture of ethyl acetate and hexane).

### EXAMPLE 9

### S-2-Diisobutylcarbamoylphenyl 2(R)-{(3S,4S)-3-[1(R)-(t-butyldimethylsilyloxy)ethyl]-2-oxo-4-azetidinyl}thiopropionate (Compound No. 2-6)

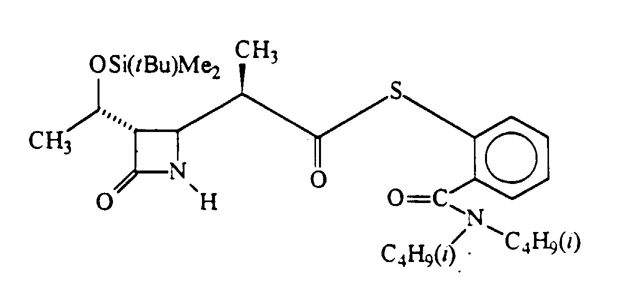

The yield of the 2R-isomer was 70%, and the ratio of the yields of 2R-isomer to 2S-isomer was 4.6 : 1.
Nuclear Magnetic Resonance Spectrum (CDCℓ₃), δ ppm:
   0.08 (6H, singlet);
   0.74 (6H, doublet, J = 7 Hz);
   0.87 (9H, singlet);
   1.02 (6H, doublet, J = 7 Hz);
   1.21 (3H, doublet, J = 6 Hz);
   1.20 - 1.40 (3H, broad);
   1.81 (1H, septet, J = 7 Hz);
   2.12 (1H, septet, J = 7 Hz);
   2.80 - 3.06 (4H, multiplet);
   3.20 - 3.57 (2H, broad);
   3.92 - 4.05 (1H, broad singlet);
   4.13 - 4.28 (1H, broad);
   5.95 - 6.15 (1H, broad);
   7.29 - 7.35 (1H, multiplet);
   7.42 - 7.50 (3H, multiplet).

The 2R-isomer was in the form of needle-like crystals, melting at 144 - 146°C (after recrystallisation from a mixture of ethyl acetate and hexane).

### EXAMPLE 10

### S-2-(N-Methyl-N-phenylcarbamoyl)phenyl 2(R)-{(3S,4S)-3-[1(R)-(t-butyldimethylsilyloxy)ethyl]-2-oxo-4-azetidinyl}thiopropionate (Compound No. 2-16)

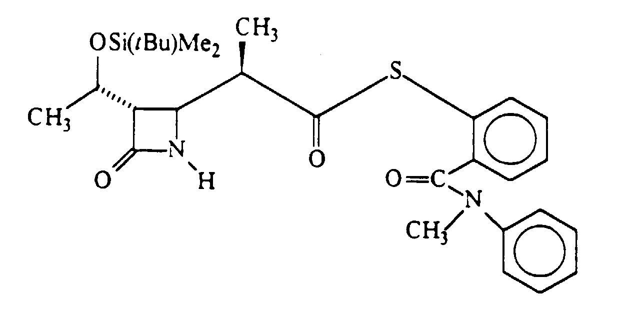

The yield of the 2R-isomer was 64%, and the ratio of the yields of 2R-isomer to 2S-isomer was 2.6 : 1.
Nuclear Magnetic Resonance Spectrum (CDCℓ₃), δ ppm:
   0.08 & 0.09 (together 6H, each singlet);
   0.88 (9H, singlet);
   1.23 (3H, doublet, J = 6 Hz);
   1.34 (3H, doublet, J = 7 Hz);
   3.01 - 3.12 (2H, multiplet);
   3.49 (3H, singlet);
   4.00 - 4.08 (1H, broad singlet);
   4.20 (1H, doublet of quartets, J = 6 & 6 Hz);
   6.05 - 6.20 (1H, broad singlet);
   6.95 - 7.63 (9H, multiplet).

The 2R-isomer was in the form of needle-like crystals, melting at 158 - 159.5°C (after recrystallisation from a mixture of ethyl acetate and hexane).

### EXAMPLE 11

### S-2-(1-Pyrrolidinylcarbonyl)phenyl 2(R)-{(3S,4S)-3-[1(R)-(t-butyldimethylsilyloxy)ethyl]-2-oxo-4-azetidinyl}thiopropionate (Compound No. 3-3)

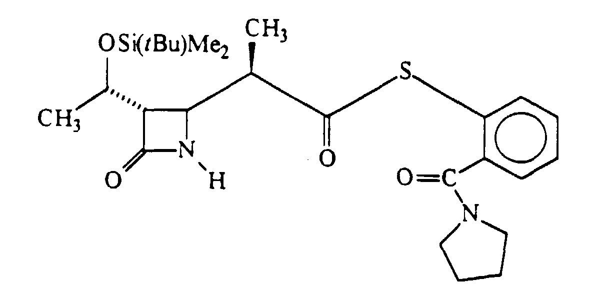

The yield of the 2R-isomer was 85%, and the ratio of the yields of 2R-isomer to 2S-isomer was 7.1 : 1.
Nuclear Magnetic Resonance Spectrum (CDCℓ₃), δ ppm:
   0.08 (6H, singlet);
   0.88 (9H, singlet);
   1.21 (3H, doublet, J = 6 Hz);
   1.29 (3H, doublet, J = 7 Hz);
   1.75 - 2.00 (4H, multiplet);
   2.95 - 3.06 (2H, multiplet);
   3.18 (2H, triplet, J = 7 Hz);
   3.60 (2H, triplet, J = 7 Hz);
   3.96 (1H, doublet of doublets, J = 2 & 4 Hz);
   4.20 (1H, doublet of quartets, J = 5 & 6 Hz);
   6.10 - 6.25 (1H, broad singlet);
   7.37 - 7.53 (4H, multiplet).

The 2R-isomer was in the form of a foam-like substance.

### EXAMPLE 12

### S-2-(1-Piperidylcarbonyl)phenyl 2(R)-{(3S,4S)-3-[1(R)-(t-butyldimethylsilyloxy)ethyl]-2-oxo-4-azetidinyl}thiopropionate (Compound No. 3-4)

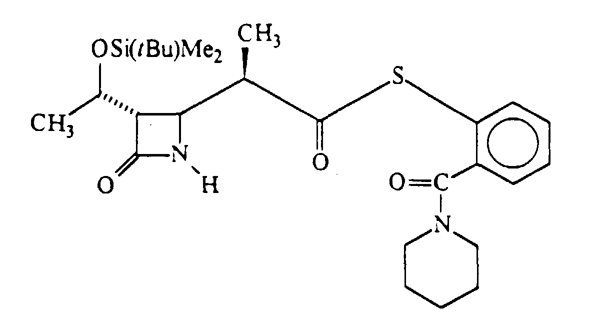

The yield of the 2R-isomer was 75%, and the ratio of the yields of 2R-isomer to 2S-isomer was 4.8 : 1.
Nuclear Magnetic Resonance Spectrum (CDCℓ₃), δ ppm:
   0.07 & 0.08 (together 6H, two singlets);
   0.87 & 0.88 (together 9H, two singlets);
   1.16 - 1.25 (3H, multiplet);
   1.28 & 1.33 (together 3H, two doublets, J = 7 & 7 Hz);
   1.37 - 1.52 (2H, broad);
   1.54 - 1.77 (4H, broad);
   2.95 - 3.26 (4H, multiplet);
   3.47 - 3.60 (1H, broad);
   3.80 - 3.95 (1H, broad);
   3.97 (1H, doublet of doublets, J = 2 & 4 Hz);
   4.12 - 4.26 (1H, broad);
   6.00 - 6.16 (1H, broad);
   7.26 - 7.52 (4H, multiplet).

The 2R-isomer was in the form of a glassy substance.

### EXAMPLE 13

### S-2-Morpholinocarbonylphenyl 2(R)-{(3S,4S)-3-[1(R)-(t-butyldimethylsilyloxy)ethyl]-2-oxo-4-azetidinyl}thiopropionate (Compound No. 3-8)

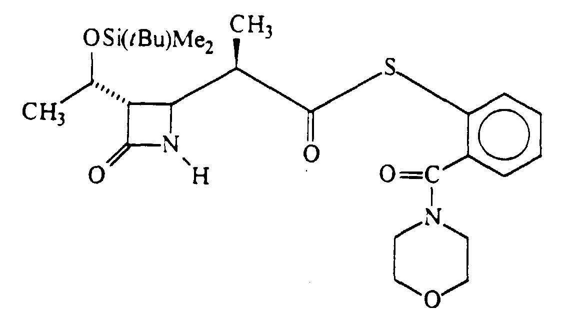

The yield of the 2R-isomer was 83%, and the ratio of the yields of 2R-isomer to 2S-isomer was 7.9 : 1.
Nuclear Magnetic Resonance Spectrum (CDCℓ₃), δ ppm:
   0.08 (6H, singlet);
   0.88 (9H, singlet);
   1.21 (3H, doublet, J = 7 Hz);
   1.22 - 1.38 (3H, multiplet);
   2.97 - 3.08 (2H, multiplet);
   3.12 - 3.32 (2H, multiplet);
   3.50 - 3.60 (2H, multiplet);
   3.70 - 3.84 (4H, broad);
   3.93 - 4.01 (1H, broad singlet);
   4.19 (1H, doublet of quartets, J = 5 & 5 Hz);
   5.90 - 6.10 (1H, broad);
   7.20 - 7.38 (1H, multiplet);
   7.42 - 7.55 (3H, multiplet).

The 2R-isomer was in the form of a glassy substance.

### EXAMPLE 14

### S-2-(1-Azepinylcarbonyl)phenyl 2(R)-{(3S,4S)-3-[1(R)-(t-butyldimethylsilyloxy)ethyl]-2-oxo-4-azetidinyl}thiopropionate (Compound No. 3-5)

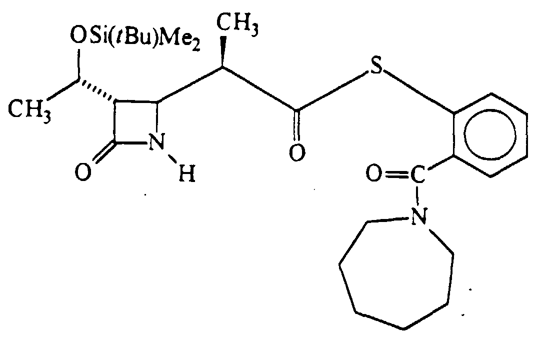

The yield of the 2R-isomer was 87%, and the ratio of the yields of 2R-isomer to 2S-isomer was 9.5 : 1.
Nuclear Magnetic Resonance Spectrum (CDCℓ₃), δ ppm:
   0.08 (6H, singlet);
   0.88 (9H, singlet);
   1.22 (3H, doublet, J = 6 Hz);
   1.20 - 1.40 (3H, broad);
   1.50 - 1.96 (8H, broad);
   2.97 - 3.10 (2H, multiplet);
   3.06 - 3.32 (2H, broad);
   3.40 - 3.90 (2H, broad);
   3.96 (1H, doublet of doublets, J = 2 & 4 Hz);
   4.20 (1H, doublet of quartets, J = 6 & 6 Hz);
   6.05 - 6.25 (1H, broad);
   7.30 - 7.37 (1H, multiplet);
   7.42 - 7.52 (3H, multiplet).

The 2R-isomer was in the form of a glassy substance.

### EXAMPLE 15

### S-2-Diethylcarbamoyl-6-methylphenyl 2(R)-{(3S,4S)-3-[1(R)-(t-butyldimethylsilyloxy)ethyl]-2-oxo-4-azetidinyl}thiopropionate (Compound No. 2-40)

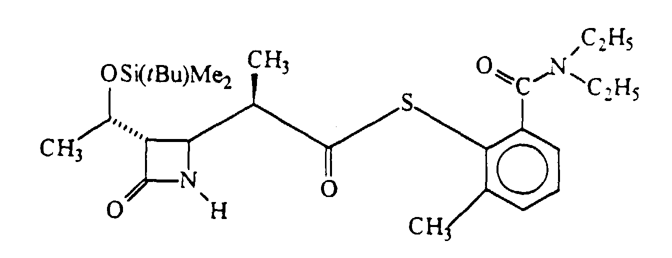

The yield of the 2R-isomer was 88%, and the ratio of the yields of 2R-isomer to 2S-isomer was 12.3 : 1.
Nuclear Magnetic Resonance Spectrum (CDCℓ₃), δ ppm:
   0.06 (3H, singlet);
   0.86 (9/2H, singlet);
   0.89 (9/2H, singlet);
   1.02 (3H, triplet, J = 7 Hz);
   1.19 - 1.29 (7.5H, multiplet);
   1.35 (1.5H, doublet, J = 7 Hz);
   2.35 (3H, singlet);
   2.92 - 3.16 (4H, multiplet);
   3.28 - 3.41 (1H, multiplet);
   3.74 (1H, doublet of quartets, J = 14 & 7 Hz);
   3.95 - 4.00 (1H, multiplet);
   4.17 (1H, quintet, J = 6 Hz);
   6.00 - 6.30 (1H, broad singlet);
   7.14 (1H, doublet of doublets, J = 3 & 6 Hz);
   7.34 - 7.41 (2H, multiplet).

The 2R-isomer was in the form of needle-like crystals, melting at 150 - 150.5°C (after recrystallisation from diisopropyl ether).

### EXAMPLE 16

### S-2-[Diethyl(thiocarbamoyl)]-6-methylphenyl 2(R)-{(3S,4S)-3-[1(R)-(t-butyldimethylsilyloxy)ethyl]-2-oxo-4-azetidinyl}thiopropionate (Compound No. 2-63)

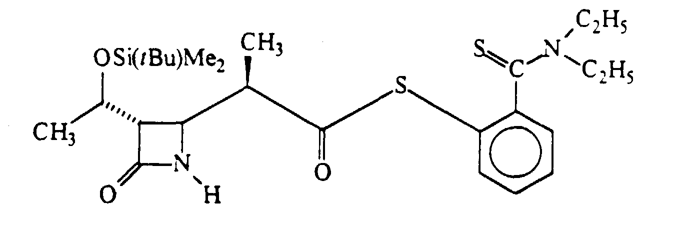

The yield of the 2R-isomer was 81%, and the ratio of the yields of 2R-isomer to 2S-isomer was 4.8 : 1.
Nuclear Magnetic Resonance Spectrum (CDCℓ₃), δ ppm:
   0.09 (6H, singlet);
   0.89 (9H, singlet);
   1.09 (3H, triplet, J = 7 Hz);
   1.22 (3H, doublet, J = 6 Hz);
   1.26 (3H, doublet, J = 7 Hz);
   1.38 (3H, triplet, J = 7 Hz);
   2.96 - 3.06 (2H, multiplet);
   3.20 (1H, doublet of quartets, J = 14 & 7 Hz);
   3.36 (1H, doublet of quartets, J = 14 & 7 Hz);
   3.76 (1H, doublet of quartets, J = 14 & 7 Hz);
   3.97 (1H, doublet of doublets, J = 2 & 5 Hz);
   4.20 (1H, doublet of quartets, J = 5 & 6 Hz);
   4.46 (1H, doublet of quartets, J = 14 & 7 Hz);
   7.22 - 7.26 (1H, multiplet);
   7.33 - 7.46 (3H, multiplet).

The 2R-isomer was in the form of needle-like crystals, melting at 163 - 165°C (after recrystallisation from a mixture of ethyl acetate and hexane).

### EXAMPLE 17

### S-2-Diethylcarbamoylphenyl 2(R)-{(3S,4S)-3-[1(R)-(t-butyldimethylsilyloxy)ethyl]-2-oxo-4-azetidinyl}propionate (Compound No. 2-56)

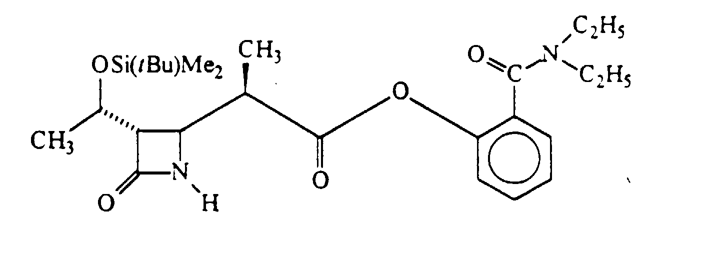

The yield of the 2R-isomer was 61%, and the ratio of the yields of 2R-isomer to 2S-isomer was 6.8 : 1.
Nuclear Magnetic Resonance Spectrum (CDCℓ₃), δ ppm:
   0.09 (6H, singlet);
   0.88 (9H, singlet);
   1.10 (3H, triplet, J = 7 Hz);
   1.22 (3H, triplet, J = 7 Hz);
   1.25 (3H, doublet, J = 6 Hz);
   1.32 (3Hz, doublet, J = 7 Hz);
   2.95 (1H, doublet of quartets, J = 4 & 7 Hz);
   3.00 (1H, doublet, J = 6 Hz);
   3.22 (2H, quartet, J = 7 Hz);
   3.42 - 3.63 (1H, multiplet);
   4.08 - 4.16 (1H, multiplet);
   4.18 (1H, quintet, J = 6 Hz);
   6.45 (1H, broad singlet);
   7.17 (1H, doublet, J = 8 Hz);
   7.26 - 7.28 (2H, multiplet);
   7.38 - 7.43 (1H, multiplet).

The 2R-isomer was in the form of a glassy substance.

### PREPARATION 1

### 2-Propionylthiopyridine

15.6 ml (1.2 equivalents) of propionyl chloride were slowly added at room temperature to a solution of 20 g of 2-pyridinethiol and 25.1 ml (1.2 equivalents) of triethylamine in 200 ml of anhydrous methylene chloride, and the resulting mixture was stirred for 1 hour. At the end of this time, the solvent was removed by distillation under reduced pressure, and the resulting residue was mixed with ethyl acetate. The organic solution thus obtained was washed twice with a dilute aqueous solution of sodium hydrogencarbonate and then with water. The organic layer was separated and then dried over anhydrous magnesium sulphate, after which the solvent was removed by distillation under reduced pressure. The residue was subjected to fractional distillation in vacuo, to give 27 g (yield 90%) of the title compound, boiling at 93°C/0.05 mmHg (6.7 Pa).
¹H Nuclear Magnetic Resonance Spectrum (CDCℓ₃, 270 MHz), δ ppm:
   1.24 (3H, doublet, J = 7.5 Hz);
   2.74 (2H, quartet, J = 7.5 Hz);
   7.30 - 7.63 (1H, multiplet);
   7.62 (1H, doublet, J = 7.9 Hz);
   7.70 - 7.76 (1H, multiplet);
   8.61 - 8.63 (1H, multiplet).

### PREPARATION 2

### 2-Propionylthioquinoline

480 mg (1.2 equivalents) of propionyl chloride were added, whilst ice-cooling, to a solution of 1 g of 2-quinolinethiol and 0.83 ml (1.2 equivalents) of triethylamine in 20 ml of anhydrous methylene chloride, and the resulting mixture was stirred for 30 minutes. At the end of this time, the solvent was removed by distillation under reduced pressure, and the resulting residue was mixed with ethyl acetate. This mixture was washed twice with a dilute aqueous solution of sodium hydrogencarbonate and was then washed with water. The organic layer was separated and dried over anhydrous magnesium sulphate and the solvent was removed by distillation under reduced pressure. The residue was purified by flash chromatography through silica gel, using a 1 : 1 by volume mixture of cyclohexane and ethyl acetate as the eluent, to give 600 mg of the title compound having an Rf value of 0.4.
¹H Nuclear Magnetic Resonance Spectrum (CDCℓ₃, 270 MHz), δ ppm:
   1.27 (3H, triplet, J = 7.2 Hz);
   2.79 (2H, quartet, J = 7.2 Hz);
   7.53 - 7.61 (1H, multiplet);
   7.67 - 7.76 (2H, multiplet);
   7.49 (1H, doublet, J = 7.9 Hz);
   8.09 (1H, doublet, J = 8.5 Hz);
   8.19 (1H, doublet, J = 8.5 Hz).

### PREPARATION 3

### (3S,4R)-3-[(1R)-1-t-Butyldimethylsilyloxyethyl]-4-phenylsulfinylazetidin-2-one

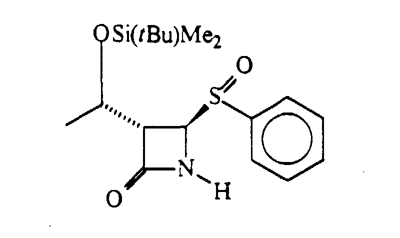

### 3(i) (3S,4R)-3-[(1R)-1-t-butyldimethylsilyloxyethyl]-4-phenylthio-2-azetidinone

0.7 g of sodium thiophenolate was added, whilst ice-cooling, to a solution of 2 g of (3S,4R)-3-[(1R)-1-t-butyldimethylsilyloxyethyl]-4-acetoxyazetidin-2-one in 20 ml of ethanol, and the resulting mixture was stirred at room temperature for 3 hours. At the end of this time, the reaction mixture was mixed with 50 ml of ethyl acetate, and the organic layer was separated and washed with water, with a saturated aqueous solution of sodium hydrogencarbonate and with water, in that order. It was then dried over anhydrous magnesium sulphate, and the solvent wag removed by distillation under reduced pressure, to give 2 g of the title compound as colourless crystals, melting at 110 - 112.5°C.
Rf = 0.5 (thin layer chromatography through silica gel; developing solvent: a 3 : 1 by volume mixture of cyclohexane and ethyl acetate).
¹H Nuclear Magnetic Resonance Spectrum (CDCℓ₃, 270 MHz), δ ppm:
   0.06 (6H, singlet);
   0.87 (9H, singlet);
   1.20 (3H, doublet, J = 6.6 Hz);
   3.02 - 3.04 (1H, multiplet);
   4.18 - 4.26 (1H, multiplet);
   5.07 (1H, doublet, J = 2.6 Hz);
   6.05 (1H, singlet);
   7.33 - 7.48 (5H, multiplet).

### 3(ii) (3S,4R)-3-[(1R)-1-t-Butyldimethylsilyloxyethyl]-4-phenylsulfinylazetidin-2-one

1.5 g of 3-chloroperoxybenzoic acid was added, whilst ice-cooling, to a solution of 2 g of (3S,4R)-3-[(1R)-1-t-butyldimethylsilyloxyethyl]-4-phenylthio-2-azetidinone [prepared as described in step (i) above] in 30 ml of methylene chloride, and the resulting mixture was stirred at room temperature for 6 hours. At the end of this time, the reaction mixture was mixed with 30 ml of methylene chloride, and the organic layer was separated and washed with water, with a saturated aqueous solution of sodium hydrogencarbonate and again with water, in that order. It was then dried over anhydrous magnesium sulphate, and the solvent was removed by distillation under reduced pressure. The resulting residue was purified by flash chromatography, using a 1 : 1 by volume mixture of cyclohexane and ethyl acetate as the eluent, to give 1.9 g of the title compound consisting of two diastereomers, differing in the configuration of the sulphoxide group, as colourless crystals, melting at 65 - 70°C.
Rf = 0.4 (developing solvent: a 1 : 1 by volume mixture of cyclohexane and ethyl acetate).
¹H Nuclear Magnetic Resonance Spectrum (CDCℓ₃, 270 MHz), δ ppm:
   0.02 - 0.04 (6H, multiplet);
   0.82 - 0.84 (9H, multiplet);
   1.43 (3H, singlet);
   3.35 - 3.36 (0.4H, multiplet);
   3.51 (0.6H, singlet);
   4.16 - 4.25 (1H, multiplet);
   4.53 (1H, singlet);
   6.57 (0.4H, singlet);
   6.69 (0.6H, singlet);
   7.51 - 7.72 (5H, multiplet).

### PREPARATION 4

### 2-Hydroxy-3-methylpyridine

A solution of 2.30 g (33.3 mmole) of sodium nitrite in 5 ml of water was added dropwise at room temperature to a solution of 3.60 g (33.3 mmole) of 2-amino-3-picoline in a mixture of 65 ml of water and 4 ml of concentrated sulphuric acid, and the resulting mixture was stirred at room temperature for 2 hours. At the end of this time, the pH of the reaction mixture was adjusted to a value of 7 by the addition of sodium carbonate, and the water was removed by distillation under reduced pressure. The mixture was then extracted three times, each time with 80 ml of warm ethanol. The extract was then freed from the ethanol by distillation under reduced pressure, and the resulting residue was recrystallised from ethyl acetate, to give 3.38 g (yield 93%) of the title compound as colourless crystals, melting at 137 - 139°C.
¹H Nuclear Magnetic Resonance Spectrum (CDCℓ₃), δ ppm:
   2.18 (3H, singlet);
   6.22 (1H, doublet of doublets, J = 7 & 7 Hz);
   7.3 - 7.4 (2H, multiplet);
   13.14 (1H, singlet).

### PREPARATION 5

### 2-Hydroxy-4-methylpyridine

Following a procedure similar to that described in Preparation 4, but using 3.60 g (33.3 mmole) of 2-amino-4-picoline, 2.32 g (yield 64%) of the title compound were obtained as colourless crystals, melting at 121 - 123°C.
¹H Nuclear Magnetic Resonance Spectrum (CDCℓ₃), δ ppm:
   2.28 (3H, singlet);
   6.14 (1H, doublet, J = 6 Hz);
   6.39 (1H, singlet);
   7.27 (1H, doublet, J = 6 Hz);
   13.24 (1H, singlet).

### PREPARATION 6

### 2-Hydroxy-5-methylpyridine

Following a procedure similar to that described in Preparation 4, but using 3.60 g (33.3 mmole) of 2-amino-5-picoline, 2.56 g (yield 71%) of the title compound were obtained as colourless crystals, melting at 175 - 177°C.
¹H Nuclear Magnetic Resonance Spectrum (CDCℓ₃), δ ppm:
   2.10 (3H, singlet);
   6.53 (1H, doublet, J = 9 Hz);
   7.16 (1H, broad singlet);
   7.33 (1H, doublet of doublets, J = 9 & 3 Hz);
   13.35 (1H, singlet).

### PREPARATION 7

### 2-Mercapto-3-methylpyridine

A mixture of 2.00 g (18.3 mmole) of 2-hydroxy-3-methylpyridine (prepared as described in Preparation 4) and 2.17 g (9.76 mmole) of phosphorus pentasulphide was heated at 160°C for 4 hours. At the end of this time, the reaction mixture was diluted with 200 ml of water, and the pH of the mixture was adjusted to a value of 6 by the addition of potassium carbonate. The mixture was then extracted twice, each time with 100 ml of chloroform. The extract was washed with a saturated aqueous solution of sodium chloride and dried over anhydrous magnesium sulphate. The solvent was then removed by distillation under reduced pressure. The resulting residue was recrystallised from benzene to give 2.15 g (yield 94%) of the title compound as yellow crystals, melting at 163 - 165°C.
¹H Nuclear Magnetic Resonance Spectrum (CDCℓ₃), δ ppm:
   2.44 (3H, singlet);
   6.74 (1H, doublet of doublets, J = 7 & 7 Hz);
   7.45 (1H, broad doublet, J = 7 Hz);
   7.53 (1H, broad doublet, J = 7 Hz);
   13.82 (1H, singlet).
Mass Spectrum m/e: 125 (M⁺)

### PREPARATION 8

### 2-Mercapto-4-methylpyridine

Following a procedure similar to that described in Preparation 7, but using 2.26 g (20.7 mmole) of 2-hydroxy-4-methylpyridine (prepared as described in Preparation 5), 2.01 g (yield 78%) of the title compound were obtained as yellow crystals, melting at 174 - 176°C.
¹H Nuclear Magnetic Resonance Spectrum (CDCℓ₃), δ ppm:
   2.27 (3H, singlet);
   6.63 (1H, doublet, J = 6 Hz);
   7.42 (1H, singlet);
   7.51 (1H, doublet, J = 6 Hz);
   13.69 (1H, singlet).

### PREPARATION 9

### 2-Mercapto-5-methylpyridine

Following a procedure similar to that described in Preparation 7, but using 2.48 g (22.7 mmole) of 2-hydroxy-5-methylpyridine (prepared as described in Preparation 6), 2.43 g (yield 86%) of the title compound were obtained as yellow crystals, melting at 178 - 181°C.
¹H Nuclear Magnetic Resonance Spectrum (CDCℓ₃), δ ppm:
   2.20 (3H, singlet);
   7.26 (1H, doublet of doublets, J = 9 & 2 Hz);
   7.43 (1H, broad singlet);
   7.49 (1H, doublet, J = 9 Hz);
   13.89 (1H, singlet).

### PREPARATION 10

### 2-Propionylthio-3-methylpyridine

1.21 ml (13.9 mmole) of propionyl chloride were added dropwise, whilst ice-cooling and under an atmosphere of nitrogen, to a solution of 1.46 g (11.7 mmole) of 2-mercapto-3-methylpyridine (prepared as described in Preparation 7) and 1.94 ml (13.9 mmole) of triethylamine in 15 ml of methylene chloride, and the resulting mixture was stirred for 1 hour. At the end of this time, the reaction mixture was mixed with 50 ml of methylene chloride, and the mixture was washed with a saturated aqueous solution of sodium hydrogencarbonate. It was then dried over anhydrous magnesium sulphate, and the solvent was removed by distillation under reduced pressure. The residue was purified by medium pressure column chromatography through silica gel, using a 5 : 1 by volume mixture of hexane and ethyl acetate as the eluent, to give 1.89 g (yield 89%) of the title compound as a light yellow oil.
¹H Nuclear Magnetic Resonance Spectrum (CDCℓ₃), δ ppm:
   1.24 (3H, triplet, J = 7 Hz);
   2.37 (3H, singlet);
   2.73 (2H, quartet, J = 7 Hz);
   7.25 (1H, doublet of doublets, J = 8 & 5 Hz);
   7.61 (1H, doublet, J = 8 Hz);
   8.48 (1H, doublet, J = 5 Hz).
Mass Spectrum m/e: 182 (M⁺ + H)

### PREPARATION 11

### 2-Propionylthio-4-methylpyridine

Following a procedure similar to that described in Preparation 10, but using 1.20 g (9.59 mmole) of 2-mercapto-4-methylpyridine (prepared as described in Preparation 8), 1.62 g (yield 93%) of the title compound were obtained as a light yellow oil.
¹H Nuclear Magnetic Resonance Spectrum (CDCℓ₃), δ ppm:
   1.24 (3H, triplet, J = 7 Hz);
   2.38 (3H, singlet);
   2.72 (2H, quartet, J = 7 Hz);
   7.10 (1H, doublet, J = 5 Hz);
   7.44 (1H, singlet);
   8.47 (1H, doublet, J = 5 Hz).

### PREPARATION 12

### 2-Propionylthio-5-methylpyridine

Following a procedure similar to that described in Preparation 10, but using 1.40 g (11.2 mmole) of 2-mercapto-5-methylpyridine (prepared as described in Preparation 9), 1.97 g (yield 97%) of the title compound were obtained as a light yellow oil.
¹H Nuclear Magnetic Resonance Spectrum (CDCℓ₃), δ ppm:
   1.23 (3H, triplet, J = 7 Hz);
   2.35 (3H, singlet);
   2.71 (2H, quartet, J = 7 Hz);
   7.47 (1H, doublet, J = 8 Hz);
   7.54 (1H, doublet of doublets, J = 8 & 2 Hz);
   8.45 (1H, doublet, J = 2 Hz).
Mass Spectrum m/e: 181 (M⁺)

### PREPARATION 13

### (3S,4S)-3-[(1R)-1-t-Butyldimethyloxyethyl]-4-{2(S)-[4-(2-p-nitrobenzyloxycarbonyloxyethyl)piperazin-1-ylcarbonyl]-1-(p-nitrobenzyloxycarbonyl)pyrrolidin-4(S)-yl]thiocarbonylethyl}azetidin-2-one

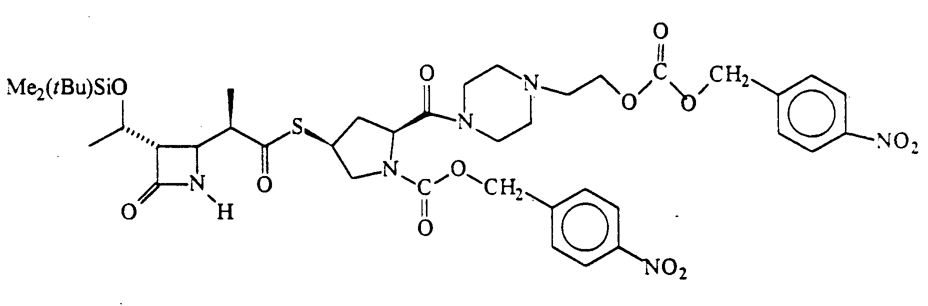

0.75 ml (5.38 mmole) of triethylamine was added, whilst ice-cooling and under an atmosphere of nitrogen, to a solution of 1.76 g (4.47 mmole) of (3S,4S)-3-[(1R)-1-t-butyldimethylsilyloxyethyl]-4-[1(R)-(2-pyridylthiocarbonyl)ethyl]azetidin-2-one (prepared as described in Example 1) and 2.79 g (4.52 mmole) of (2S,4S)-2-{4-[2-(p-nitrobenzyloxycarbonyloxy)ethyl]-1-piperazinylcarbonyl)-4-mercapto-1-(p-nitrobenzyloxycarbonyl)pyrrolidine in 40 ml of methylene chloride, and the resulting mixture was stirred at room temperature for 7 hours. At the end of this time, the reaction mixture was mixed with 100 ml of methylene chloride, and the organic mixture was then washed twice, each time with 50 ml of a cooled 1N aqueous solution of sodium hydroxide, after which it was washed with a saturated aqueous solution of sodium chloride. It was then dried over anhydrous magnesium sulphate, and the solvent gas removed by distillation under reduced pressure. The resulting residue was recrystallised from 40 ml of ethyl acetate, to give 3.03 g (yield 75%) of the title compound as colourless crystals, melting at 141 - 143°C.

### PREPARATION 14

### (3S,4S)-3-[(1R)-1-Hydroxyethyl]-4-{2(S)-[4-(2-p-nitrobenzyloxycarbonyloxyethyl)piperazin-1-ylcarbonyl]-1-(p-nitrobenzyloxycarbonyl)pyrrolidin-4(S)-yl]-thiocarbonylethyl}azetidin-2-one

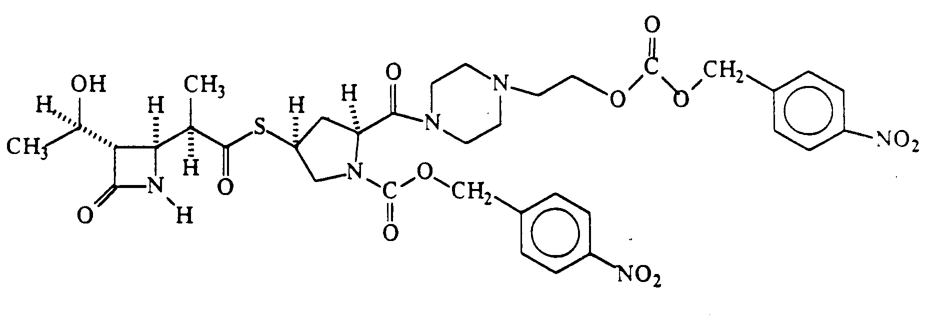

24 ml of 3N aqueous hydrochloric acid were added dropwise, whilst ice-cooling, to a solution of 8.1 g of (3S,4S)-3-[(1R)-1-t-butyldimethyloxyethyl]-4-{2(S)-[4-(2-p-nitrobenzyloxycarbonyloxyethyl)piperazin-1-ylcarbonyl]-1-(p-nitrobenzyloxycarbonyl)pyrrolidin-4(S)-yl]thiocarbonylethyl}azetidin-2-one (prepared as described in Preparation 13) in 80 ml of methanol, after which the resulting mixture was stirred at the same temperature for 30 minutes and then allowed to stand overnight in a refrigerator. At the end of this time, the pH of the reaction mixture was adjusted to a value of 5 to 6 by the addition of sodium hydrogencarbonate, whilst ice-cooling. The mixture was then concentrated by evaporation under reduced pressure, and the concentrate was mixed with a small amount of water and extracted with ethyl acetate. The aqueous layer was separated, saturated with sodium chloride and extracted with ethyl acetate. The extracts were combined and the solvent was then removed by distillation under reduced pressure. The resulting residue was purified by flash chromatography through 150 g of silica gel (Merck Art No. 9385) using a gradient elution method with mixtures of ethyl acetate and methanol ranging from 20 : 1 to 10 : 1 by volume as the eluent, to give 5.9 g of the title compound as a colourless foam.
Infrared Absorption Spectrum (CHCℓ₃), νₘₐₓ cm⁻¹:
   1752, 1710, 1650, 1607, 1522, 1443, 1405, 1347, 1263.
Nuclear Magnetic Resonance Spectrum (CDCℓ₃, 270 MHz), δ ppm:
   1.27 (3H, doublet, J = 6.83 Hz);
   1.28 (3H, doublet, J = 6.35 Hz);
   1.82 - 1.99 (1H, multiplet);
   2.10 - 2.18 (1H, multiplet);
   2.40 - 2.95 (7H, multiplet);
   3.03 (1H, doublet of doublets, J = 1.95 & 6.35 Hz);
   3.37 - 3.80 (5H, multiplet);
   3.78 (1H, doublet of doublets, J = 1.95 & 6.84 Hz);
   3.95 - 4.48 (6H, multiplet);
   4.68 & 4.73 (together 1H, two triplets, J = 8.06 & 7.33 Hz);
   5.06 & 5.32 (together 1H, two doublets, J = 13.43 & 13.43 Hz);
   5.21 (1H, singlet);
   5.26 (2H, singlet);
   5.99 (1H, broad singlet);
   7.45 & 7.50 (together 2H, two doublets, J = 8.30 & 8.79 Hz);
   7.56 (2H, doublet, J = 8.79 Hz);
   8.18 - 8.26 (4H, multiplet).

### PREPARATION 15

### (3S,4S)-3-[(1R)-1-(Trimethylsilyloxy)ethyl]-4-{2(S)-[4-(2-p-nitrobenzyloxycarbonyloxyethyl)piperazin-1-ylcarbonyl]-1-(p-nitrobenzyloxycarbonyl)pyrrolidin-4(S)-yl]thiocarbonylethyl}azetidin-2-one

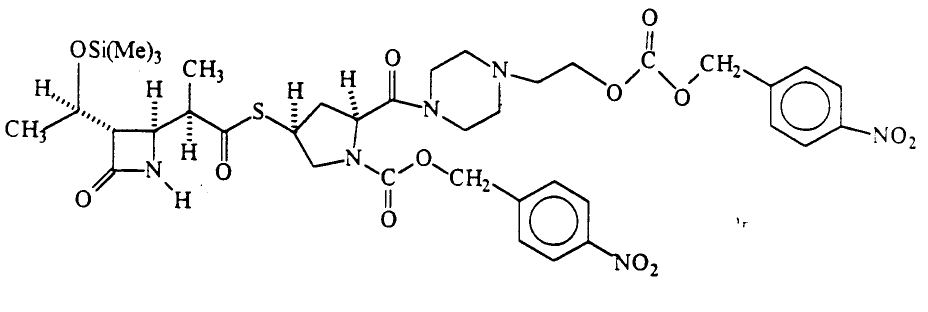

3.6 g of triethylamine and then 2.76 g of chlorotrimethylsilane were added dropwise, whilst ice-cooling and under a stream of nitrogen, to a solution of 4.0 g of (3S,4S)-3-[(1R)-1-hydroxyethyl]-4-{2(S)-[4-(2-p-nitrobenzyloxycarbonyloxyethyl)piperazin-1-ylcarbonyl]-1-(p-nitrobenzyloxycarbonyl)pyrrolidin-4(S)-yl]-thiocarbonylethyl}azetidin-2-one (prepared as described in Preparation 14) in 40 ml of methylene chloride, and the resulting mixture was stirred at room temperature for 20 minutes. The reaction mixture was then again cooled, and 25 ml of methanol and 3.3 g of silica gel (Merck Art No. 7734) were added to the mixture, after which it was stirred at room temperature for 3 hours. The mixture was then concentrated by evaporation under reduced pressure, and the concentrate was mixed with water and extracted with ethyl acetate. The extract was then washed with a saturated aqueous solution of sodium chloride and dried over anhydrous magnesium sulphate, after which the solvent was removed by distillation under reduced pressure. The resulting residue was purified by flash chromatography through 40 g of silica gel (Merck Art No. 9385), using a gradient elution method with mixtures of ethyl acetate and methanol ranging from 40 : 1 to 20 : 1 by volume as the eluent, to give 3.63 g of the title compound as a colourless foam.
Infrared Absorption Spectrum (KBr), νₘₐₓ cm⁻¹:
   1756, 1713, 1656, 1529, 1443, 1405, 1347, 1251.
Nuclear Magnetic Resonance Spectrum (CDCℓ₃, 270 MHz), δ ppm:
   0.11 (9H, singlet);
   1.18 (3H, doublet, J = 6.34 Hz);
   1.23 (3H, doublet, J = 6.84 Hz);
   1.84 - 1.92 (1H, multiplet);
   2.30 - 2.80 (8H, multiplet);
   2.82 - 2.92 (1H, multiplet);
   3.01 & 3.03 (together 1H, two doublets, J = 1.71 & 5.13 Hz);
   3.35 - 3.65 (4H, multiplet);
   3.77 & 3.79 (together 1H, two doublets, J = 1.71 & 5.86 Hz);
   3.93 - 4.03 (1H, multiplet);
   4.09 - 4.17 (2H, multiplet);
   4.22 - 4.37 (2H, multiplet);
   4.64 - 4.77 (1H, multiplet);
   5.06 & 5.32 (together 1H, two doublets, J = 13.67 & 13.67 Hz);
   5.22 (1H, doublet, J = 1.95 Hz);
   5.26 (2H, singlet);
   5.86 (1H, broad singlet);
   7.44 & 7.50 (together 2H, two doublets, J = 8.79 & 8.79 Hz);
   7.55 (2H, doublet, J = 8.79 Hz);
   8.15 - 8.27 (4H, multiplet).

### PREPARATION 16

### (3S,4S)-3-[(1R)-1-(Trimethylsilyloxy)ethyl]-4-{2(S)-[4-(2-p-nitrobenzyloxycarbonyloxyethyl)piperazin-1-ylcarbonyl]-1-(p-nitrobenzyloxycarbonyl)pyrrolidin-4(S)-yl]thiocarbonylethy}-1-(4-nitrobenzyloxyoxalyl)azetidin-2-one

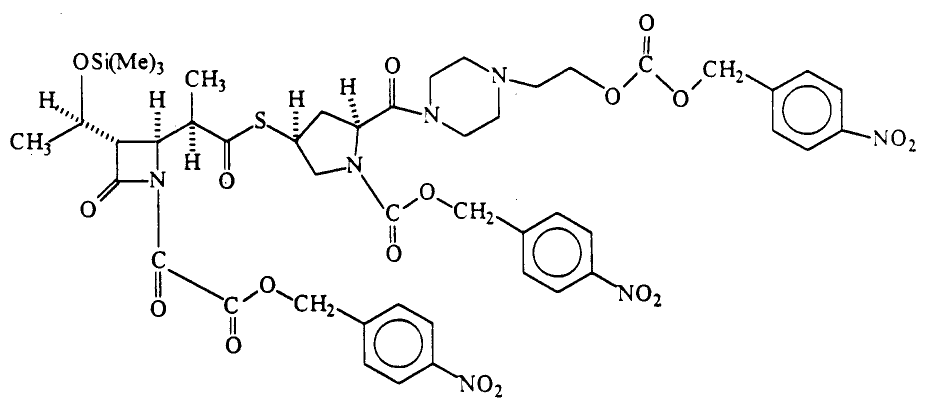

1.39 g of triethylamine were added dropwise, whilst ice-cooling and under a stream of nitrogen, to a solution of 3.94 g of (3S,4S)-3-[(1R)-1-(trimethylsilyloxy)ethyl]-4-{2(S)-[4-(2-p-nitrobenzyloxycarbonyloxyethyl)piperazin-1-ylcarbonyl]-1-(p-nitrobenzyloxycarbonyl)pyrrolidin-4(S)-yl]thiocarbonylethyl}azetidin-2-one (prepared as described in Preparation 15) in 30 ml of methylene chloride. A solution of 3.35 g of p-nitrobenzyloxyoxalyl chloride in 30 ml of methylene chloride was then added to the mixture at a temperature below 5°C over a period of 15 minutes, and the resulting mixture was stirred at the same temperature for 10 minutes. After this, 1.04 ml of isopropanol were added dropwise, and the mixture was stirred for a further 10 minutes. At the end of this time, the solvent was removed by distillation under reduced pressure, and the resulting residue was diluted with 50 ml of ethyl acetate. The mixture was washed with cold water and with a saturated aqueous solution of sodium chloride, in that order, and the organic layer was separated and dried over anhydrous magnesium sulphate. The solvent was then removed by distillation under reduced pressure, to give 6.89 g of the title compound as an oil.
Infrared Absorption Spectrum (KBr), νₘₐₓ cm⁻¹:
   1809, 1754, 1708, 1524, 1348, 1253.
Nuclear Magnetic Resonance Spectrum (CDCℓ₃, 270 MHz), δ ppm:
   0.03 (9H, singlet);
   1.20 (3H, doublet, J = 6.35 Hz);
   1.28 (3H, doublet, J = 6.83 Hz);
   1.81 - 1.92 (1H, multiplet);
   2.25 - 2.39 (1H, multiplet);
   2.40 - 2.75 (6H, multiplet);
   3.35 - 3.60 (6H, multiplet);
   3.63 - 3.75 (1H, multiplet);
   3.85 - 4.02 (1H, multiplet);
   4.05 - 4.18 (1H, multiplet);
   4.20 - 4.40 (4H, multiplet);
   4.63 & 4.71 (together 1H, two triplets, J = 7.81 & 7.81 Hz);
   5.05 & 5.31 (together 1H, two doublets, J = 13.18 & 13.18 Hz);
   5.22 (1H, doublet, J = 2.44 Hz);
   5.25 (2H, singlet);
   5.35 & 5.43 (together 2H, two doublets, J = 12.90 & 12.90 Hz);
   7.44 & 7.50 (together 2H, two doublets, J = 8.79 & 8.79 Hz);
   7.54 (2H, doublet, J = 8.06 Hz);
   7.57 (2H, doublet, J = 8.06 Hz);
   8.15 - 8.28 (6H, multiplet).

### PREPARATION 17

### 4-Nitrobenzyl (1R,5S,6S)-2-{2(S)-[4-(2-4'-nitrobenzyloxycarbonyloxyethyl)-1-piperazinylcarbonyl]-1-(4-nitrobenzyloxycarbonyl)pyrrolidin-4(S)-ylthio}-6-[(R)-1-(trimethylsilyloxy)ethyl]-1-methyl-1-carbapen-2-em-3-carboxylate

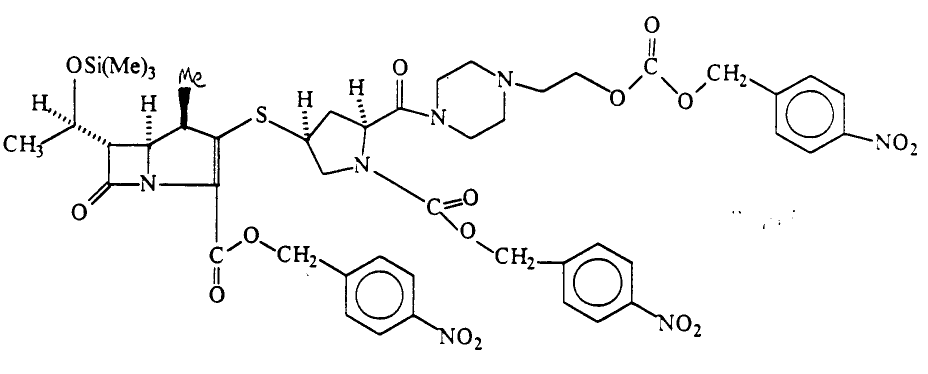

A homogenous mixture of 6.89 g of (3S,4S)-3-[(1R)-1-(trimethylsilyloxy)ethyl]-4-{2(S)-[4-(2-p-nitrobenzyloxycarbonyloxyethyl)piperazin-1-ylcarbonyl]-1-(p-nitrobenzyloxycarbonyl)pyrrolidin-4(S)-yl]-thiocarbonylethyl}-1-(4-nitrobenzyloxyoxalyl)azetidin-2-one (prepared as described in Preparation 16) and 11.2 ml of freshly distilled triethyl phosphite was heated at 60°C for 4 hours, whilst stirring under an atmosphere of nitrogen. At the end of this time, any excess of triethyl phosphite was removed by distillation at a temperature below 30°C and under reduced pressure. The residue was washed three times, each time with 50 ml of hexane, and then again three times, each time with 50 ml of diisopropyl ether. The solvent was then finally removed by decantation, and the mixture was dried by evaporation under reduced pressure, to give 6.68 g of an ylide as a brown oil.

5.18 g of this ylide were dissolved in 350 ml of freshly distilled mesitylene, and the solution was heated on an oil bath kept at 170 - 175°C for 7 hours whilst stirring and under an atmosphere of nitrogen. At the end of this time, the reaction mixture was cooled to room temperature and washed with ice-water and with a saturated aqueous solution of sodium chloride, in that order. The resulting mixture was dried over anhydrous magnesium sulphate and treated with active charcoal; the solvent was then removed by distillation under reduced pressure and at a temperature below 45°C. The residue was washed with diisopropyl ether and dried by evaporation under reduced pressure, to give 4.18 g of the title compound as a brown foam.
Infrared Absorption Spectrum (KBr), νₘₐₓ cm⁻¹:
   1771, 1751, 1712, 1657, 1607, 1522, 1442, 1404, 1377, 1347, 1321.
Nuclear Magnetic Resonance Spectrum (CDCℓ₃, 270 MHz), δ ppm:
   0.12 (9H, singlet);
   1.26 (3H, doublet, J = 6.35 Hz);
   1.27 (3H, doublet, J = 6.35 Hz);
   1.81 - 2.00 (1H, multiplet);
   2.32 - 2.78 (6H, multiplet);
   3.20 - 3.27 (1H, multiplet);
   3.27 - 3.75 (8H, multiplet);
   4.00 - 4.37 (5H, multiplet);
   4.63 - 4.78 (1H, multiplet);
   5.07 & 5.30 (together 1H, two doublets, J = 13.67 & 13.67 Hz);
   5.22 (1H, singlet);
   5.26 (2H, singlet);
   5.25 & 5.47 (together 2H, two doublets, J = 14.15 & 14.15 Hz);
   7.44 & 7.51 (together 2H, two doublets, J = 8.79 & 8.79 Hz);
   7.55 (2H, doublet, J = 8.79 Hz);
   7.65 (2H, doublet, J = 8.79 Hz);
   8.18 - 8.25 (6H, multiplet).

### PREPARATION 18

### 4-Nitrobenzyl (1R,5S,6S)-2-{2(S)-[4-(2-4'-nitrobenzyloxycarbonyloxyethyl)-1-piperazinylcarbonyl]-1-(4-nitrobenzyloxycarbonyl)pyrrolidin-4(S)-ylthio}-6-[(R)-1-hydroxyethyl]-1-methyl-1-carbapen-2-em-3-carboxylate

A solution of 0.704 g of potassium fluoride in 11.6 ml of water and then 1.61 ml of acetic acid were added, whilst ice-cooling, to a solution of 4.18 g of 4-nitrobenzyl (1R,5S,6S)-2-{2(S)-[4-(2-4'-nitrobenzyloxycarbonyloxyethyl)-1-piperazinylcarbonyl]-1-(4-nitrobenzyloxycarbonyl)pyrrolidin-4(S)-ylthio}-6-[(R)-1-(trimethylsilyloxy)ethyl]-1-methyl-1-carbapen-2-em-3-carboxylate (prepared as described in Preparation 17) in 39 ml of acetone, and the resulting mixture was stirred for 45 minutes. At the end of this time, the reaction mixture was freed from the solvent by distillation under reduced pressure. The resulting residue was mixed with ethyl acetate, and the mixture was washed with water and with a saturated aqueous solution of sodium chloride, in that order. The mixture was dried, and the solvent was removed by distillation under reduced pressure. The residue was washed three times with diethyl ether, to give 2.87 g of the title compound as a powder.
Infrared Absorption Spectrum (KBr), νₘₐₓ cm⁻¹:
   1769, 1751, 1710, 1653, 1607, 1521, 1443, 1347.
Nuclear Magnetic Resonance Spectrum (CDCℓ₃, 270 MHz), δ ppm:
   1.27 & 1.28 (together 3H, two doublets, J = 7.33 & 7.33 Hz);
   1.37 (3H, doublet, J = 6.35 Hz);
   1.78 - 1.98 (1H, multiplet);
   2.31 - 2.80 (7H, multiplet);
   3.27 (1H, doublet of doublets, J = 6.83 & 2.44 Hz);
   3.31 - 3.76 (8H, multiplet);
   4.01 - 4.33 (5H, multiplet);
   4.68 & 4.74 (together 1H, two triplets, J = 7.81 & 7.81 Hz);
   5.04 - 5.52 (6H, multiplet);
   7.44 & 7.51 (together 2H, two doublets, J = 8.79 & 8.79 Hz);
   7.55 & 7.65 (together 4H, two doublets, J = 8.79 & 8.79 Hz);
   8.17 - 8.25 (6H, multiplet).

The resulting compound can be converted to a known carbapenem derivative having excellent antibacterial activity by deprotecting the hydroxy- and carboxy-protecting groups by conventional means.

### PREPARATION 19

### Z(O)-1-t-Butyldimethylsilyloxy-1-(2-diethylcarbamoylphenylthio)-1-pronene and its E(O)-isomer

A solution of 729 mg (2.75 mmole) of S-2-diethylcarbamoylphenyl thiopropionate (prepared as described in Preparation 30), 832 mg (5.52 mmole) of t-butyldimethylsilyl chloride and 621 mg (3.47 mmole) of hexamethylphosphoric triamide in 6 ml of tetrahydrofuran was cooled to -78°C, and 3.0 ml (3.0 mmole) of a 1.0 M solution of lithium bis(trimethylsilyl)amide in tetrahydrofuran was added dropwise to the mixture over a period of 7 minutes. The resulting mixture was then stirred at the same temperature for 10 minutes, after which the reaction was terminated by adding 2 ml of a saturated aqueous solution of sodium hydrogencarbonate. The reaction mixture was then mixed with hexane and washed with water to remove tetrahydrofuran and hexamethylphosphoric triamide. The solvent was then removed by distillation under reduced pressure, and the resulting residue was purified by column chromatography through 20 g of alumina, using a 1 : 1 by volume mixture of methylene chloride and hexane as the eluent, to give 922 mg (yield 88%) of the title compound as a colourless oil. By analysis of the nuclear magnetic resonance spectrum (270 MHz), the product was shown to be a mixture of the Z(O)- and E(O)-isomers in the ratio of 4 to 1.
Nuclear Magnetic Resonance Spectrum (CDCℓ₃, 270 MHz), δ ppm:
   0.10 (1.2H, singlet);
   0.11 (4.8H, singlet);
   0.80 (7.2H, singlet);
   0.89 (1.8H, singlet);
   1.07 (3H, triplet, J = 7 Hz);
   1.27 (3H, triplet, J = 7 Hz);
   1.70 (0.8H, doublet, J = 7 Hz);
   1.78 (0.2H, doublet, J = 7 Hz);
   3.16 (1.6H, quartet, J = 7 Hz);
   3.18 (0.4H, quartet, J = 7 Hz);
   3.46 - 3.65 (2H, broad);
   5.35 (1H, quartet, J = 7 Hz);
   7.11 - 7.20 (2H, multiplet);
   7.22 - 7.32 (1H, multiplet);
   7.37 - 7.45 (1H, multiplet).

### PREPARATIONS 20 TO 29

Following a similar procedure to that described in Preparation 19, the following compounds were also prepared.

### PREPARATION 20

### Z(O)-1-t-Butyldimethylsilyloxy-1-(2-dimethylcarbamoylphenylthio)-1-propene and its E(O)-isomer

The yield of the Z(O)-isomer was 85%, and the ratio of the yields of Z(O)-isomer to E(O)-isomer was 4 : 1.
Nuclear Magnetic Resonance Spectrum (CDCℓ₃, 270 MHz), δ ppm:
   0.10 (1.2H, singlet);
   0.11 (4.8H, singlet);
   0.79 (1.8H, singlet);
   0.89 (7.2H, singlet);
   1.70 (2.4H, doublet, J = 7 Hz);
   1.78 (0.6H, doublet, J = 7 Hz);
   2.88 (3H, singlet);
   3.12 (3H, singlet);
   5.33 (0.2 H, quartet, J = 7 Hz);
   5.35 (0.8H, quartet, J = 7 Hz);
   7.15 - 7.20 (2H, multiplet);
   7.24 - 7.33 (1H, multiplet);
   7.40 - 7.47 (1H, multiplet).

### PREPARATION 21

### Z(O)-1-t-Butyldimethylsilyloxy-1-(2-dipropylcarbamoylphenylthio)-1-propene and its E(O)-isomer

The yield of the Z(O)-isomer was 88%, and the ratio of the yields of Z(O)-isomer to E(O)-isomer was 2 : 1.
Nuclear Magnetic Resonance Spectrum (CDCℓ₃, 270 MHz), δ ppm:
   0.10 (2H, singlet);
   0.11 (4H, singlet);
   0.73 (3H, triplet, J = 7 Hz);
   0.81 (3H, singlet);
   0.89 (6H, singlet);
   1.00 (3H, triplet, J = 7 Hz);
   1.43 - 1.60 (2H, multiplet);
   1.62 - 1.80 (2H, multiplet);
   1.70 (2H, doublet, J = 7 Hz);
   1.77 (1H, doublet, J = 7 Hz);
   3.05 (1.3H, triplet, J = 7 Hz);
   3.08 (0.7H, triplet, J = 7 Hz);
   3.46 - 3.60 (2H, broad singlet);
   5.35 (0.67H, quartet, J = 7 Hz);
   5.37 (0.33H, quartet, J = 7 Hz);
   7.10 - 7.19 (2H, multiplet);
   7.22 - 7.31 (1H, multiplet);
   7.36 - 7.44 (1H, multiplet).

### PREPARATION 22

### Z(O)-1-t-Butyldimethylsilyloxy-1-(2-diisobutylcarbamoylphenylthio)-1-propene and its E(O)-isomer

The yield of the Z(O)-isomer was 58%, and the ratio of the yields of Z(O)-isomer to E(O)-isomer was 7 : 1.
Nuclear Magnetic Resonance Spectrum (CDCℓ₃, 270 MHz), δ ppm:
   0.11 (6H, singlet);
   0.75 (5.3H, doublet, J = 7 Hz);
   0.77 (0.7H, doublet, J = 7 Hz);
   0.82 (1.1H, singlet);
   0.90 (7.9H, singlet);
   1.03 (6H, doublet, J = 7 Hz);
   1.71 (2.6H, doublet, J = 7 Hz);
   1.76 (0.4H, doublet, J = 7 Hz);
   1.80 - 1.95 (1H, multiplet);
   2.07 - 2.25 (1H, multiplet);
   2.99 (1.75H, doublet, J = 8 Hz);
   3.02 (0.25H, doublet, J = 8 Hz);
   3.10 - 3.70 (2H, broad singlet);
   5.36 (0.88H, quartet, J = 7 Hz);
   5.42 (0.12H, quartet, J = 7 Hz);
   7.09 - 7.22 (2H, multiplet);
   7.23 - 7.30 (1H, multiplet);
   7.34 - 7.42 (1H, multiplet).

### PREPARATION 23

### Z(O)-1-t-Butyldimethylsilyloxy-1-(2-N-methyl-N-phenylcarbamoylphenylthio)-1-propene and its E(O)-isomer

The yield of the Z(O)-isomer was 87%, and the ratio of the yields of Z(O)-isomer to E(O)-isomer was 9 : 1.
Nuclear Magnetic Resonance Spectrum (CDCℓ₃, 270 MHz), δ ppm:
   0.10 (0.6H, singlet);
   0.15 (5.4H, singlet);
   0.84 (0.9H, singlet);
   0.92 (8.1H, singlet);
   1.69 (2.7H, doublet, J = 7 Hz);
   1.79 (0.3H, doublet, J = 7 Hz);
   3.35 - 3.55 (3H, broad singlet);
   5.34 (0.9H, quartet, J = 7 Hz);
   5.39 (0.1H, quartet, J = 7 Hz);
   6.85 - 7.27 (8H, broad);
   7.31 - 7.38 (1H, broad).

### PREPARATION 24

### Z(O)-1-t-Butyldimethylsilyloxy-1-[2-(1-pyrrolidinyl)carbonylphenylthio]-1-propene and its E(O)-isomer

The yield of the Z(O)-isomer was 94%, and the ratio of the yields of Z(O)-isomer to E(O)-isomer was 5 : 1.
Nuclear Magnetic Resonance Spectrum (CDCℓ₃, 270 MHz), δ ppm:
   0.10 (6H, singlet);
   0.79 (1.5H, singlet);
   0.88 (7.5H, singlet);
   1.69 (2.5H, doublet, J = 7 Hz);
   1.79 (0.5H, doublet, J = 7 Hz);
   1.80 - 2.02 (4H, multiplet);
   3.25 (2H, triplet, J = 7 Hz);
   3.65 (2H, triplet, J = 7 Hz);
   5.33 (0.17H, quartet, J = 7 Hz);
   5.36 (0.83H, quartet, J = 7 Hz);
   7.12 - 7.33 (3H, multiplet);
   7.39 - 7.47 (1H, multiplet).

### PREPARATION 25

### Z(O)-1-t-Butyldimethylsilyloxy-1-[2-(1-piperidyl)carbonylphenylthio]-1-propene and its E(O)-isomer

The yield of the Z(O)-isomer was 94%, and the ratio of the yields of Z(O)-isomer to E(O)-isomer was 3 : 1.
Nuclear Magnetic Resonance Spectrum (CDCℓ₃, 270 MHz), δ ppm:
   0.10 (1.5H, singlet);
   0.11 (4.5H, singlet);
   0.80 (2.3H, singlet);
   0.89 (6.7H, singlet);
   1.40 - 1.73 (6H, broad);
   1.69 (2.25H, doublet, J = 7 Hz);
   1.77 (0.75H, doublet, J = 7 Hz);
   3.15 - 3.28 (2H, broad);
   3.55 - 3.95 (2H, broad);
   5.34 (0.25H, quartet, J = 7 Hz);
   5.35 (0.75H, quartet, J = 7 Hz);
   7.10 - 7.19 (2H, multiplet);
   7.22 - 7.32 (1H, multiplet);
   7.38 - 7.46 (1H, multiplet).

### PREPARATION 26

### Z(O)-1-t-Butyldimethylsilyloxy-1-(2-morpholinocarbonylphenylthio]-1-propene and its E(O)-isomer

The yield of the Z(O)-isomer was 99%, and the ratio of the yields of Z(O)-isomer to E(O)-isomer was 5 : 1.
Nuclear Magnetic Resonance Spectrum (CDCℓ₃, 270 MHz), δ ppm:
   0.10 (1H, singlet);
   0.11 (5H, singlet);
   0.79 (1.5H, singlet);
   0.89 (7.5H, singlet);
   1.70 (2.5H, doublet, J = 7 Hz);
   1.78 (0.5H, doublet, J = 7 Hz);
   3.18 - 3.40 (2H, broad);
   3.52 - 3.85 (2H, broad singlet);
   3.65 - 3.90 (4H, broad singlet);
   5.34 (0.17H, quartet, J = 7 Hz);
   5.35 (0.83H, quartet, J = 7 Hz);
   7.13 - 7.21 (2H, multiplet);
   7.25 - 7.36 (1H, multiplet);
   7.40 - 7.47 (1H, multiplet).

### PREPARATION 27

### Z(O)-1-t-Butyldimethylsilyloxy-1-[2-(1-azepinyl)carbonylphenylthio]-1-propene and its E(O)-isomer

The yield of the Z(O)-isomer was 86%, and the ratio of the yields of Z(O)-isomer to E(O)-isomer was 3 : 1.
Nuclear Magnetic Resonance Spectrum (CDCℓ₃, 270 MHz), δ ppm:
   0.10 (1.5H, singlet);
   0.12 (4.5H, singlet);
   0.79 (2.3H, singlet);
   0.90 (6.7H, singlet);
   1.53 - 1.72 (6H, broad);
   1.69 (2.25H, doublet, J = 7 Hz);
   1.78 (0.75H, doublet, J = 7 Hz);
   1.75 - 1.90 (2H, broad);
   3.19 - 3.35 (2H, broad);
   3.55 - 3.80 (2H, broad);
   5.33 (0.25H, quartet, J = 7 Hz);
   5.34 (0.75H, quartet, J = 7 Hz);
   7.10 - 7.19 (2H, multiplet);
   7.21 - 7.33 (1H, multiplet);
   7.38 - 7.46 (1H, multiplet).

### PREPARATION 28

### Z(O)-1-t-Butyldimethylsilyloxy-1-(2-diethylcarbamoyl-6-methylphenylthio)-1-propene and its E(O)-isomer

The yield of the Z(O)-isomer was 76%, and the ratio of the yields of Z(O)-isomer to E(O)-isomer was 11 : 1.
Nuclear Magnetic Resonance Spectrum (CDCℓ₃, 270 MHz), δ ppm:
   0.21 (3H, singlet);
   0.23 (3H, singlet);
   0.96 (9H, singlet);
   1.03 (3H, triplet, J = 7 Hz);
   1.27 (3H, triplet, J = 7 Hz);
   1.49 (3H, doublet, J = 7 Hz);
   2.51 (3H, singlet);
   3.08 (1H, doublet of quartets, J = 14 & 7 Hz);
   3.11 (1H, doublet of quartets, J = 14 & 7 Hz);
   3.43 (1H, doublet of quartets, J = 14 & 7 Hz);
   3.70 (1H, doublet of quartets, J = 14 & 7 Hz);
   4.12 (1H, quartet, J = 7 Hz);
   7.08 - 7.13 (1H, multiplet);
   7.25 - 7.32 (2H, multiplet).

### PREPARATION 29

### Z(O)-1-t-Butyldimethylsilyloxy-1-[2-diethyl(thiocarbamoyl)-6-methylphenylthio)-1-propene and its E(O)-isomer

The yield of the Z(O)-isomer was 100%, and the ratio of the yields of Z(O)-isomer to E(O)-isomer was 20 : 1.
Nuclear Magnetic Resonance Spectrum (CDCℓ₃, 270 MHz), δ ppm:
   0.12 (3H, singlet);
   0.14 (3H, singlet);
   0.80 (9/21H, singlet);
   0.90 (180/21H, singlet);
   1.11 (3H, triplet, J = 7 Hz);
   1.40 (3H, triplet, J = 7 Hz);
   1.70 (60/21H, doublet, J = 7 Hz);
   1.78 (3/21H, doublet, J = 7 Hz);
   3.29 (1H, doublet of quartets, J = 7 & 14 Hz);
   3.44 (1H, doublet of quartets, J = 7 & 14 Hz);
   3.67 (1H, doublet of quartets, J = 7 & 14 Hz);
   4.57 (1H, doublet of quartets, J = 7 & 14 Hz);
   5.37 (1H, quartet, J = 7 Hz);
   7.10 - 7.25 (3H, multiplet);
   7.31 - 7.40 (1H, multiplet).

### PREPARATION 30

### S-2-Diethylcarbamoylphenyl thiopropionate

### Method A:

A solution of 34.84 g (0.476 mole) of diethylamine and 70.0 ml (0.502 mole) of triethylamine in 50 ml of methylene chloride was added dropwise, over a period of 1 hour, to an ice-cooled suspension of 71.62 g (0.209 mole) of 2,2'-dithiobenzoyl chloride in 300 ml of methylene chloride, and the resulting mixture was stirred at the same temperature for a further 30 minutes. At the end of this time, the solvent was removed by distillation under reduced pressure, and the resulting residue was diluted with ethyl acetate. The resulting organic solution was washed with water and with a saturated aqueous solution of sodium chloride, in that order. The solvent was again distilled off to give an amide compound. About 4 g of this amide compound was mixed with 14.36 g (0.220 mole) of zinc powder and 80.0 ml (0.624 mole) of propionic anhydride, and the mixture was heated at 100°C for 5 minutes. In the course of this reaction, the zinc in the reaction mixture was activated. The remainder of the amide compound was dissolved in 100 ml of benzene, and the resulting solution was then added dropwise to the reaction mixture at the same temperature over a period of 20 minutes. The resulting mixture was then heated under reflux for 90 minutes, after which it was cooled. The crystals which separated were collected by filtration and washed with ethyl acetate. The filtrate and the washings were combined, and the resulting mixture was washed with water; the solvent was then removed by distillation under reduced pressure. The residue was then subjected to fractional distillation under reduced pressure, to give 106.97 g of the title compound, boiling at 167 - 170°C/0.95 - 1.1 mmHg (12.7 - 14.7 Pa), representing a total yield of 96% over the two steps.
Infrared Absorption Spectrum (liquid), νₘₐₓ cm⁻¹:
   1710, 1635, 1292, 932.
Nuclear Magnetic Resonance Spectrum (CDCℓ₃, 270 MHz), δ ppm:
   1.02 (3H, triplet, J = 7 Hz);
   1.20 (3H, triplet, J = 7 Hz);
   1.23 (3H, triplet, J = 7 Hz);
   2.66 (2H, quartet, J = 7 Hz);
   2.90 - 3.20 (2H, broad);
   3.10 - 4.00 (2H, broad);
   7.29 - 7.36 (1H, multiplet);
   7.40 - 7.52 (1H, multiplet).
Mass spectrum (m/z): 266 (M⁺ + 1), (M⁺, C₁₄H₁₉NO₂S).

### Method B:

### 30B(i) N,N-diethyl-2-benzoylthiobenzamide

2.85 g (20.3 mmole) of benzoyl chloride were added dropwise, whilst ice-cooling, to a solution of 3.13 g (20.3 mmole) of thiosalicylic acid and 2.46 g (24.4 mmole) of triethylamine in 60 ml of methylene chloride, and the resulting mixture was stirred at room temperature for 1 hour. At the end of this time, the reaction mixture was washed twice with 0.2 N aqueous hydrochloric acid and once with a saturated aqueous solution of sodium chloride, and the solvent was removed by distillation under reduced pressure, to give 5.24 g of benzoylthiobenzoic acid as crude crystals in a quantitative yield. The whole of this product was dissolved in 100 ml of methylene chloride, and 5.45 g (21.3 mmole) of 2-chloro-1-methylpyridinium iodide, 1.78 g (24.4 mmole) of diethylamine and 4.51 g (44.7 mmole) of triethylamine were added, in that order, to the resulting solution, whilst ice-cooling. The resulting mixture was then stirred at room temperature for 20 hours, after which the solvent was removed by distillation under reduced pressure. The residue was partitioned between ethyl acetate and dilute aqueous hydrochloric acid, and the organic layer was washed with water. The solvent was removed by distillation under reduced pressure, and the residue was purified by column chromatography through 120 g of silica gel, using a gradient elution method with mixtures of hexane and acetone ranging from 4 : 1 to 3 : 1 by volume as the eluent, to give 4.86 g of the title compound as an oil in a 76% yield.
Nuclear Magnetic Resonance Spectrum (CDCℓ₃, 60 MHz), δ ppm:
   0.99 (3H, triplet, J = 7 Hz);
   1.02 (3H, triplet, J = 7 Hz);
   3.09 (2H, quartet, J = 7 Hz);
   3.0 - 3.9 (2H, broad);
   7.2 - 7.7 (7.5H, multiplet);
   7.9 - 8.2 (1.5H, multiplet).

### 30B(ii) S-2-Diethylcarbamoylphenyl thiopropionate

0.84 g (15.5 mmole) of sodium methoxide was added, whilst ice-cooling, to a solution of 4.86 g (15.5 mmole) of N,N-diethyl-2-benzoylthiobenzamide [prepared as described in step (i) above] in 60 ml of methanol, and the resulting mixture was stirred for 20 minutes. At the end of this time, the reaction mixture was neutralised by adding about 15 drops of concentrated aqueous hydrochloric acid. Ethanol and was then added, and the solvent was removed by distillation under reduced pressure. In order to eliminate moisture, the residue was mixed with 20 ml of ethanol and 30 ml of benzene and the solvent was removed by distillation under reduced pressure. The resulting residue, containing N,N-diethyl-2-mercaptobenzamide, was suspended in 60 ml of methylene chloride, and 4.30 g (46.5 mmole) of propionyl chloride and 6.26 g (62.0 mmole) of triethylamine were added, whilst ice-cooling. The reaction mixture was then stirred for 2.5 hours, after which the reaction was terminated by adding water. The reaction mixture was then diluted with methylene chloride and the organic layer was washed first with dilute aqueous hydrochloric acid and then with water. The solvent was removed by distillation under reduced pressure, and the resulting residue was purified by column chromatography through 100 g of silica gel, to give 3.26 g (yield 79%) of the title compound. The product obtained by this method was identical with that prepared in Method A.

### PREPARATIONS 31 TO 38

Following the procedure described in Preparation 30 (Method A or B), the following compounds were prepared. In these Preparations, only the infrared carbonyl group absorption is reported.

### PREPARATION 31

### S-2-Dimethylcarbamoylphenyl thiopropionate

The title compound was obtained in a total yield of 78% for the two steps.
Infrared Absorption Spectrum (liquid), νₘₐₓ cm⁻¹:
   1709, 1639.

### PREPARATION 32

### S-2-Dipropylcarbamoylphenyl thiopropionate

The title compound was obtained in a total yield of 93% for the two steps.
Infrared Absorption Spectrum (liquid), νₘₐₓ cm⁻¹:
   1705, 1632.

### PREPARATION 33

### S-2-Diisobutylcarbamoylphenyl thiopropionate

The title compound was obtained in a total yield of 99% for the two steps.
Infrared Absorption Spectrum (liquid), νₘₐₓ cm⁻¹:
   1712, 1635.

### PREPARATION 34

### S-2-(N-Methyl-N-phenylcarbamoyl)phenyl thiopropionate

The title compound was obtained in a total yield of 96% for the two steps.
Infrared Absorption Spectrum (liquid), νₘₐₓ cm⁻¹:
   1705, 1645.

### PREPARATION 35

### S-2-(1-Pyrrolidinylcarbonyl)phenyl thiopropionate

The title compound was obtained in a total yield of 74% for the two steps.
Infrared Absorption Spectrum (liquid), νₘₐₓ cm⁻¹:
   1702, 1630.

### PREPARATION 36

### S-2-(1-Piperidylcarbonyl)phenyl thiopropionate

The title compound was obtained in a total yield of 98% for the two steps.
Infrared Absorption Spectrum (liquid), νₘₐₓ cm⁻¹:
   1702, 1630.

### PREPARATION 37

### S-2-Morpholinocarbonylphenyl thiopropionate

The title compound was obtained in a total yield of 89% for the two steps.
Infrared Absorption Spectrum (liquid), νₘₐₓ cm⁻¹:
   1702, 1635.

### PREPARATION 38

### S-2-(1-Azepinylcarbonyl)phenyl thiopropionate

The title compound was obtained in a total yield of 93% for the two steps.
Infrared Absorption Spectrum (liquid), νₘₐₓ cm⁻¹:
   1705, 1630.

### PREPARATION 39

### (3S,4S)-3-[(1R)-1-(t-Butyldimethylsilyloxy)ethyl]-4-{2(S)-[4-(2-p-nitrobenzyloxycarbonyloxyethyl)piperazin-1-ylcarbonyl]-1-(p-nitrobenzyloxycarbonyl)pyrrolidin-4(S)-yl]-thiocarbonylethyl}azetidin-2-one

A solution of 99 mg (0.20 mmole) of S-2-diethylcarbamoylphenyl 2(R)-{(3S,4S)-3-[1(R)-(t-butyldimethylsilyloxy)ethyl]-2-oxo-4-azetidinyl}thiopropionate (prepared as described in Example 6), 135 mg (0.22 mmole) of (2S,4S)-2-{4-[2-(4-nitrobenzyloxycarbonyl)oxyethyl]-1-piperazinylcarbonyl}-4-mercapto-1-(4-nitrobenzyloxycarbonyl)pyrrolidine and 30 mg (0.30 mmole) of triethylamine in 2 ml of methylene chloride was stirred at room temperature for 17 hours. At the end of this time, the solvent was removed by distillation under reduced pressure, and the residue was dissolved in ethyl acetate. The resulting solution was washed with a 2 N aqueous solution of sodium hydroxide, with water and with a saturated aqueous solution of sodium chloride, in that order. The solvent was then removed by distillation under reduced pressure, and the residue was purified by column chromatography through 25 g of silica gel, using a 3 : 2 by volume mixture of acetone and hexane as the eluent, to give 182 mg of the title compound as a foam in a quantitative yield.

### PREPARATION 40

### (3S,4S)-3-[(1R)-1-Hydroxyethyl]-4-{2(S)-[4-(2-p-nitrobenzyloxycarbonyloxyethyl)piperazin-1-ylcarbonyl]-1-(p-nitrobenzyloxycarbonyl)pyrrolidin-4(S)-yl]-thiocarbonylethyl}azetidin-2-one

24 ml of 3 N aqueous hydrochloric acid were added dropwise, whilst ice-cooling and stirring, to a solution of 8.1 g of (3S,4S)-3-[(R)-1-(t-butyldimethylsilyloxy)ethyl]-4-{1(R)-{2(S)-[4-(2-4'-nitrobenzyloxycarbonyloxyethyl)piperazin-1-ylcarbonyl]-1-(4-nitrobenzyloxycarbonyl)pyrrolidin-4(S)-ylthiocarbonyl}ethyl}azetidin-2-one (prepared as described in Preparation 39) in 80 ml of methanol, and the mixture was stirred at the same temperature for 30 minutes and then allowed to stand overnight in a refrigerator. At the end of this time, the reaction mixture was ice-cooled and its pH was adjusted to a value of 5 to 6 by the addition of sodium hydrogencarbonate. The mixture was then concentrated by evaporation under reduced pressure, and the resulting residue was mixed with a small amount of water and extracted with ethyl acetate. The aqueous layer was saturated with sodium chloride and extracted with ethyl acetate. The extracts were combined and then freed from the solvent by distillation under reduced pressure. The residue was purified by column chromatography through 150 g of silica gel, using a gradient elution method with mixtures of ethyl acetate and methanol ranging from 20 : 1 to 10 : 1 by volume as the eluent, to give 5.9 g of the title compound as a colourless foam.
Infrared Absorption Spectrum (CHCℓ₃), νₘₐₓ cm⁻¹:
   1752, 1710, 1650, 1607, 1522, 1443, 1405, 1347, 1263.
Nuclear Magnetic Resonance Spectrum (CDCℓ₃, 270 MHz), δ ppm:
   1.27 (3H, doublet, J = 6.83 Hz);
   1.28 (3H, doublet, J = 6.35 Hz);
   1.82 - 1.99 (1H, multiplet);
   2.10 - 2.18 (1H, multiplet);
   2.40 - 2.95 (7H, multiplet);
   3.03 (1H, doublet of doublets, J = 1.95 & 6.35 Hz);
   3.37 - 3.80 (5H, multiplet);
   3.78 (1H, doublet of doublets, J = 1.95 & 6.84 Hz);
   3.95 - 4.48 (6H, multiplet);
   4.68 & 4.73 (together 1H, two triplets, J = 8.06 & 7.33 Hz);
   5.06 & 5.32 (together 1H, two doublets, J = 13.43 & 13.43 Hz);
   5.21 (1H, singlet);
   5.26 (2H, singlet);
   5.99 (1H, broad);
   7.45 & 7.50 (together 2H, two doublets, J = 8.30 & 8.79 Hz);
   7.56 (2H, doublet, J = 8.79 Hz);
   8.18 - 8.26 (4H, multiplet).

### PREPARATION 41

### S-2-diethylaminocarbonyl-6-methylphenyl thiopropionate

Following a procedure similar to that described in Preparation 30 (Method A), the title compound was obtained in a yield of 38%
Infrared Absorption Spectrum (KBr), νₘₐₓ cm⁻¹:
   1703, 1635.

### PREPARATION 42

### S-2-diethylaminothiocarbonylphenyl thiopropionate

216 mg (0.534 mmole) of Lawesson's reagent were added to a solution of 276 mg (1.04 mmole) of S-2-diethylcarbamoylphenyl thiopropionate (prepared as described in Preparation 30) in 5 ml of toluene, and the resulting mixture was stirred at 100°C for 20 minutes. It was then cooled, and the resulting mixure was purified by column chromatography through 25 g of silica gel, using a gradient elution method, with mixtures of methylene chloride and hexane ranging from 3 : 0 to 3 : 1 by volume as the eluent, to give 286 mg of the title compound, melting at 67.5 - 68.5°C.
Infrared Absorption Spectrum (Nujol), νₘₐₓ cm⁻¹:
   1712, 1505, 1308, 1242, 928.
Nuclear Magnetic Resonance Spectrum (CDCℓ₃, 270 MHz), δ ppm:
   1.08 (3H, triplet, J = 7 Hz),
   1.20 (3H, triplet, J = 7 Hz),
   1.37 (3H, triplet, J = 7 Hz),
   2.66 (2H, quartet, J = 7 Hz),
   3.19 (1H, doublet of quartets, J = 14 & 7 Hz),
   3.38 (1H, doublet of quartets, J = 14 & 7 Hz),
   3.70 (1H, doublet of quartets, J = 14 & 7 Hz),
   4.53 (1H, doublet of quartets, J = 14 & 7 Hz),
   7.25 (1H, doublet of quartets, J = 7 & 3 Hz),
   7.31 - 7.47 (3H, multiplet).
Mass spectrum (m/z) : 281(M⁺, C₁₄H₁₉NOS₂).

### PREPARATION 43

### 2-Diethylcarbamoylphenyl propionate

2.0 ml (23 mmole) of oxalyl chloride and 0.050 ml of N,N-dimethylformamide were added, whilst ice-cooling, to a solution of 2.40 g (12.4 mmole) of 2-propionyloxybenzoic acid in 24 ml of methylene chloride, and the resulting mixture was stirred for 1 hour. At the end of this time, the solvent and excess oxalyl chloride were removed by distillation under reduced pressure, and 20 ml of methylene chloride were added to the residue. 1.36 g (13.5 mmole) of triethylamine and 986 mg (13.5 mmole) of diethylamine were then added, whilst ice-cooling, to the resulting solution, and the mixture was stirred for 1 hour. The mixture was then diluted with ethyl acetate, and the organic layer was washed with water. The solvent was removed by distillation under reduced pressure, and the residue was purified by column chromatography through 25 g of silica gel, using a gradient elution method, with mixtures of methylene chloride and ethyl acetate ranging from 10 : 1 to 7 : 1 by volume as the eluent, to give 3.0 g (yield 97%) of 2-diethylaminocarbonylphenyl propionate as an oil.
Infrared Absorption Spectrum (liquid film), νₘₐₓ cm⁻¹:
   1765, 1638, 1430, 1293, 1142.
Nuclear Magnetic Resonance Spectrum (CDCℓ₃, 60 MHz), δ ppm:
   1.06 (3H, triplet, J = 7 Hz);
   1.20 (6H, triplet, J = 7 Hz),
   2.52 (2H, quartet, J = 7 Hz),
   3.15 (2H, quartet, J = 7 Hz),
   3.49 (2H, quartet, J = 7 Hz),
   7.0 - 7.6 (4H, multiplet).
Mass spectrum (m/z) : 249(M⁺, C₁₄H₁₉NO₃).

## Claims

1. A compound of formula (Ia): 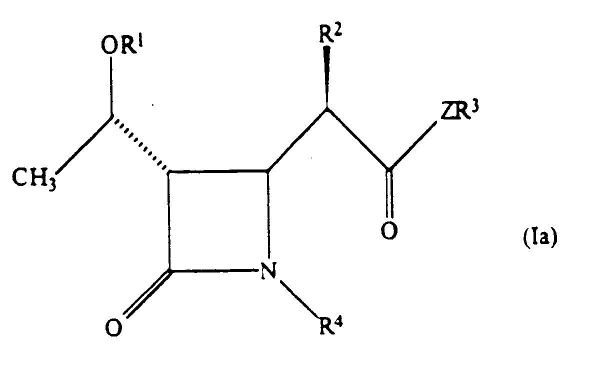 in which:
R¹ represents a hydrogen atom or a hydroxy-protecting group;
R² represents a methyl group;
R³ represents:
a phenyl group which has a substituent, at the 2-position on the ring, of formula -CYNR⁵R⁶ and no further substituent or has one alkyl substituent having from 1 to 6 carbon atoms, wherein Y represents an oxygen or sulphur atom, and R⁵ and R⁶ are the same or different and each represents an alkyl group having from 1 to 6 carbon atoms, an aryl group as defined below, or R⁵ and R⁶ together with the nitrogen atom to which they are attached form a group selected from the group comprising pyrrolidino, piperidino, morpholino and azepino groups;
R⁴ represents a hydrogen atom or an amino-protecting group; and
Z represents a sulphur atom or an oxygen atom;
said aryl groups have from 6 to 10 carbon atoms in at least one aromatic ring and are unsubstituted.

2. A compound according to Claim 1, in which R¹ represents:
a hydrogen atom;
an aliphatic acyl group having from 1 to 25 carbon atoms;
a halogenated alkanoyl group having from 2 to 6 carbon atoms;
an alkoxyalkanoyl group in which the alkoxy part has from 1 to 5 carbon atoms and the alkanoyl part has from 2 to 6 carbon atoms;
an alkenoyl or alkynoyl group having from 3 to 6 carbon atoms;
an aromatic acyl group, in which the aryl part has from 6 to 14 ring carbon atoms and is a carbocyclic aromatic group, which is unsubstituted or has from 1 to 5 of substituents (b), defined below;
a heterocyclic group having 5 or 6 ring atoms, of which 1 or 2 are oxygen and/or sulphur and/or nitrogen hetero-atoms, which groups are unsubstituted or are substituted by at least one of substituents (b), defined below, and/or by an oxygen atom;
a tri-substituted silyl group, in which all three or two or one of the substituents are alkyl groups having from 1 to 5 carbon atoms, and, correspondingly, none, one or two of the substituents are aryl groups, in which the aryl part has from 6 to 14 ring carbon atoms and is a carbocyclic aromatic group, which is unsubstituted or has from 1 to 5 of substituents (b), defined below;
an alkoxyalkyl group, in which the alkoxy and alkyl parts each have from 1 to 5 carbon atoms;
a lower alkoxy-substituted lower alkoxymethyl group, in which each alkoxy part has from 1 to 5 carbon atoms;
a halogenated lower alkoxymethyl group, in which the alkoxy part has from 1 to 5 carbon atoms;
a halogenated ethyl group;
an arylselenyl-substituted ethyl group, in which the aryl part has from 6 to 14 ring carbon atoms and is a carbocyclic aromatic group, which is unsubstituted or has from 1 to 5 of substituents (b), defined below;
an aralkyl group, in which the alkyl part has from 1 to 4 carbon atoms and which is substituted with from 1 to 3 aryl groups, in which the aryl part has from 6 to 14 ring carbon atoms and is a carbocyclic aromatic group, which is unsubstituted or has from 1 to 5 of substituents (b), defined below;
an alkoxycarbonyl group having from 2 to 7 carbon atoms and which is unsubstituted or is substituted with a halogen atom or a tri-substituted silyl group, as defined above;
an alkenyloxycarbonyl group in which the alkenyl part has from 2 to 6 carbon atoms;
a sulpho group; or
an aralkyloxycarbonyl group, in which the alkyl part has from 1 to 4 carbon atoms and which is substituted with from 1 to 3 aryl groups, in which the aryl part has from 6 to 14 ring carbon atoms and is a carbocyclic aromatic group, which is unsubstituted or has from 1 to 5 of substituents (b), defined below;
said substituents (b) are selected from alkyl groups having from 1 to 6 carbon atoms, alkoxy groups having from 1 to 6 carbon atoms, halogen atoms, cyano groups, nitro groups, hydroxy groups, amino groups, alkylamino groups in which the alkyl part has from 1 to 4 carbon atoms, dialkylamino groups in which each alkyl part has from 1 to 4 carbon atoms, alkylenedioxy groups having from 1 to 3 carbon atoms, alkoxycarbonyl groups having from 2 to 7 carbon atoms and which are unsubstituted or are substituted with a halogen atom or a tri-substituted silyl group, and carbocyclic aryl groups which have from 6 to 14 ring carbon atoms and are unsubstituted or have from 1 to 5 of substituents (b), other than an aryl group.

3. A compound according to Claim 1 or Claim 2, in which Z represents a sulphur atom.

4. A compound according to Claim 1, in which R¹ represents:
a hydrogen atom;
a tri-substituted silyl group, in which all three or two or one of the substituents are alkyl groups having from 1 to 5 carbon atoms, and none, one or two of the substituents are aryl groups, as defined in Claim 2;
an aliphatic acyl group having from 1 to 6 carbon atoms;
a halogenated alkanoyl group having from 2 to 6 carbon atoms;
an alkoxyalkanoyl group in which the alkoxy part has from 1 to 5 carbon atoms and the alkanoyl part has from 2 to 6 carbon atoms; or
an aralkyloxycarbonyl group, in which the aryl part is as defined in Claim 2, and the alkyl part has from 1 to 4 carbon atoms.

5. A compound according to any one of Claims 1 to 4, in which R⁴ represents a hydrogen atom or a tri-substituted silyl group, in which all three or two or one of the substituents are alkyl groups having from 1 to 5 carbon atoms, and none, one or two of the substituents are aryl groups, as defined in Claim 1.

6. A compound according to Claim 1, in which:
R³ represents a phenyl group which has a substituent, at the 2-position on the ring, of formula -CYNR⁵R⁶ and no further substituent or a methyl substituent, wherein Y, R⁵ and R⁶ are as defined in Claim 1; and
Z represents a sulphur atom.

7. A compound according to Claim 6, in which:
R¹ represents:
a hydrogen atom;
a tri-substituted silyl group, in which all three or two or one of the substituents are alkyl groups having from 1 to 5 carbon atoms, and none, one or two of the substituents are aryl groups, as defined below;
an aliphatic acyl group having from 1 to 6 carbon atoms;
a halogenated alkanoyl group having from 2 to 6 carbon atoms;
an alkoxyalkanoyl group in which the alkoxy part has from 1 to 5 carbon atoms and the alkanoyl part has from 2 to 6 carbon atoms; or
an aralkyloxycarbonyl group, in which the aryl part is as defined below, and the alkyl part has from 1 to 4 carbon atoms;
R³ represents:
a phenyl group which has a substituent, at the 2-position on the ring, of formula -CONR⁵R⁶ and no further substituent or a methyl substituent, where R⁵ and R⁶ are the same or different and each represents an alkyl group having from 1 to 3 carbon atoms, a phenyl group, or R⁵ and R⁶ together with the nitrogen atom to which they are attached form a group selected from the group comprising pyrrolidino, piperidino, morpholino and azepino groups;
R⁴ represents a hydrogen atom or a tri-substituted silyl group, in which all three or two or one of the substituents are alkyl groups having from 1 to 5 carbon atoms, and none, one or two of the substituents are aryl groups, as defined below;
said aryl groups are carbocyclic aryl groups which have from 6 to 10 carbon atoms in at least one aromatic ring and which are unsubstituted.

8. A compound according to Claim 7, in which:
R³ represents:
a phenyl group which has a substituent, at the 2-position on the ring, of formula -CONR⁵R⁶ and no further substituent or a methyl substituent, where R⁵ and R⁶ are the same or different and each represents an alkyl group having from 1 to 3 carbon atoms, or R⁵ and R⁶ together with the nitrogen atom to which they are attached form a group selected from the group comprising pyrrolidino, piperidino, morpholino and azepino groups; and
R⁴ represents a hydrogen atom.

9. A compound according to claim 1, selected from:
S-2-diethylcarbamoylphenyl 2(R)-{(3S,4S)-3-[1(R)-(t-butyldimethylsilyloxy)ethyl]-2-oxo-4-azetidinyl}thiopropionate;
S-2-dipropylcarbamoylphenyl 2(R)-{(3S,4S)-3-[1(R)-(t-butyldimethylsilyloxy)ethyl]-2-oxo-4-azetidinyl}thiopropionate;
2-diethylcarbamoylphenyl 2(R)-{(3S,4S)-3-[1(R)-(t-butyldimethylsilyloxy)ethyl]-2-oxo-4-azetidinyl}propionate;
S-2-[diethyl(thiocarbamoyl)]phenyl 2(R)-{(3S,4S)-3-[1(R)-(t-butyldimethylsilyloxy)ethyl]-2-oxo-4-azetidinyl}thiopropionate;
S-2-diethylcarbamoylphenyl 2(R)-{(3S,4S)-3-[1(R)-hydroxyethyl]-2-oxo-4-azetidinyl}thiopropionate;
S-2-(1-pyrrolidinylcarbonyl)phenyl 2(R)-{(3S,4S)-3-[1(R)-(t-butyldimethylsilyloxy)ethyl]-2-oxo-4-azetidinyl}thiopropionate;
S-2-(1-piperidylcarbonyl)phenyl 2(R)-{(3S,4S)-3-[1(R)-(t-butyldimethylsilyloxy)ethyl]-2-oxo-4-azetidinyl}thiopropionate;
S-2-(1-azepinylcarbonyl)phenyl 2(R)-{(3S,4S)-3-[1(R)-(t-butyldimethylsilyloxy)ethyl]-2-oxo-4-azetidinyl}thiopropionate; and
S-2-morpholinocarbonylphenyl 2(R)-{(3S,4S)-3-[1(R)-(t-butyldimethylsilyloxy)ethyl]-2-oxo-4-azetidinyl}thiopropionate.

10. A process for preparing a compound according to any one of the preceding Claims, which comprises reacting a compound of formula (II): 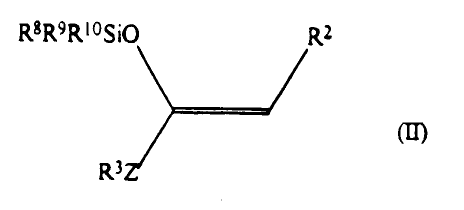 (in which R², R³ and Z are as defined in Claim 1, and R⁸, R⁹ and R¹⁰ are the same or different and each represents an alkyl group having from 1 to 4 carbon atoms or a phenyl group) with a compound of formula (IIIa): 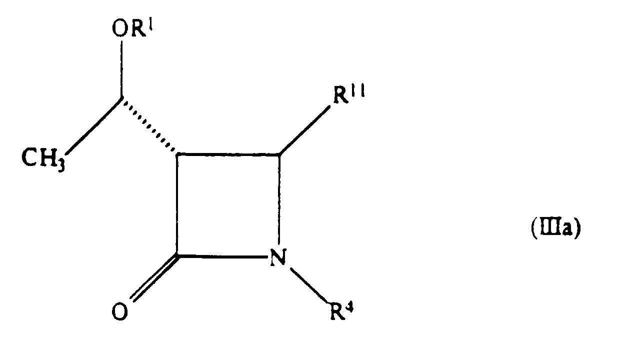 (in which R¹ and R⁴ are as defined in Claim 1, and R¹¹ represents an acyloxy, alkylsulphonyl, arylsulphonyl, alkylsulphinyl or arylsulphinyl group).

11. A process for preparing a carbapenem compound of formula (XIX'): 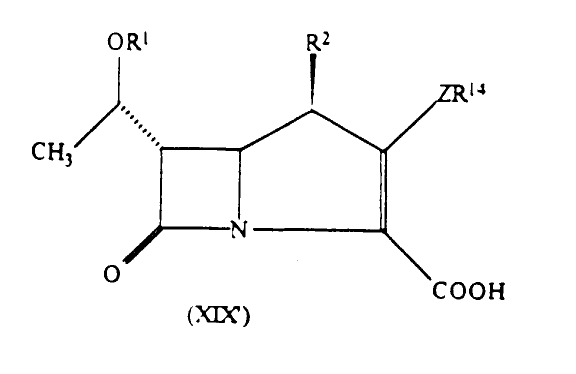 in which R¹, R² and Z are as defined in Claim 1 and R¹⁴ represents a variety of organic groups of the type commonly employed, at the indicated position, in carbapenem derivatives, which process comprises reacting a compound of formula (Ia), as defined in any one of Claims 1 to 9, with a mercapto compound of formula R¹⁴SH, and cyclising the product.

## Claims (Claims for the following Contracting State(s): ES, GR)

1. A process for preparing a compound of formula (Ia): 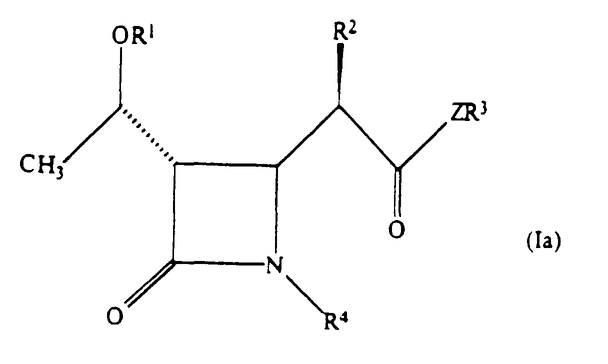 in which:
R¹ represents a hydrogen atom or a hydroxy-protecting group;
R² represents a methyl group;
R³ represents:
a phenyl group which has a substituent, at the 2-position on the ring, of formula -CYNR⁵R⁶ and no further substituent or has one alkyl substituent having from 1 to 6 carbon atoms, wherein Y represents an oxygen or sulphur atom, and R⁵ and R⁶ are the same or different and each represents an alkyl group having from 1 to 6 carbon atoms, an aryl group as defined below, or R⁵ and R⁶ together with the nitrogen atom to which they are attached form a group selected from the group comprising pyrrolidino, piperidino, morpholino and azepino groups;
R⁴ represents a hydrogen atom or an amino-protecting group; and
Z represents a sulphur atom or an oxygen atom;
said aryl groups have from 6 to 10 carbon atoms in at least one aromatic ring and are unsubstituted;
which process comprises reacting a compound of formula (II): 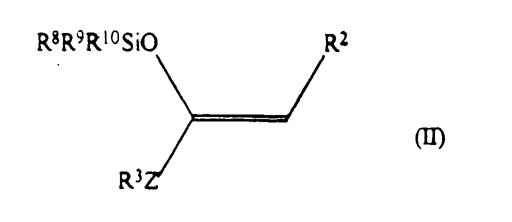 (in which R², R³ and Z are as defined above, and R⁸, R⁹ and R¹⁰ are the same or different and each represents an alkyl group having from 1 to 4 carbon atoms or a phenyl group) with a compound of formula (IIIa): 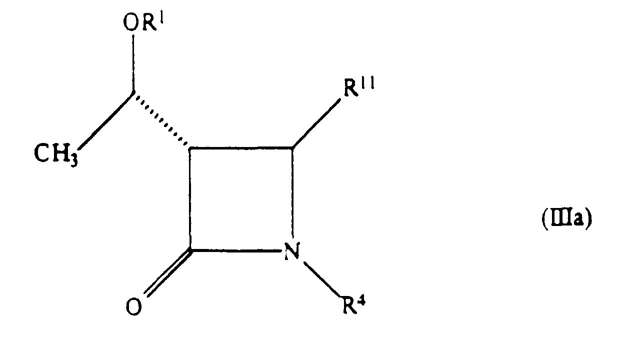 (in which R¹ and R⁴ are as defined above, and R¹¹ represents an acyloxy, alkylsulphonyl, arylsulphonyl, alkylsulphinyl or arylsulphinyl group).

2. A process according to Claim 1, in which the reagents and reaction conditions are chosen so as to prepare a compound of formula (Ia), in which R¹ represents:
a hydrogen atom;
an aliphatic acyl group having from 1 to 25 carbon atoms;
a halogenated alkanoyl group having from 2 to 6 carbon atoms;
an alkoxyalkanoyl group in which the alkoxy part has from 1 to 5 carbon atoms and the alkanoyl part has from 2 to 6 carbon atoms;
an alkenoyl or alkynoyl group having from 3 to 6 carbon atoms;
an aromatic acyl group, in which the aryl part has from 6 to 14 ring carbon atoms and is a carbocyclic aromatic group, which is unsubstituted or has from 1 to 5 of substituents (b), defined below;
a heterocyclic group having 5 or 6 ring atoms, of which 1 or 2 are oxygen and/or sulphur and/or nitrogen hetero-atoms, which groups are unsubstituted or are substituted by at least one of substituents (b), defined below, and/or by an oxygen atom;
a tri-substituted silyl group, in which all three or two or one of the substituents are alkyl groups having from 1 to 5 carbon atoms, and, correspondingly, none, one or two of the substituents are aryl groups, in which the aryl part has from 6 to 14 ring carbon atoms and is a carbocyclic aromatic group, which is unsubstituted or has from 1 to 5 of substituents (b), defined below;
an alkoxyalkyl group, in which the alkoxy and alkyl parts each have from 1 to 5 carbon atoms; a lower alkoxy-substituted lower alkoxymethyl group, in which each alkoxy part has from 1 to 5 carbon atoms;
a halogenated lower alkoxymethyl group, in which the alkoxy part has from 1 to 5 carbon atoms;
a halogenated ethyl group;
an arylselenyl-substituted ethyl group, in which the aryl part has from 6 to 14 ring carbon atoms and is a carbocyclic aromatic group, which is unsubstituted or has from 1 to 5 of substituents (b), defined below;
an aralkyl group, in which the alkyl part has from 1 to 4 carbon atoms and which is substituted with from 1 to 3 aryl groups, in which the aryl part has from 6 to 14 ring carbon atoms and is a carbocyclic aromatic group, which is unsubstituted or has from 1 to 5 of substituents (b), defined below;
an alkoxycarbonyl group having from 2 to 7 carbon atoms and which is unsubstituted or is substituted with a halogen atom or a tri-substituted silyl group, as defined above;
an alkenyloxycarbonyl group in which the alkenyl part has from 2 to 6 carbon atoms;
a sulpho group; or
an aralkyloxycarbonyl group, in which the alkyl part has from 1 to 4 carbon atoms and which is substituted with from 1 to 3 aryl groups, in which the aryl part has from 6 to 14 ring carbon atoms and is a carbocyclic aromatic group, which is unsubstituted or has from 1 to 5 of substituents (b), defined below;
said substituents (b) are selected from alkyl groups having from 1 to 6 carbon atoms, alkoxy groups having from 1 to 6 carbon atoms, halogen atoms, cyano groups, nitro groups, hydroxy groups, amino groups, alkylamino groups in which the alkyl part has from 1 to 4 carbon atoms, dialkylamino groups in which each alkyl part has from 1 to 4 carbon atoms, alkylenedioxy groups having from 1 to 3 carbon atoms, alkoxycarbonyl groups having from 2 to 7 carbon atoms and which are unsubstituted or are substituted with a halogen atom or a tri-substituted silyl group, and carbocyclic aryl groups which have from 6 to 14 ring carbon atoms and are unsubstituted or have from 1 to 5 of substituents (b), other than an aryl group.

3. A process according to Claim 1 or Claim 2, in which the reagents and reaction conditions are chosen so as to prepare a compound of formula (Ia), in which Z represents a sulphur atom.

4. A process according to Claim 1, in which the reagents and reaction conditions are chosen so as to prepare a compound of formula (Ia), in which R¹ represents:
a hydrogen atom;
a tri-substituted silyl group, in which all three or two or one of the substituents are alkyl groups having from 1 to 5 carbon atoms, and none, one or two of the substituents are aryl groups, as defined in Claim 2;
an aliphatic acyl group having from 1 to 6 carbon atoms;
a halogenated alkanoyl group having from 2 to 6 carbon atoms;
an alkoxyalkanoyl group in which the alkoxy part has from 1 to 5 carbon atoms and the alkanoyl part has from 2 to 6 carbon atoms; or
an aralkyloxycarbonyl group, in which the aryl part is as defined in Claim 2, and the alkyl part has from 1 to 4 carbon atoms.

5. A process according to any one of Claims 1 to 4, in which the reagents and reaction conditions are chosen so as to prepare a compound of formula (Ia), in which R⁴ represents a hydrogen atom or a tri-substituted silyl group, in which all three or two or one of the substituents are alkyl groups having from 1 to 5 carbon atoms, and none, one or two of the substituents are aryl groups, as defined in Claim 1.

6. A process according to Claim 1, in which the reagents and reaction conditions are chosen so as to prepare a compound of formula (Ia), in which:
R³ represents a phenyl group which has a substituent, at the 2-position on the ring, of formula -CYNR⁵R⁶ and no further substituent or a methyl substituent, wherein Y, R⁵ and R⁶ are as defined in Claim 1; and
Z represents a sulphur atom.

7. A process according to Claim 6, in which the reagents and reaction conditions are chosen so as to prepare a compound of formula (Ia), in which:
R¹ represents:
a hydrogen atom;
a tri-substituted silyl group, in which all three or two or one of the substituents are alkyl groups having from 1 to 5 carbon atoms, and none, one or two of the substituents are aryl groups, as defined below;
an aliphatic acyl group having from 1 to 6 carbon atoms;
a halogenated alkanoyl group having from 2 to 6 carbon atoms;
an alkoxyalkanoyl group in which the alkoxy part has from 1 to 5 carbon atoms and the alkanoyl part has from 2 to 6 carbon atoms; or
an aralkyloxycarbonyl group, in which the aryl part is as defined below, and the alkyl part has from 1 to 4 carbon atoms;
R³ represents:
a phenyl group which has a substituent, at the 2-position on the ring, of formula -CONR⁵R⁶ and no further substituent or a methyl substituent, where R⁵ and R⁶ are the same or different and each represents an alkyl group having from 1 to 3 carbon atoms, a phenyl group, or R⁵ and R⁶ together with the nitrogen atom to which they are attached form a group selected from the group comprising pyrrolidino, piperidino, morpholino and azepino groups;
R⁴ represents a hydrogen atom or a tri-substituted silyl group, in which all three or two or one of the substituents are alkyl groups having from 1 to 5 carbon atoms, and none, one or two of the substituents are aryl groups, as defined below;
said aryl groups are carbocyclic aryl groups which have from 6 to 10 carbon atoms in at least one aromatic ring and which are unsubstituted.

8. A process according to Claim 7, in which the reagents and reaction conditions are chosen so as to prepare a compound of formula (Ia), in which:
R³ represents:
a phenyl group which has a substituent, at the 2-position on the ring, of formula -CONR⁵R⁶ and no further substituent or a methyl substituent, where R⁵ and R⁶ are the same or different and each represents an alkyl group having from 1 to 3 carbon atoms, or R⁵ and R⁶ together with the nitrogen atom to which they are attached form a group selected from the group comprising pyrrolidino, piperidino, morpholino and azepino groups; and
R⁴ represents a hydrogen atom.

9. A process according to claim 1, in which the reagents and reaction conditions are chosen so as to prepare:
S-2-diethylcarbamoylphenyl 2(R)-{(3S,4S)-3-[1(R)-(t-butyldimethylsilyloxy)ethyl]-2-oxo-4-azetidinyl}thiopropionate;
S-2-dipropylcarbamoylphenyl 2(R)-{(3S,4S)-3-[1(R)-(t-butyldimethylsilyloxy)ethyl]-2-oxo-4-azetidinyl}thiopropionate;
2-diethylcarbamoylphenyl 2(R)-{(3S,4S)-3-[1(R)-(t-butyldimethylsilyloxy)ethyl]-2-oxo-4-azetidinyl}propionate;
S-2-[diethyl(thiocarbamoyl)]phenyl 2(R)-{(3S,4S)-3-[1(R)-(t-butyldimethylsilyloxy)ethyl]-2-oxo-4-azetidinyl}thiopropionate;
S-2-diethylcarbamoylphenyl 2(R)-{(3S,4S)-3-[1(R)-hydroxyethyl]-2-oxo-4-azetidinyl}thiopropionate;
S-2-(1-pyrrolidinylcarbonyl)phenyl 2(R)-{(3S,4S)-3-[1(R)-(t-butyldimethylsilyloxy)ethyl]-2-oxo-4-azetidinyl}thiopropionate;
S-2-(1-piperidylcarbonyl)phenyl 2(R)-{(3S,4S)-3-[1(R)-(t-butyldimethylsilyloxy)ethyl]-2-oxo-4-azetidinyl}thiopropionate;
S-2-(1-azepinylcarbonyl)phenyl 2(R)-{(3S,4S)-3-[1(R)-(t-butyldimethylsilyloxy)ethyl]-2-oxo-4-azetidinyl}thiopropionate; or
S-2-morpholinocarbonylphenyl 2(R)-{(3S,4S)-3-[1(R)-(t-butyldimethylsilyloxy)ethyl]-2-oxo-4-azetidinyl}thiopropionate.

10. A process for preparing a carbapenem compound of formula (XIX'): 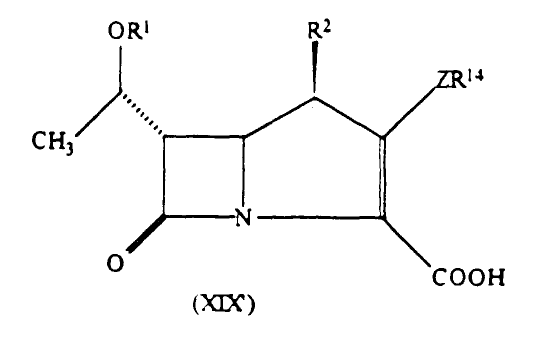 in which R¹, R² and Z are as defined in Claim 1 and R¹⁴ represents a variety of organic groups of the type commonly employed, at the indicated position, in carbapenem derivatives, which process comprises reacting a compound of formula (Ia), as defined in any one of Claims 1 to 9, with a mercapto compound of formula R¹⁴SH, and cyclising the product.
